# EUROPEAN PATENT SPECIFICATION

(11) **EP 1 987 160 B1**
(45) Date of publication and mention of the grant of the patent: **14.05.2014**
(21) Application number: 07709824.2
(22) Date of filing: 25.01.2007
(51) Int. Cl.: C12Q 1/68, G01N 33/574

(54) **TUMOR VASCULATURE MARKERS AND METHODS OF USE THEREOF**
TUMORGEFÄSSSYSTEMMARKER UND VERFAHREN ZUR VERWENDUNG DAVON
MARQUEURS DE SYSTÈME VASCULAIRE TUMORAL ET LEURS PROCÉDÉS D'UTILISATION

(30) Priority: 26.01.2006 US 762787 P; 12.04.2006 US 791212 P; 14.09.2006 US 844347 P
(43) Date of publication of application: 05.11.2008
(73) Proprietor: The Trustees of the University of Pennsylvania, Philadelphia, PA 19104-6283 (US)
(72) Inventor: COUKOS, George, Yardley, PA 19067 (US); BUCKANOVICH, Ronald, J., Ann Arbor, MI 48103 (US); GIMOTTY, Phyllis, A., Wallingford, PA 19086 (US)
(74) Representative: Dawson, Elizabeth Ann
(86) International application number: PCT/US2007/001959
(87) International publication number: WO 2007/089513

(56) References cited:
- EP-A- 1 524 523
- WO-A2-02/101357
- GB-A- 1 270 049
- US-A1- 2004 002 467
- US-B1- 6 423 494
- BREKKEN R A ET AL: "VEGF-VEGF receptor complexes as markers of tumor vascular endothelium" JOURNAL OF CONTROLLED RELEASE, ELSEVIER, AMSTERDAM, NL, vol. 74, no. 1-3, 6 July 2001 (2001-07-06), pages 173-181, XP004297522 ISSN: 0168-3659
- THORPE PHILIP E: "Vascular targeting agents as cancer therapeutics." CLINICAL CANCER RESEARCH : AN OFFICIAL JOURNAL OF THE AMERICAN ASSOCIATION FOR CANCER RESEARCH 15 JAN 2004, vol. 10, no. 2, 15 January 2004 (2004-01-15), pages 415-427, XP002543457 ISSN: 1078-0432
- ZHU Z ET AL: "INHIBITION OF TUMOR GROWTH AND METASTASIS BY TARGETING TUMOR-ASSOCIATED ANGIOGENESIS WITH ANTAGONISTS TO THE RECEPTORS OF VASCULAR ENDOTHELIAL GROWTH FACTOR" INVESTIGATIONAL NEW DRUGS, MARTINUS NIJHOFF PUBLISHERS, BOSTON, US, vol. 17, no. 3, 1 January 1999 (1999-01-01), pages 195-212, XP000940444 ISSN: 0167-6997
- BAMBERGER E S ET AL: "Angiogenesis in epithelian ovarian cancer." MOLECULAR PATHOLOGY, vol. 55, no. 6, December 2002 (2002-12), pages 348-359, XP002543458 ISSN: 1366-8714
- CHEN B ET AL: "Combining vascular and cellular targeting regimens enhances the efficacy of photodynamic therapy" INTERNATIONAL JOURNAL OF RADIATION: ONCOLOGY BIOLOGY PHYSICS, PERGAMON PRESS, US, vol. 61, no. 4, 15 March 2005 (2005-03-15), pages 1216-1226, XP025262935 ISSN: 0360-3016 [retrieved on 2005-03-15]
- UEDA MASATSUGU ET AL: "Tumor angiogenesis and molecular target therapy in ovarian carcinomas." HUMAN CELL : OFFICIAL JOURNAL OF HUMAN CELL RESEARCH SOCIETY MAR 2005, vol. 18, no. 1, March 2005 (2005-03), pages 1-16, XP002543459 ISSN: 0914-7470
- LU J.J. AND LORENZI M.V.: 'Identifying and Validating Oncology Therapeutic Targets in the Post-Genomics Era' CURRENT GENOMICS vol. 4, no. 1, 2003, pages 51 - 61, XP008129114
- KASOF G.M. ET AL.: 'Tumor necrosis factor-alpha induces the expression of DR6, a member of the TNF receptor family, through activation of NF-kappaB' ONCOGENE vol. 20, no. 55, 2001, pages 7965 - 7975, XP002967958
- BRIDGHAM J.T. ET AL.: 'Conservation of Death Receptor-6 in Avian and Piscine Vertebrates' BIOCHEMICAL AND BIOPHYSICAL RESEARCH COMMUNICATIONS vol. 284, no. 5, 2001, pages 1109 - 1115, XP008129115
- BUCKANOVICH R.J. ET AL.: 'Tumor Vasculature Proteins As Biomarkers in Ovarian Cancer' JOURNAL OF CLINICAL ONCOLOGY vol. 25, no. 7, 2007, pages 852 - 861, XP008129116

## Description

### FIELD OF INVENTION

This invention provides a ligand selected from a nucleic acid molecule which can hybridize to a nucleic acid molecule set forth in SEQ ID No: 1 and an antibody to a protein encoded by a nucleic acid molecule of SEQ ID No:1, for use in localizing an ovarian tumor vasculature. Also provided is said ligand for use in treating a solid ovarian tumor in a subject, wherein said treating an ovarian tumor comprises (a) contacting said subject with a the photoactivatable cytotoxic drug; (b) localizing an ovarian tumor vasculature with said ligand; and (c) contacting said solid ovarian tumor with a concentrated light source. Also provided is said ligand for use in treating an ovarian tumor in a subject, wherein said antibody or nucleic acid molecule is conjugated to a cytotoxic agent.

### BACKGROUND OF THE INVENTION

Brekken and Thorpe (J of Controlled Release 74 (2001) 173-181) disclose VEGF-VEGF receptor complexes as markers of tumor vascular endothelium. Thorpe (Clin Cancer Res. 415 (2004) vol 10, 415-427) discloses vascular targeting agents as cancer therapeutics. Zhu and Witte (Investigational New Drugs 17 (1999), 195-212) disclose the inhibition of tumor growth by targeting tumor associated angiogenesis. Bamberger and Perrett (J Clin Pathol:Mol Pathol (2002) 55:348-359) provide a review of angiogenesis in epithelial ovarian cancer. Chen et al. (Int J. Radiation Oncology Biol. Phys. 61; 4, pp1216-1226, 2005) disclose that combining vascular and cellular targeting regimens enhances the efficacy of photodynamic therapy. US2004/0002467 discloses antisense modulation of ADAM12 expression. EP1524523 discloses use of ADAM12 for diagnosis and therapy of preeclampsia. GB1270049 discloses pharmaceutical compositions useful for the treatment of malignant tumours. Ueda et al. (Human Cell 2005, vol 18; 1, 1-16) concerns tumor angiogenesis and molecular target therapy in ovarian carcinomas.

Markers of solid tumors and their vasculature have been difficult to identify because of difficulty in isolating these cells in contaminant-free preparations. Such markers are urgently need for use in a variety of therapeutic and diagnostic applications.

### SUMMARY OF THE INVENTION

This invention provides ligands as defined above for use in methods of detecting and localizing ovarian tumor vasculature cells (TVC) and ovarian solid tumors; and treating, impeding vascularization of, and determining the stage of solid ovarian tumors in a subject, comprising the step of contacting a subject or a TVC with a ligand of the present invention.

In one embodiment, the present invention provides a use of a ligand that is a nucleic acid molecule which hybridises with an ADAM12-encoding nucleic acid molecule of SEQ ID NO: 1 or that is an antibody to an ADAM12 protein encoded by SEQ ID NO:1 in the manufacture an agent for localizing a solid ovarian tumor vasculature in a subject. In another embodiment, the subject is contacted with the agent, and the ligand is localized, thereby localizing the solid tumor vasculature. The solid tumor is an ovarian tumor. Each possibility represents a separate embodiment of the present invention.

Said nucleic acid molecule preferably hybridises to the long isoform of SEQ ID NO:1.

In another embodiment, the present invention provides a ligand of the invention that is conjugated to a cytotoxic agent or cytotoxic drug for use in treating an ovarian tumor in a subject. The tumor may be a solid tumor.

### BRIEF DESCRIPTION OF THE FIGURES

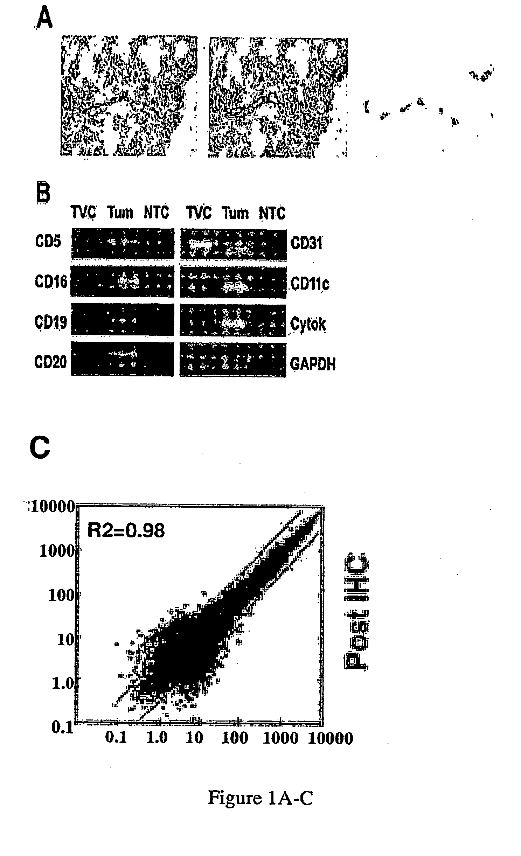

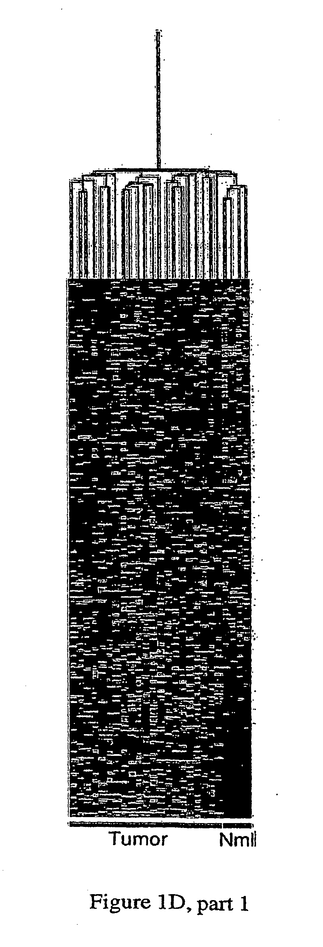

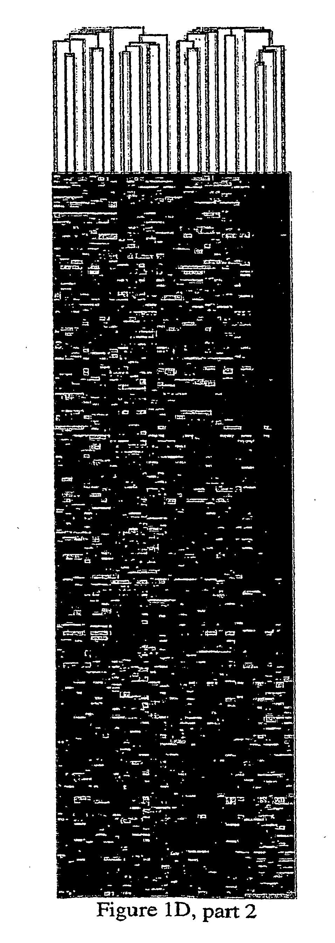

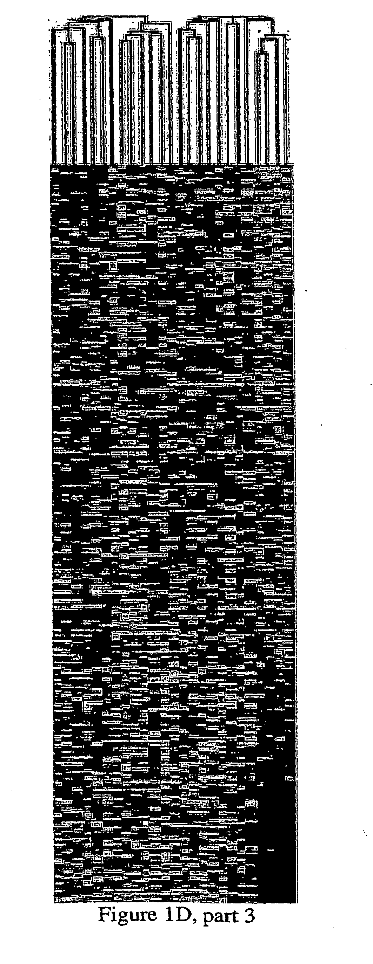

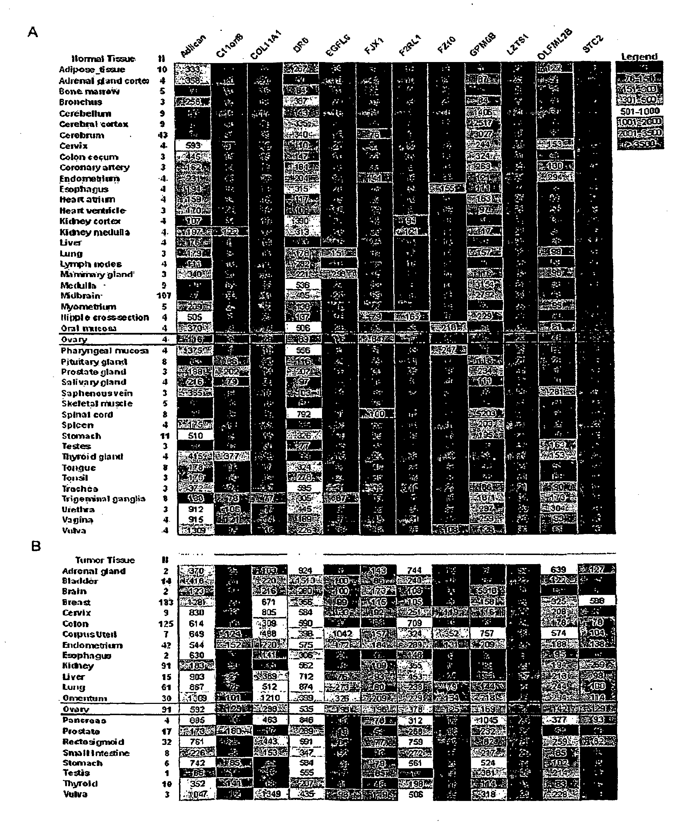

Figure 1: A. Immuno-LCM steps. Left panel: Rapid IHC for CD31 allows prompt identification of vasculature in ovarian cancer frozen sections. Middle panel: Tissue section after LCM of CD31⁺ cells. Right panel: captured tumor vascular cells. B. RT-PCR analysis of lineage-specific markers in RNA from tumor vascular cells (TVC) isolated with immuno-LCM, whole tumor (Tum) or a no template control (NTC). C. Scatter plot and correlation value of amplified RNA from unstained tissue (Pre IHC) versus RNA amplified after IHC optimized as in Table 1 (Post IHC). D. Part 1. Heat map condition tree developed using a hierarchical clustering algorithm, excluding all genes where the difference between the means of the tumor and normal vascular samples was less than its standard error. D, part 2: Red color shown alone. D, part 3: Green color shown alone. E. Archived Gene Expression Datasets. Data used for expression of the TVM in normal and tumor tissue samples; also available in the Gene Expression Omnibus (GEO; National Center for Biotechnology Information [NCBI]) with series numbers GSE3526 and GSE2109, respectively. All CEL-files were similarly processed using the Robust Multi-array Average (RMA) algorithm.

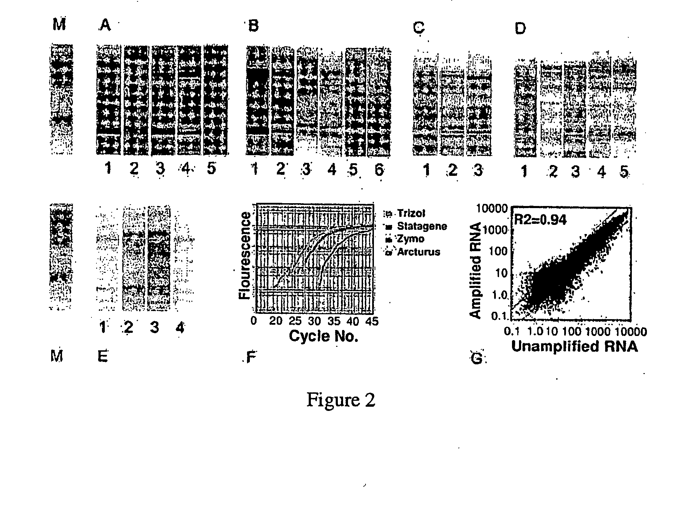

Figure 2. Quality of total RNA isolated from 8 µm frozen ovarian cancer tissue sections through different methodologies and analyzed by Agilent Bioanalyzer (A-E), quantitative real-time PCR (F) or Affymetrix U133A arrays (G). A. RNA distribution profiles following fixation with different fixatives. (Lanes: 1, ethanol; 2, methanol; 3, acetone; 4, acetic acid + ethanol; 5, paraformaldehyde). B. RNA profiles after isolation without immunostaining (Lane 1) or following IHC with or without RNAse inhibitor. (Lanes: 2, RNA Protector; 3, Placental RNAse inhibitor; 4, SuperRNASin; 5, RNA Protector+ SuperRNASin; 6, no RNAse inhibitor. C. RNA profiles after different immunostaining procedures. (Lanes: 1, IHC using DAB; 2, IHC using AEC; 3, immunofluorescence). D. Time course demonstrating RNA stability after IHC performed with procedure optimized as in Table 1. (Lanes: 1, 0 min; 2, 30 min; 3, one hr; 4, two hrs; 5, three hrs). E. RNA profiles following different RNA isolation methods. (Lanes: 1, Arcturus kit; 2, Stratagene kit; 3, modified Trizol; 4, Zymo kit). F. qRT-PCR for β-actin transcripts with RNA purified with the indicated RNA purification protocols. G. Scatter plots and correlation values of amplified RNA (y-axis) to unamplified total tumor RNA (x-axis).

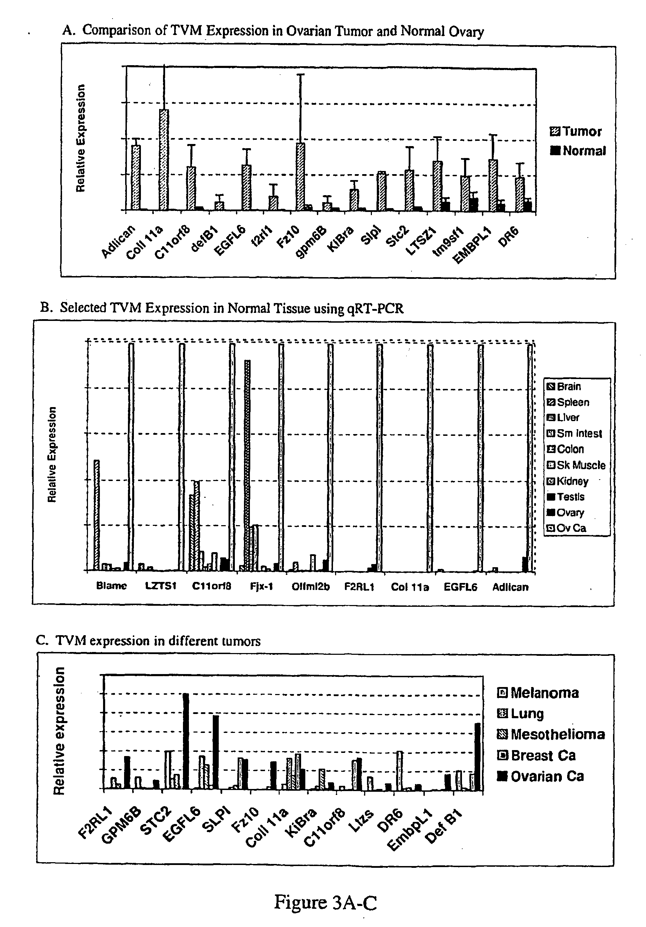

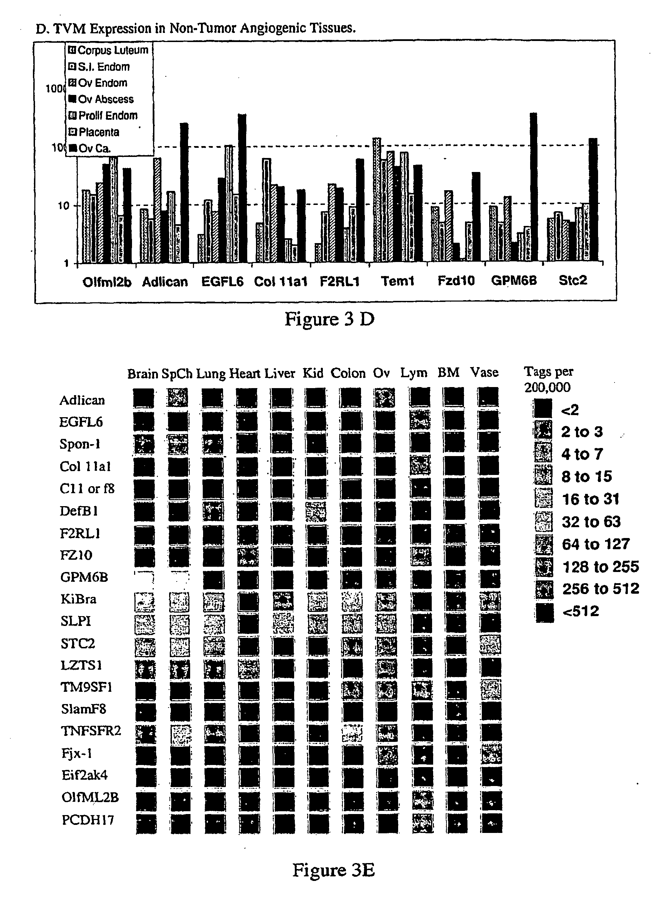

Figure 3: Expression of novel TVM. A. qRT-PCR analysis of tumor vascular markers (TVM) in whole epithelial ovarian cancer (Ovarian Ca, n=20) and normal ovaries (Nml Ovary, n=5). Results are expressed as relative expression units against □-actin. B. Expression of select TVM by qRT-PCR in a panel of normal human tissues (Sm Intest, small intestine; Sk Muscle, skeletal muscle); expression in ovarian cancer (Ov Ca) is defined as 100%. C. Expression of select TVM by qRT-PCR in different tumor types; expression in ovarian cancer is defined as 100%. Data were normalized against CD31. D. Expression of TVM in tissues with physiologic angiogenesis including corpus luteum (Corp Lut); proliferative endometrium (Prolif Endomet); and placenta, as well as ovarian cancer (Ov Ca). Data were normalized against CD31. E. Expression of TVM in normal human tissues by digital northern derived from the NIH SAGE expression database. Sp Ch (spinal cord), Kid (kidney, Ov (ovary), Lym (lymph node), BM (bone marrow), Vasc (vasculature). Squares with 32-63 tags are indicated by a double arrow to the left. Squares with 16-31 tags have a single arrow to the left. The remainder of the light-colored squares had 15 or fewer tags. "EPB41 L3" refers to EPB41 L3. This figure also includes additional markers that do not appear in Table 5; selection of these additional markers is described below in Example 11.

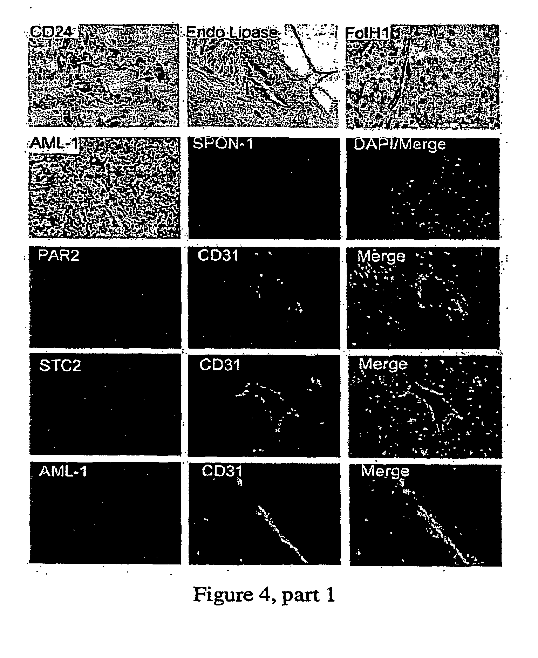

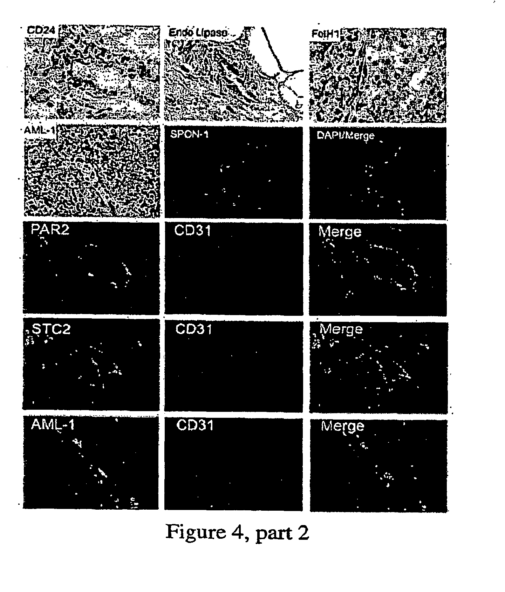

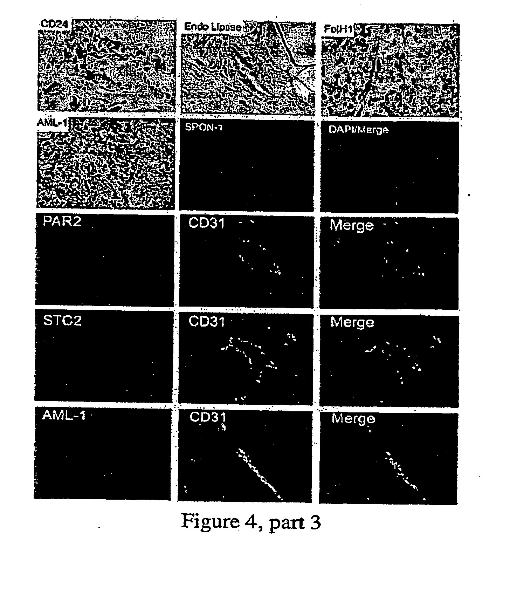

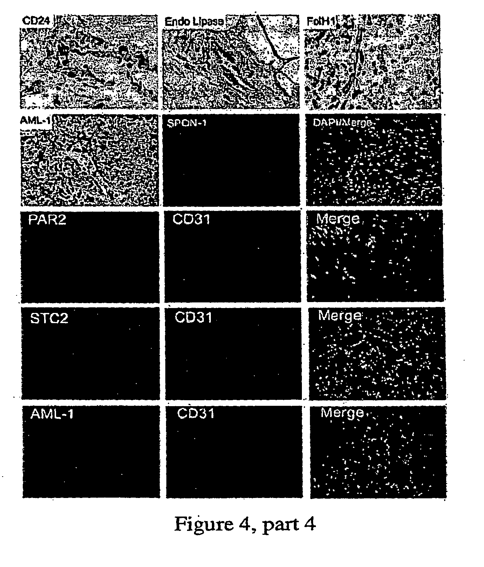

Figure 4. Part 1. Immuno-localization of identified markers to tumor vasculature. IHC is shown for CD24, endothelial lipase (Endo Lipase), FoIH1 and AML-1. Immunofluorescent localization of SPON-1 to vascular structures and co-staining of PAR2, STC2 and AML-1 (red) with CD31 (green) in microvascular structures in human ovarian tumors. Merge includes nuclear staining with DAPI. Red fluorescence is shown alone in part 2. Green fluorescence is shown alone in part 3. Blue fluorescence from DAPI is shown alone in part 4.

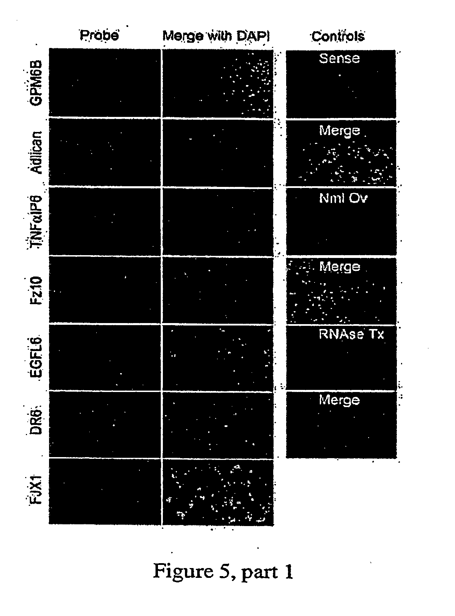

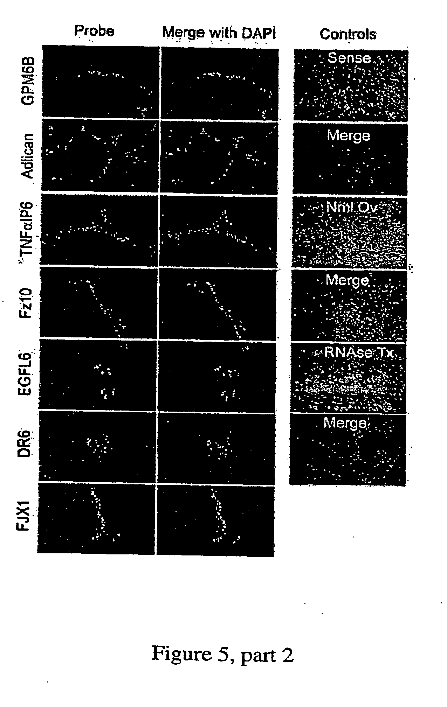

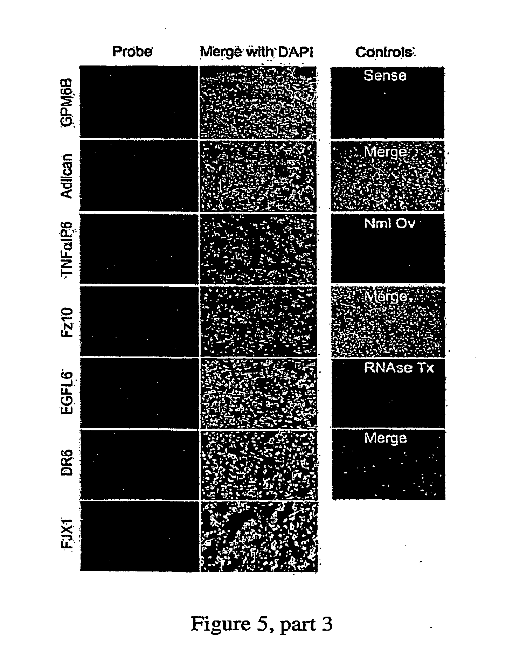

Figure 5. A. In-situ hybridization demonstrates expression of mRNA for the indicated tumor vascular markers in association with vascular structures in ovarian cancer. Negative controls included hybridization of tumor section with sense strand (Sense), hybridization of normal ovary with antisense probe (Nml Ov) and hybridization of RNAse-pretreated tumor section with antisense probe (RNAse Tx) for all TVM. Representative controls are shown for adlican, but were identical for all TVM. Merge is shown with DAPI nuclear staining. Red fluorescence is shown alone in part 2. Blue fluorescence from DAPI is shown alone in part 3.

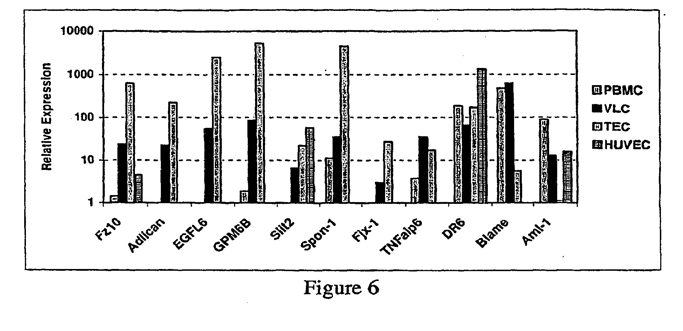

Figure 6. Expression of select TVM by qRT-PCR in FACS-purified VE-cadherin⁺ CD146⁺ CD45⁻ tumor endothelial cells (TEC) and VE-cadherin⁺ CD146⁺ CD45⁺ vascular leukocytes (VLC) compared with peripheral blood mononuclear cells (PBMC). Data were normalized against β-actin.

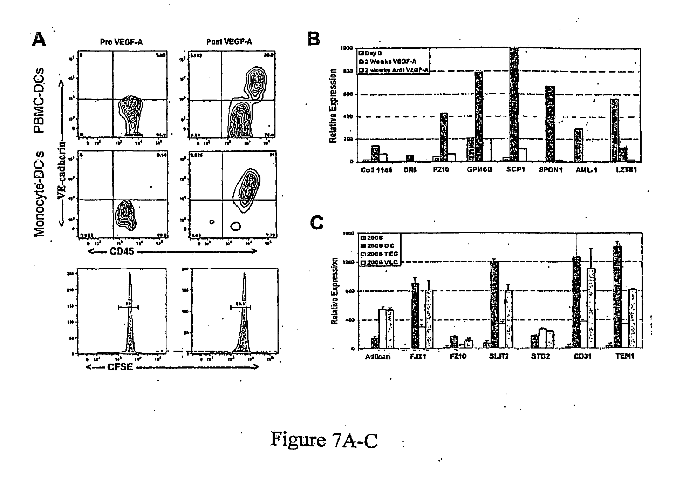

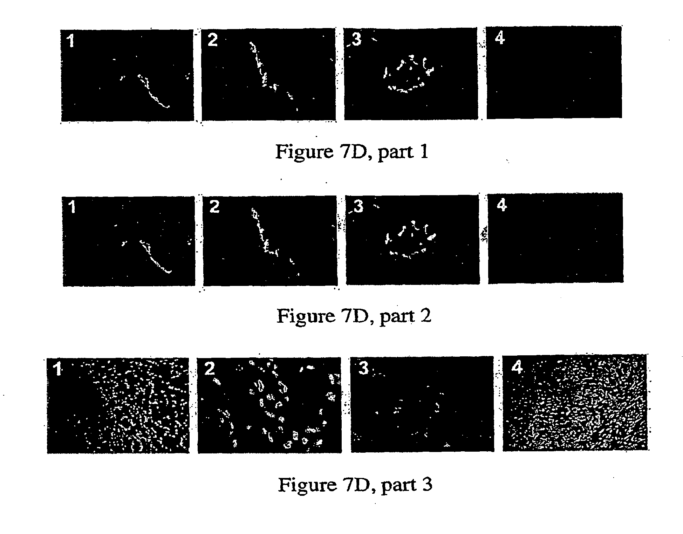

Figure 7, part 1. Expression of novel human TVM by myeloid monocytic cells. A. Flow cytometry analysis; Top, Expression of CD45 (x-axis) and VE-cadherin (y-axis) in DC derived from PBMC prior to exposure to rhVEGF₁₆₅ (Pre VEGF-A) and following 7-day treatment with rhVEGF₁₆₅ (Post VEGF-A); VE-cadherin⁺ CD45⁺ cells appear after VEGF165 treatment. Middle, Expression of CD45 and VE-cadherin in DC derived from enriched monocytes prior to (Pre VEGF-A) and following 7-day treatment with rhVEGF₁₆₅ (Post VEGF-A); 91 % of output cells after VEGF₁₆₅ treatment were VE-cadherin⁺CD45⁺, indicating that double positive cells derived from the monocyte fraction. Bottom, No significant CFSE dilution was noted in monocyte-derived DC after VEGF₁₆₅ treatment, indicating no cell proliferation in these cultures. B. Expression of markers by PBMC-derived DC at baseline (Day 0) and following exposure to rhVEGF₁₆₅, with or without antibody against VEGF-A for 2 weeks. Induction of several tumor vascular markers and suppression of AML-1 and LZTS1 are observed after VEGF₁₆₅. This was abrogated by VEGF blockade, except for AML-1 and LTZS1. Data were normalized against β-actin. C. Expression of TVM in human 2008 ovarian cancer xenografts injected alone (2008) or enriched with human PBMC-derived DC pretreated with VEGF₁₆₅ ex vivo (DC), FACS-purified human tumor endothelial cells (TEC) or FACS-purified human tumor vascular leukocytes (VLC). TVM are expressed by xenografts enriched with human myeloid cells or TEC but not by xenografts generated with human tumor cells only. Data were normalized against human β-actin. D. Immunofluorescent detection of human CD34⁺ vascular structures within 2008 tumors supplemented with human PBMC-derived DC pretreated with VEGF₁₆₅: 1: One CD34-PE positive vessel was noted in the early tumor xenograft. 2 and 3: Merge of CD34-PE and DAPI depicts vascular structures at higher magnification. 4: Control tumor generated with 2008 cells alone without VEGF-pretreated DC, TEC or VLC shows no expression of human CD34. Similar results were obtained with immunostaining for human CD31. E. Raw data-described in legend for Figure 7C.
Figure 7, parts 2-3. Red fluorescence is shown alone in part 2. Blue fluorescence from DAPI is shown alone in part 3.

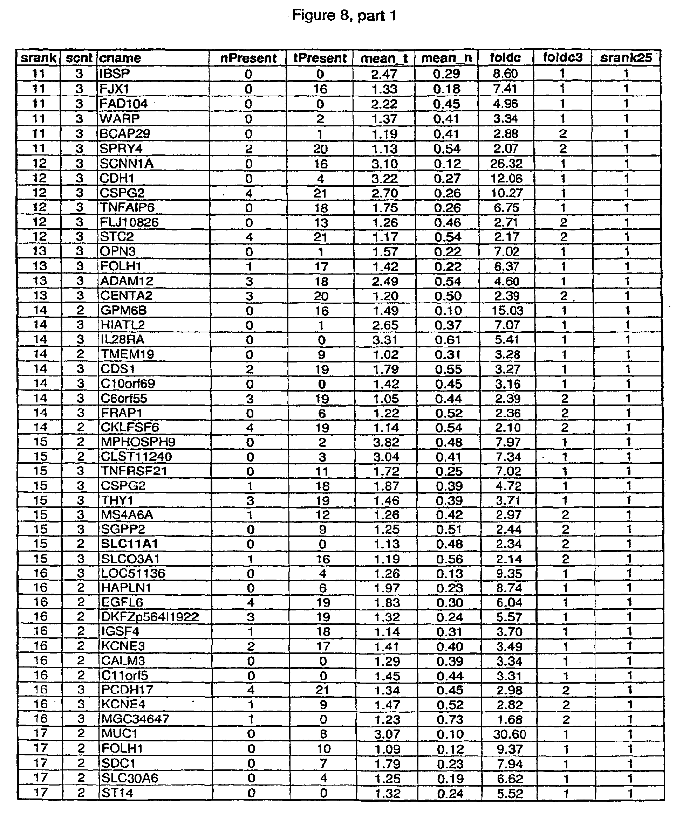

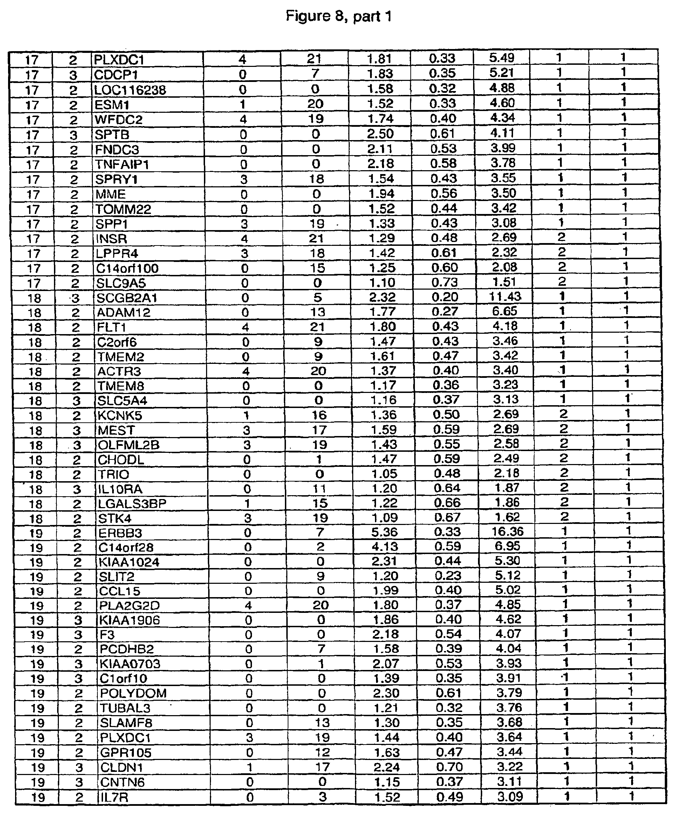

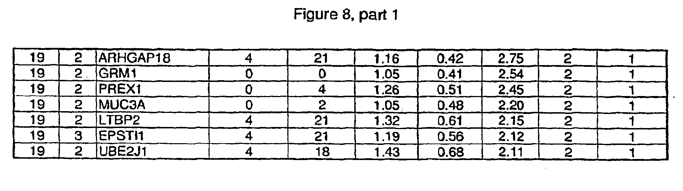

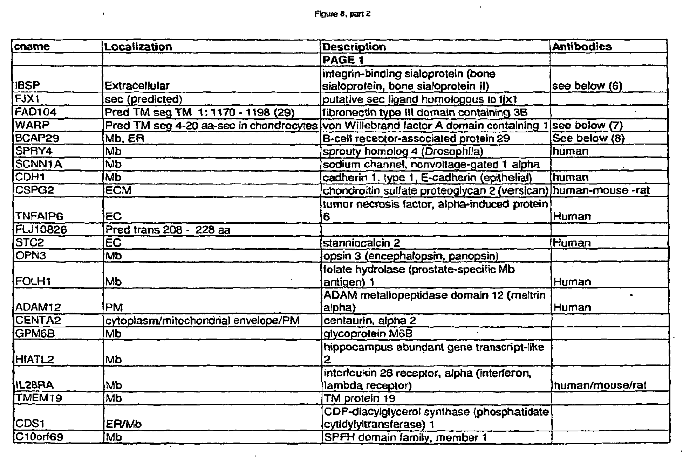

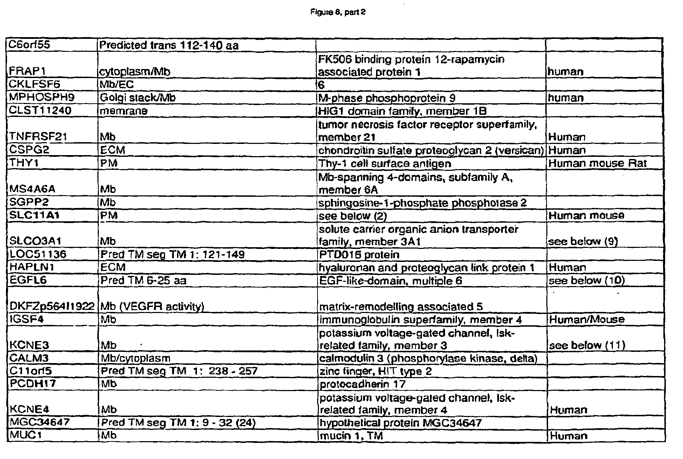

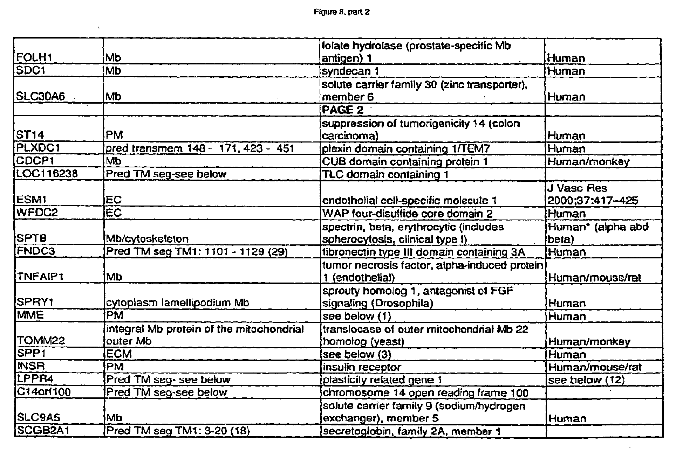

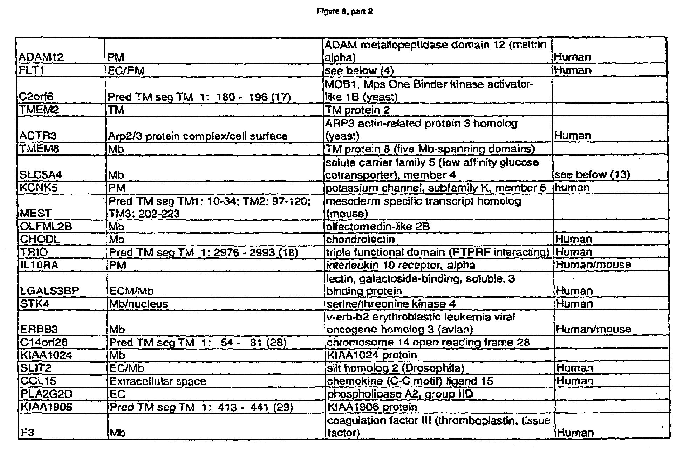

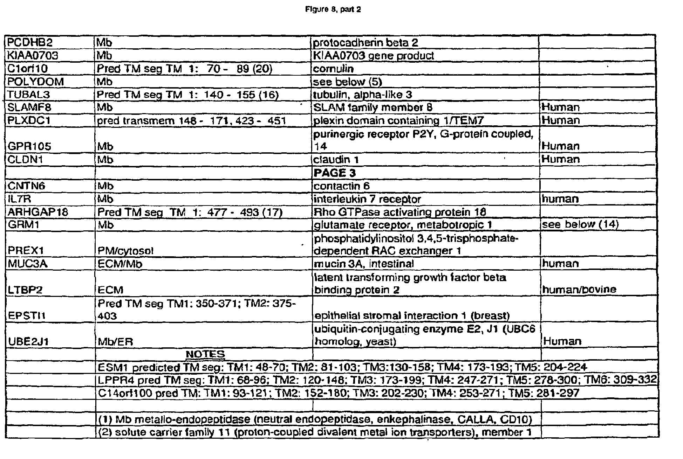

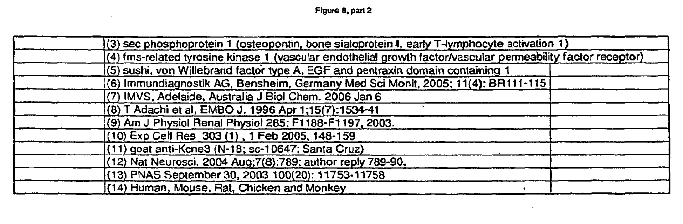

Figure 8, part 1. Secreted (sec) and transmembrane (TM) TVM. Column 1: expression rank (described in Example 2). Column 2: "Scnt": The number of normal samples that ranked in the bottom 4 positions. (e.g. if snt = 4, the 4 lowest expressing samples are all normal samples). Column 3: Gene symbol. Column 4: Number of normal samples (4 total) in which the transcript was classified as present. Column 5: Number of tumor samples (21 total) in which the transcript was classified as present. Column 6: Mean signal from the tumor samples. Column 7: Mean signal from the normal samples. Column 8: Fold increase of expression in tumor samples. Column 9: foldc3: Value of 1 means that the fold change was (>3). 2 fold change was (<3). Column 10: srank25. 1 means that the sum of ranks <25. Criteria for this list was a value of "1" in at least one of column 9 and column 10.
Figure 8, part 2. Column 1: Gene symbol. Column 2: Localization. Column 3: Description. Column 4: Available antibodies. Abbreviations: Pred: predicted; Mb: membrane; EC: extracellular; ECM extracellular matrix; PM: plasma membrane.
Figure 9. A. Real-time PCR analysis of Adlican in normal tissues and ovarian cancer. B. *In situ* hybridization of adlican in ovarian cancer. C. Real-time PCR analysis of adlican in ovarian tumors. D. Western blot shows adlican protein in tumor tissue lysate (T1-3) but not normal ovary (Nml) (left) and in serum (S 1-2) and ascites (A1-2) of patients with ovarian cancer but not normal human serum (NHS).

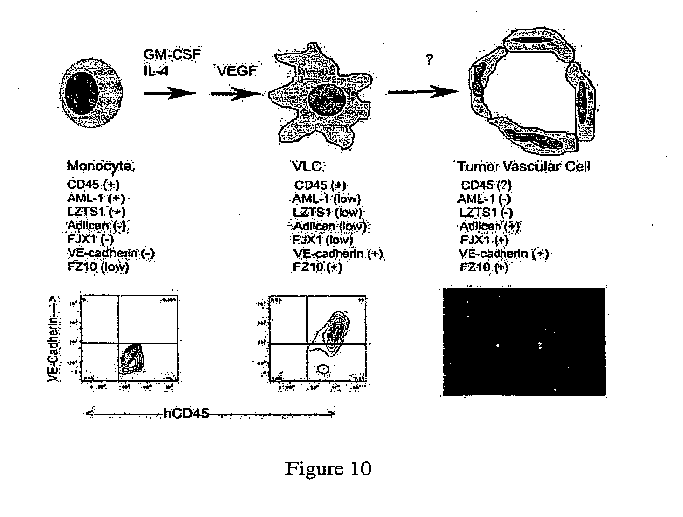

Figure 10. Model of step-wise vascular differentiation of monocyte-derived DC precursors in tumors. VEGF-A alone induces in monocytes downregulation of transcriptional regulators AML-1 and LZTS1, while newly identified markers (collagen 11α1, DR6, FZD10, GPM6B, SCP1 and SPON-1) as well as known endothelial markers (VE-cadherin, TEM-1) are upregulated, yielding VLC. Additional factors from the tumor microenvironment cooperate with VEGF-A to induce additional TVM found in TEC, as myeloid cells structurally participate in the assembly of vasculature.
Figure 11. Additional TVM.
Figure 12. List of additional TVM from another analysis.
Figure 13. A. DR6 mRNA expression by qPCR in normal tissues and ovarian tumor. B-D. IHC of DR6 in ovarian tumor (B), high-magnification image thereof (C), and normal ovary tissue (D).
Figure 14. DR6 protein is detected at higher levels in the serum of patients with ovarian tumors compared to healthy individuals. A- Western. B- quantitation.
Figure 15. Quantitation of ADAM12 mRNA relative expression by qPCR in normal tissues and ov tumor. A. Long isoform. B. Short isoform.
Figure 16. Staining with anti-ADAM12 Ab in frozen ovarian tumor sections
Figure 17. Double fluorescent staining with anti-ADAM12 Ab (red in original) and anti-CD31 Ab(green in original) in frozen ovarian tumor sections. Nuclei are stained blue. Top panel: green color alone (appears as white). Bottom: red color alone (appears as white).
Figure 18. Double fluorescent staining with anti-ADAM12 Ab (red in original) and anti-CD45 Ab (green in original) in frozen ovarian tumor sections. Nuclei are stained blue. Top panel: green color alone (appears as white). Bottom: orange color alone, representing overlap of green and red staining (appears as white).
Figure 19. CDCP1 mRNA relative expression in normal tissues and ovarian tumor (qPCR). A. Long isoform. B. Short isoform. Bars represent, from left to right: Ovarian tumor, bladder, esophagus, colon, liver, lung, brain, cervix, kidney, heart, adipose, testes, thymus, skeletal muscle, small intestine, spleen, trachea, ovary, placenta, thyroid, prostate.
Figure 20. Staining with anti-CDCP1 Ab in frozen ovarian tumor sections.
Figure 21. SLC11A1 mRNA expression by qPCR in normal tissues and ovarian tumor.
Figure 22. Staining with anti-SLC11A1 Ab in frozen ovarian tumor sections. Left panels: sample from first patient, at low (top) and high (bottom) magnification. Right panels: sample from second patient, at low (top) and high (bottom) magnification.
Figure 23. Double fluorescent staining with anti-SLC11A1 Ab (red in original) and anti-CD31 Ab (green in original) in frozen ovarian tumor sections. Nuclei are stained blue. A: green color alone (appears as white). B: red color alone (appears as white).
Figure 24. Double fluorescent staining with anti-SLC11A1 Ab (red in original) and anti-CD45 Ab (green in original) in frozen ovarian tumor sections. Nuclei are stained blue. A: green color alone (appears as white). B: orange color alone, representing overlap of green and red staining (appears as white).
Figure 25. BLAME mRNA expression by qPCR in normal tissues and ovarian tumor.
Figure 26. Staining with anti-BLAME Ab in frozen ovarian tumor sections. A: samples from 2 patients at low magnification (40x). B: samples from 2 patients at high magnification (60x).
Figure 27. A-B. Double fluorescent staining with anti-BLAME Ab (red in original) and anti-CD31 Ab (green in original) in frozen ovarian tumor sections. Nuclei are stained blue. A: green color alone. B: red color alone. C-E. Double fluorescent staining with anti-BLAME Ab (red in original) and anti-CD45 Ab (green in original) in frozen ovarian tumor sections. Nuclei are stained blue. C: green color alone. D: red color alone. E. orange color alone, representing overlap of green and red staining. Selected color (green, red, or orange) appears as white in each panel.

### DETAILED DESCRIPTION OF THE INVENTION

This invention provides a ligand selected from a nucleic acid molecule which can hybridize to a nucleic acid molecule set forth in SEQ ID No: 1 and an antibody to a protein encoded by a nucleic acid molecule of SEQ ID No: 1 for use in methods of detecting and localizing ovarian tumor vasculature cells (TVC) and solid tumors; and treating, impeding vascularization thereof, comprising the step of contacting a subject or a TVC with a ligand of the present invention.

In one embodiment, the present invention provides a ligand of the invention for use in a method of localizing an ovarian tumor vasculature in a subject, the method comprising the steps of: (a) contacting the subject with a ligand of the invention that binds an ADAM12-encoding nucleic acid molecule of SEQ ID NO: 1 or that binds an ADAM12 protein; and (b) localizing the ligand, thereby localizing an ovarian tumor vasculature in a subject. The tumor may be a solid tumor. The nucleic acid molecule may be the short isoform or the long isoform of SEQ ID NO: 1.

"Ligand" refers, in one embodiment, to antibodies to a protein encoded by a nucleic acid molecule of SEQ ID NO: 1. In another embodiment, the term includes nucleotide molecules that hybridize to a nucleic acid molecule of SEQ ID NO:1. Each possibility represents a separate embodiment of the present invention.

A "means of detecting" a TVM can be any means of detecting a nucleotide, protein, or fragment thereof known in the art. Means for detecting nucleotides and proteins in a subject are well known in the art. The means may be Western blotting, an antibody ELISA kit, an RT-PCR kit, an RNA isolation kit, a cDNA synthesis kitor any other suitable means known in the art.

In another embodiment, the present invention provides a ligand selected from a nucleic acid molecule which can hybridize to a nucleic acid molecule set forth in SEQ ID No: 1 and an antibody to a protein encoded by a nucleic acid molecule of SEQ ID No: 1, for use in treating an ovarian tumor in a subject, wherein said antibody or nucleic acid molecule is conjugated to a cytotoxic agent. Said ovarian tumor may be a solid tumor.

"Nucleic acid molecule" and "nucleotide" refer, in another embodiment, to an RNA molecule. In another embodiment, the terms refer to a DNA molecule. In another embodiment, the terms refer to any other type of nucleic acid molecule enumerated herein. In another embodiment, the terms referto any other type of nucleic acid molecule known in the art. Each possibility represents a separate embodiment of the present invention.

"Imaging" refers, in another embodiment, to localizing a ligand of interest using an imaging or scanning technology. In another embodiment, the ligand is a fluorescent ligand. In another embodiment, the ligand is radioactive. In another embodiment, the ligand is bound by a molecule (e.g. an antibody) that is detectable by the imaging or scanning technology. In another embodiment, any suitable imaging or scanning technology known in the art may be utilized. Each possibility represents a separate embodiment of the present invention.

As disclosed herein, a rapid protocol was developed and optimized for immuno-LCM of TVC, followed by extraction and amplification of RNA for array analysis of tumor vascular cells (Example 1 This enabled identification of the novel tumor vasculature markers (TVM). The identified transcripts and proteins encoded thereby were validated as TVM by a number of independent lines of evidence, including enrichment in independent tumor samples, relative to normal vascular samples; enrichment in tumor tissue relative to a variety of tissue samples; and comparison of expression levels between tumor tissue and tissues with physiologic angiogenesis (Example 3).

As disclosed herein, certain TVM transcripts and the proteins encoded thereby are efficacious in localizing solid tumors and vasculature thereof (Examples 4 and 5).

Localizing or detecting a tumor, TVC, or tumor vasculature may utilize positron-emission tomography (PET) scanning or any other imaging technique known in the art.

As provided in the Examples herein, certain TVM are expressed at detectable levels only by TVC. or the TVM are expressed at higher levels by TVC than by healthy tissue. Thus, TVM provide a means of specifically targeting therapeutic modalities to solid tumors and their vasculature.

The anti-cancer agent utilized in uses of the present invention is, in another embodiment, a radioactive isotope. In another embodiment, the anti-cancer agent is a cytotoxic agent. In another embodiment, the anti-cancer agent is a cytotoxic drug. In another embodiment, the anti-cancer agent is a nucleic acid molecule. In another embodiment, the anti-cancer agent is an antisense molecule. In another embodiment, the anti-cancer agent is an RNA inhibitory molecule. In another embodiment, the anti-cancer agent is an anti-tumor agent. In another embodiment, the anti-cancer agent is a cytotoxic virus. In another embodiment, the anti-cancer agent is a cytotoxic pathogen. In another embodiment, the anti-cancer agent is a nanosphere. In another embodiment, the nanosphere is loaded with a cytotoxic compound. In another embodiment, the nanosphere is loaded with a chemotherapy drug. In another embodiment, the nanosphere is loaded with a toxin. In another embodiment, the nanosphere is loaded with an anti-cancer compound. In another embodiment, the anti-cancer agent is a nanoparticle. In another embodiment, the anti-cancer agent is an engineered T cell. In another embodiment, the anti-cancer agent is an engineered cytotoxic cell. In another embodiment, the anti-cancer agent is any other type of engineered molecule known in the art. In another embodiment, the anti-cancer agent is any other agent used in cancer therapy. In another embodiment, the anti-cancer agent is any other type of anti-cancer agent known in the art. Each possibility represents a separate embodiment of the present invention.

"Engineered T cell" refers, in another embodiment, to a T cell designed to recognize a cell containing or expressing a molecule of interest. In another embodiment, the molecule of interest is a TVM of the present invention. In another embodiment, the term refers to a T cell with redirected specificity (T-bodies) for a TVM. In another embodiment, an engineered T cell of the present invention expresses a ligand that binds to or interacts with a TVM. In another embodiment, the engineered T cell exhibits specific activity against a TVC.

In another embodiment, an engineered T cell of the present invention expresses a chimeric immunoreceptor (CIR) directed against a TVM. In another embodiment, the CIR contains a bi-partite signaling module. In another embodiment, the extracellular module of the CIR is a single chain variable fragment (scFv) antibody that binds or interacts with a TVM. In another embodiment, the intracellular module of the CIR contains a costimulatory domain. In another embodiment, the costimulatory domain is a 4-1 BB domain. In another embodiment, the costimulatory domain is a TCRζ domain. In another embodiment, the CIR contains both a 4-1 BB domain and a TCRζ domain.

In another embodiment, an engineered T cell of the present invention is expanded in culture. In another embodiment, an engineered T cell of the present invention is activated in culture.

Each type of engineered T cell represents a separate embodiment of the present invention.

"Cytotoxic virus" refers, in another embodiment, to a virus capable of lysing a cell. In another embodiment, the term refers to a virus capable of lysing a tumor cell. In another embodiment, the virus is a recombinant virus that has been engineered to exhibit a characteristic favorable for anti-tumor activity. In another embodiment, the virus is wild-type, other than is conjugation to an antibody or ligand of the present invention. In another embodiment, the virus is an attenuated virus. Each possibility represents a separate embodiment of the present invention.

In another embodiment, the cytotoxic agent or anti-tumor agent is concentrated in the solid tumor. In another embodiment, the cytotoxic agent or anti-tumor agent is targeted to the solid tumor. In another embodiment, concentration of the cytotoxic agent or anti-tumor agent induces cytotoxicity in a tumor cell of the solid tumor. Each possibility represents a separate embodiment of the present invention.

In another embodiment the invention provides an antisense or RNAi molecule that is taken up by a vasculature cell for use in impeding a vascularization of an ovarian tumor in a subject, wherein the antisense or RNAi molecule is capable of inhibiting an activity of a nucleic acid molecule set forth in SEQ ID No: 1 or a protein encoded by said nucleic acid molecule.

Also disclosed is a method of impeding an angiogenesis in a subject, the method comprising the step of contacting the subject with a ligand capable of binding to a nucleic acid molecule, wherein the nucleic acid molecule is a TVM disclosed herein, whereby the ligand is taken up by a vasculature cell of the solid tumor or a precursor of the vasculature cell, thereby impeding an angiogenesis in a subject.

Also disclosed is a method of impeding an angiogenesis in a subject, the method comprising the step of contacting the subject with an antibody or ligand that binds to a protein that is a TVM disclosed herein, whereby the ligand inhibits an activity of a vasculature cell of the solid tumor or a precursor of the vasculature cell, thereby impeding an angiogenesis in a subject.

"Vasculature cell" refers, in another embodiment, to a cell capable of forming a tumor vasculature. In another embodiment, the term refers to a cell of a tumor vasculature. In another embodiment, "precursor of the vasculature cell" refers to a cell capable of differentiating into a cell capable of forming a tumor vasculature. In another embodiment, the term refers to a cell capable of differentiating into a cell of a tumor vasculature. Each possibility represents a separate embodiment of the present invention.

"Activity" refers, in another embodiment, to an angiogenic activity. In another embodiment, the term refers to production of a protein disclosed herein. In another embodiment, the term refers to any other tumorigenic activity known in the art. Each possibility represents a separate embodiment of the present invention.

In another embodiment, the ligand reduces the expression of the nucleic acid to which it binds, or the expression of the protein encoded by the nucleic acid. In another embodiment, the ligand reduces the activity of the protein to which it binds. In another embodiment, binding to the ligand results in degradation of the nucleic acid molecule or protein to which it binds. In another embodiment, the ligand antagonizes the nucleic acid molecule or protein to which it binds via any other mechanism known in the art. Each possibility represents a separate embodiment of the present invention.

As provided herein, certain TVM of the present invention are up-regulated upon differentiation of precursor cells into TVC. Thus, these TVM (both the nucleic acid molecules and the proteins encoded thereby) play important roles in the function of TVC in angiogenesis, and thus in the pathogenesis of solid tumors. Accordingly, targeting the expression or activity of the nucleic acids and proteins represents an efficacious means of impeding vascularization of solid tumors.

The ligand utilized in uses of the present invention is, in another embodiment, an antisense molecule. In another embodiment, the ligand is an RNA molecule. In another embodiment, the ligand is an inhibitory RNA (RNAi) molecule. In another embodiment, the RNA molecule is a short hairpin RNA (shRNA). In another embodiment, the RNA molecule is a small inhibitory RNA (siRNA). In another embodiment, the RNA molecule is a microRNA (miRNA). The use of siRNA and miRNA has been described, inter alia, in (Caudy AA et al, Genes & Devel 16: 2491-96 and references cited therein). In another embodiment, the RNA molecule is an anti-sense locked-nucleic acid (LNA) oligonucleotide. In another embodiment, the RNA molecule is any type of inhibitory RNA enumerated or described in Banan M et al (The ins and outs of RNAi in mammalian cells. Curr Pharm Biotechnol. 2004 Oct;5(5):441-50. In another embodiment, the RNA molecule is any type of RNAi known in the art.

In another embodiment, an RNA molecule utilized in uses of the present invention comprises a string of at least two base-sugar-phosphate combinations. The term includes, in another embodiment, compounds comprising nucleotides in which the sugar moiety is ribose. In another embodiment, the term includes both RNA and RNA derivates in which the backbone is modified. "Nucleotides" refers, in one embodiment, to the monomeric units of nucleic acid polymers. RNA may be, in another embodiment, in the form of a tRNA (transfer RNA), snRNA (small nuclear RNA), rRNA (ribosomal RNA), mRNA (messenger RNA), and ribozymes. In addition, these forms of RNA may be single, double, triple, or quadruple stranded. The term also includes, in another embodiment, artificial nucleic acids that may contain other types of backbones but the same bases. In one embodiment, the artificial nucleic acid is a PNA (peptide nucleic acid). PNA contain peptide backbones and nucleotide bases and are able to bind, in one embodiment, to both DNA and RNA molecules. In another embodiment, the nucleotide is oxetane modified. In another embodiment, the nucleotide is modified by replacement of one or more phosphodiester bonds with a phosphorothioate bond. In another embodiment, the artificial nucleic acid contains any other variant of the phosphate backbone of native nucleic acids known in the art. The use of phosphothiorate nucleic acids and PNA are known to those skilled in the art, and are described in, for example, Neilsen PE (Curr Opin Struct Biol 9:353-57); and Raz NK et al (Biochem Biophys Res Commun. 297:1075-84). The production and use of nucleic acids is known to those skilled in art and is described, for example, in Molecular Cloning, (2001), Sambrook and Russell, eds. and Methods in Enzymology: Methods for molecular cloning in eukaryotic cells (2003) Purchio and G. C. Fareed. Each nucleic acid derivative represents a separate embodiment of the present invention.

In another embodiment, the ligand induces degradation of the target nucleic acid molecule. In another embodiment, the ligand inhibits transcription of the nucleic acid molecule. In another embodiment, the ligand kills the TVC. In another embodiment, the ligand induces apoptosis of the TVC. In another embodiment, the ligand induces necrosis of the TVC. In another embodiment, the ligand inhibits proliferation of the TVC. In another embodiment, the ligand inhibits division of the TVC. In another embodiment, the ligand inhibits growth of the solid tumor. In another embodiment, the ligand induces regression of the solid tumor. Each possibility represents a separate embodiment of the present invention.

The activity of a vasculature cell that is inhibited by uses of the present invention, is, another embodiment, a catalytic activity. In another embodiment, the activity is interaction with another cell. In another embodiment, the activity is cell-cell adhesion. In another embodiment, the activity is contact repulsion. In another embodiment, the activity is DNA binding. In another embodiment, the activity is transcriptional regulation. In another embodiment, the activity is cartilage condensation. In another embodiment, the activity is protein binding. In another embodiment, the activity is extracellular structure organization and/or biogenesis. In another embodiment, the activity is phosphate transport. In another embodiment, the activity is G-protein coupled receptor protein signaling. In another embodiment, the activity is intracellular calcium signaling. In another embodiment, the activity is rhodopsin-like receptor activity. In another embodiment, the activity is thrombin receptor activity. In another embodiment, the activity is cell cycle modulation. In another embodiment, the activity is cell cycle inhibition. In another embodiment, the activity is cell surface receptor-linked signal transduction. In another embodiment, the activity is inflammatory signaling. In another embodiment, the activity is anti-inflammatory signaling. In another embodiment, the activity is hyaluronic acid binding. In another embodiment, the activity is any other activity performed by a protein encoded by a nucleic acid molecule having a sequence set forth in SEQ ID No: 1. Each possibility represents a separate embodiment of the present invention.

As disclosed herein, TVM are upregulated upon differentiation to TVC, both *in vitro* and *in vivo* (Examples 6-7), showing that expression levels of these proteins, and nucleotides encoding same can be used to determine the sate of a solid tumor.

Disclosed is a method of early diagnosis of a tumor, comprising contacting subject with a ligand that binds a TVM disclosed herein.

The TVM, nucleic acid molecule, or protein encoded thereby that is detected, bound, inhibited, or targeted by a use of the present invention is, in another embodiment, in the target cell (e.g. the vasculature cell, solid tumor cell, or the like). In another embodiment, the nucleic acid molecule or protein is associated with the target cell. In another embodiment, the nucleic acid molecule or protein is expressed by the target cell. Each possibility represents a separate embodiment of the present invention.

In another embodiment, the present invention provides a ligand of the invention for use in a method of treating a solid ovarian tumor in a subject, the method comprising the steps of: (a) contacting the subject with a photo-activatable cytotoxic drug; (b) localizing the solid ovarian tumor with a ligand of the present invention; and (c) contacting the solid tumor with a concentrated light source, thereby treating a solid ovarian tumor in a subject.

Methods for isolation of VLC are well known in the art, and are described, for example, in Conejo-Garcia, J. R., Buckanovich, R. J., Benencia, F., Courreges, M. C., Rubin, S. C., Carroll, R. G. & Coukos, G. (2005) Blood 105: 679-81. In another embodiment, "VLC" refers to VE- cadherin⁺CD146⁺ CD45⁺ cells. In another embodiment, the term refers to human myeloid vascular cells with endothelial-like behavior.

In another embodiment, a VLC of the present invention is a precursor of a TEC of the present invention. In another embodiment, a VLC of the present invention is a separate lineage from of a TEC of the present invention. In another embodiment, VLC of the present invention cooperate with TEC of the present invention in neo-vessel formation. Each possibility represents a separate embodiment of the present invention.

In another embodiment, a TVM of the present invention is expressed by pericytes, in addition to TVC. In another embodiment, the TVM is expressed by a subset of pericytes. In another embodiment, the TVM is not expressed on pericytes.

A TVC of the present invention is, in another embodiment, an endothelial cell. In another embodiment, the TVC is a perivascular cell. In another embodiment, the TVC derives from a myeloid DC. In another embodiment, the TVC derives from a myeloid monocytic precursor. Each possibility represents a separate embodiment of the present invention.

Disclosed is a method for isolation of a TVC, as exemplified in the Examples herein.

A TVM of the present invention has a sequence set forth in SEQ ID No: 1.

In another embodiment, the TVM is an ADAM12 protein. In another embodiment, the marker is a nucleic acid molecule encoding an ADAM12 protein. In another embodiment, the ADAM12 nucleotide is a long isoform of ADAM12. In another embodiment, the ADAM12 nucleotide is a short isoform of ADAM12. In another embodiment, the ADAM12 protein is encoded by a nucleic acid molecule having the sequence: 
cactaacgctcttcctagtccccggccaactcggacagtttgctcatttattgcaacggtcaaggctggcttgtgccagaacggcgcgcgcgc
gacgcacgcacacacacggggggaaactttttaaaaatgaaaggctagaagagctcagcggcggcgcgggccgtgcgcgagggctccg
gagctgactcgccgaggcaggaaatccctccggtcgcgacgcccggccccgctcggcgcccgcgtgggatggtgcagcgctcgccgccg
ggcccgagagctgctgcactgaaggccggcgacgatggcagcgcgcccgctgcccgtgtcccccgcccgcgccctcctgctcgccctggc
cggtgctctgctcgcgccctgcgaggcccgaggggtgagcttatggaaccaaggaagagctgatgaagttgtcagtgcctctgttcggagtgg
ggacctctggatcccagtgaagagcttcgactccaagaatcatccagaagtgctgaatattcgactacaacgggaaagcaaagaactgatca
taaatctggaaagaaatgaaggtctcattgccagcagtttcacggaaacccactatctgcaagacggtactgatgtctccctcgctcgaaattac
acggtaattctgggtcactgttactaccatggacatgtacggggatattctgattcagcagtcagtctcagcacgtgttctggtctcaggggacttatt
gtgtttgaaaatgaaagctatgtcttagaaccaatgaaaagtgcaaccaacagatacaaactcttcccagcgaagaagctgaaaagcgtccg
gggatcatgtggatcacatcacaacacaccaaacctcgctgcaaagaatgtgtttccaccaccctctcagacatgggcaagaaggcataaaa
gagagaccctcaaggcaactaagtatgtggagctggtgatcgtggcagacaaccgagagtttcagaggcaaggaaaagatctggaaaag
ttaagcagcgattaatagagattgctaatcacgttgacaagttttacagaccactgaacattcggatcgtgttggtaggcgtggaagtgtggaatg
acatggacaaatgctctgtaagtcaggacccattcaccagcctccatgaatttctggactggaggaagatgaagcttctacctcgcaaatcccat
gacaatgcgcagcttgtcagtggggtttatttccaagggaccaccatcggcatggccccaatcatgagcatgtgcacggcagaccagtctggg
ggaattgtcatggaccattcagacaatccccttggtgcagccgtgaccctggcacatgagctgggccacaatttcgggatgaatcatgacacac
tggacaggggctgtagctgtcaaatggcggttgagaaaggaggctgcatcatgaacgcttccaccgggtacccatttcccatggtgttcagcag
ttgcagcaggaaggacttggagaccagcctggagaaaggaatgggggtgtgcctgtttaacctgccggaagtcagggagtctttcgggggcc
agaagtgtgggaacagatttgtggaagaaggagaggagtgtgactgtggggagccagaggaatgtatgaatcgctgctgcaatgccaccac
ctgtaccctgaagccggacgctgtgtgcgcacatgggctgtgctgtgaagactgccagctgaagcctgcaggaacagcgtgcagggactcca
gcaactcctgtgacctcccagagttctgcacaggggccagccctcactgcccagccaacgtgtacctgcacgatgggcactcatgtcaggatg
tggacggctactgctacaatggcatctgccagactcacgagcagcagtgtgtcacactctggggaccaggtgctaaacctgcccctgggatct
gctttgagagagtcaattctgcaggtgatccttatggcaactgtggcaaagtctcgaagagttcctttgccaaatgcgagatgagagatgctaaat
gtggaaaaatccagtgtcaaggaggtgccagccggccagtcattggtaccaatgccgtttccatagaaacaaacatcccctgcagcaagg
aggccggattctgtgccgggggacccacgtgtacttggcgatgacatgccggacccagggcttgtgcttgcaggcacaaagtgtgcagatg
gaaaaatctgcctgaatcgtcaatgtcaaaatattagtgtctttggggttcacgagtgtgcaatgcagtgccacggcagaggggtgtgcaacaa
caggaagaactgccactgcgaggcccactgggcacctcccttctgtgacaagtttggctttggaggaagcacagacagcggccccatccgg
caagcagataaccaaggtttaaccataggaattctggtgaccatcctgtgtcttcttgctgccggatttgtggtttatctcaaaaggaagaccttgat
acgactgctgtttacaaataagaagaccaccattgaaaaactaaggtgtgtgcgcccttcccggccaccccgtggcttccaaccctgtcaggct
cacctcggccaccttggaaaaggcctgatgaggaagccgccagattcctacccaccgaaggacaatcccaggagattgctgcagtgtcaga
atgttgacatcagcagacccctcaacggcctgaatgtccctcagccccagtcaactcagcgagtgcttcctcccctccaccggccccacgtg
cacctagcgtccctgccagacccctgccagccaagcctgcacttaggcaggcccaggggacctgtaagccaaacccccctcagaagcctct
gcctgcagatcctctggccagaacaactcggctcactcatgccttggccaggaccccaggacaatgggagactgggctccgcctggcaccc
ctcagacctgctccacaatatccacaccaagtgcccagatccacccacaccgcctatattaagtgagaagccgacacctttttcaacagtgaa
gacagaagtttgcactatctttcagctccagttggagtttttgtaccaacttttaggatttttttaatgtttaaaacatcattactataagaactttgagct
actgccgtcagtgctgtgctgtgctatggtgctctgtctacttgcacaggtacttgtaaattattaatttatgcagaatgttgattacagtgcagtgcgct
gtagtaggcatttttaccatcactgagttttccatggcaggaaggcttgttgtgcttttagtatttagtgaacttgaaatatcctgcttgatgggattctgg
acaggatgtgtttgctttctgatcaaggccttattggaaagcagtcccccaactacccccagctgtgcttatggtaccagatgcagctcaagagat
cccaagtagaatctcagttgattttctggattccccatctcaggccagagccaaggggcttcaggtccaggctgtgtttggctttcagggaggccct
gtgccccttgacaactggcaggcaggctcccagggacacctgggagaaatctggcttctggccaggaagctttggtgagaacctgggttgca
 
gacaggaatcttaaggtgtagccacaccaggatagagactggaacactagacaagccagaacttgaccctgagctgaccagccgtgagca
tgtttggaaggggtctgtagtgtcactcaaggcggtgcttgatagaaatgccaagcacttctttttctcgctgtcctttctagagcactgccaccagta
ggttatttagcttgggaaaggtggtgtttctgtaagaaacctactgcccaggcactgcaaaccgccacctccctatactgcttggagctgagcaa
atcaccacaaactgtaatacaatgatcctgtattcagacagatgaggactttccatggaccacaactattttcagatgtgaaccattaaccagat
ctagtcaatcaagtctgtttactgcaaggttcaacttattaacaattaggcagactctttatgcttgcaaaaactacaaccaatggaatgtgatgttc
atgggtatagttcatgtctgctatcattattcgtagatattggacaaagaaccttctctatggggcatcctctttttccaacttggctgcaggaatctttaa
aagatgcttttaacagagtctgaacctatttcttaaacacttgcaacctacctgttgagcatcacagaatgtgataaggaaatcaacttgcttatcaa
cttcctaaatattatgagatgtggcttgggcagcatccccttgaactcttcactcttcaaatgcctgactagggagccatgtttcacaaggtctttaaa
gtgactaatggcatgagaaatacaaaaatactcagataaggtaaaatgccatgatgcctctgtcttctggactggttttcacattagaagacaatt
gacaacagttacataattcactctgagtgttttatgagaaagccttcttttggggtcaacagttttcctatgctttgaaacagaaaaatatgtaccaag
aatcttggtttgccttccagaaaacaaaactgcatttcactttcccggtgttccccactgtatctaggcaacatagtattcatgactatggataaacta
aacacgtgacacaaacacacacaaaagggaacccagctctaatacattccaactcgtatagcatgcatctgtttattctatagttattaagttcttt
aaaatgtaaagccatgctggaaaataatactgctgagatacatacagaattactgtaactgattacacttggtaattgtactaaagccaaacatat
atatactattaaaaaggtttacagaattttatggtgcattacgtgggcattgtctttttagatgcccaaatccttagatctggcatgttagcccttcctcca
attataagaggatatgaaccaaaaaaaaaaaaaaaaaa (SEQ ID No: 1).
 As provided herein, the long isoform of ADAM12 was particularly efficacious, under the conditions utilized, in distinguishing between tumor vasculature and healthy tissue (Example 20). In another embodiment, the ADAM12 nucleotide of methods and compositions of the present invention is a long isoform thereof. In another embodiment, the ADAM12 nucleotide is a short isoform. In another embodiment, the ADAM12 nucleotide is any other ADAM12 nucleotide known in the art. Each possibility represents a separate embodiment of the present invention.

An ADAM12 protein of methods and compositions of the present invention is, in another embodiment, a long isoform thereof. In another embodiment, the ADAM12 protein is a short isoform. In another embodiment, the ADAM12 protein is any other ADAM12 protein known in the art. Each possibility represents a separate embodiment of the present invention.

The cancer treated by a use of present invention is ovarian cancer.

In another embodiment of uses of the present invention, a subject having an ovarian tumor is contacted with a nucleotide molecule or antibody of the present invention. In another embodiment, an ovarian tumor is contacted with the nucleotide molecule or antibody of the invention.

A transmembrane (TM) protein may be accessible to antibodies and/or non-cell membrane-permeable agents and ligands. A plasma membrane-associated protein may be accessible to antibodies and/or non-cell membrane-permeable agents and ligands. A plasma membrane-associated protein may be a TM protein. The plasma membrane-associated protein may be an extracellular peripheral membrane protein. The plasma membrane-associated protein may be an intracellular peripheral membrane protein.

A TVM disclosed herein may be specific for vasculogenesis or associated with vasculogenesis. "Vasculogenesis" may refer to recruitment of endothelial progenitors of hematopoietic origin." orto de novo formation of tumor vasculature. The uses disclosed herein may be capable of detecting or localizing vasculogenesis or inhibiting vasculogenesis.

The TVM may be a secreted protein,an extracellular matrix (ECM) protein, a protein associated with the plasma membrane of the TVC, on the extracellular side. The TVM may be capable of shedding from the shed into a bodily fluid. It may be detected in a bodily fluid. The bodily fluid may be blood, lymph,saliva, sperm,cerebro-signal fluid, cervico-vaginal fluidor any other bodily fluid known in the art.

Each of TVM disclosed herein, refers, preferably, to a human TVM. Certain TVM are homologues of proteins known by a different name in another species, as indicated herein.

In another embodiment, the TVM of the present invention exhibit the advantage over tumor cell markers that TVC are genetically stable, relative to tumor cells; thus, TVC are much less likely to switch their expression of the TVM, thus evading localization, detection and therapeutic methods of the present invention. In another embodiment, the TVM of the present invention exhibit the advantage that tumor vasculature is significantly different than physiologic vasculature (for example, as demonstrated herein in Example 3). In another embodiment, the TVM of the present invention exhibit the advantage over tumor cell markers that TVC are more accessible via the bloodstream, relative to tumor cells; thus, TVC are more accessible for localization, detection and anti-tumor therapy by methods of the present invention. In another embodiment, a ligand that binds a TVM of the present invention is administered to a subject via the bloodstream. In another embodiment, the TVM of the present invention exhibit the advantage over tumor cell markers that the TVM are expressed on early-as well as late stage tumors. Each possibility represents a separate embodiment of the present invention.

In another embodiment, the therapeutic uses of the present invention exhibit the advantage that they can target any tumors above 2-3 mm in size, as tumors require vascularization to grow beyond this size. In another embodiment, the localization uses of the present invention exhibit the advantage that they can target any tumors above 2-3 mm in size, as tumors require vascularization to grow beyond this size; thus, these uses enable localization of small solid tumors. Each possibility represents a separate embodiment of the present invention.

In another embodiment, a nucleic acid molecule of the present invention is homologous to a nucleic acid molecule of SEQ ID NO: 1.

Homology is determined via determination of candidate sequence hybridization, methods of which are well described in the art (See, for example, "Nucleic Acid Hybridization" Hames, B. D., and Higgins S. J., Eds. (1985); Sambrook et al., 2001, Molecular Cloning, A Laboratory Manual, Cold Spring Harbor Press, N.Y.; and Ausubel et al., 1989, Current Protocols in Molecular Biology, Green Publishing Associates and Wiley Interscience, N.Y). For example methods of hybridization may be carried out under moderate to stringent conditions, to the complement of a DNA encoding a native caspase peptide. Hybridization conditions being, for example, overnight incubation at 42 °C in a solution comprising: 10-20 % formamide, 5 X SSC (150 mM NaCl, 15 mM trisodium citrate), 50 mM sodium phosphate (pH 7. 6), 5 X Denhardt's solution, 10 % dextran sulfate, and 20 µg/ml denatured, sheared salmon sperm DNA.

Disclosed is a kit comprising a reagent utilized in performing a method disclosed herein or a composition, tool, or instrument disclosed herein.

"Contacting," in another embodiment, refers to directly contacting the target cell with a composition of the present invention. In another embodiment, "contacting" refers to indirectly contacting the target cell with a composition of the present invention. Each possibility represents a separate embodiment of the present invention. In another embodiment, the composition of the present invention is carried in the subjects' bloodstream to the target cell. In another embodiment, the composition is carried by diffusion to the target cell. In another embodiment, the composition is carried by active transport to the target cell. In another embodiment, the composition is administered to the subject in such a way that it directly contacts the target cell. Each possibility represents a separate embodiment of the present invention.

### Pharmaceutical Compositions and Methods of Administration

Pharmaceutical compositions containing compositions of the present invention can be, in another embodiment, administered to a subject by any method known to a person skilled in the art, such as parenterally, paracancerally, transmucosally, transdermally, intramuscularly, intravenously, intradermally, subcutaneously, intra-peritonealy, intra-ventricularly, intra-cranially, intra-vaginally or intra-tumorally.

In another embodiment of uses of the present invention, the pharmaceutical compositions are administered orally, and are thus formulated in a form suitable for oral administration, i.e. as a solid or a liquid preparation. Suitable solid oral formulations include tablets, capsules, pills, granules, pellets and the like. Suitable liquid oral formulations include solutions, suspensions, dispersions, emulsions, oils and the like. In another embodiment of the present invention, the active ingredient is formulated in a capsule. In accordance with this embodiment, the compositions of the present invention comprise, in addition to the active compound and the inert carrier or diluent, a hard gelating capsule.

In another embodiment, the pharmaceutical compositions are administered by intravenous, intraarterial, or intra-muscular injection of a liquid preparation. Suitable liquid formulations include solutions, suspensions, dispersions, emulsions, oils and the like. In another embodiment, the pharmaceutical compositions are administered intravenously and are thus formulated in a form suitable for intravenous administration. In another embodiment, the pharmaceutical compositions are administered intra-arterially and are thus formulated in a form suitable for intra-arterial administration. In another embodiment, the pharmaceutical compositions are administered intra-muscularly and are thus formulated in a form suitable for intra-muscular administration.

In another embodiment, the pharmaceutical compositions are administered topically to body surfaces and are thus formulated in a form suitable for topical administration. Suitable topical formulations include gels, ointments, creams, lotions, drops and the like. In another embodiment, for topical administration, the compositions are prepared and applied as solutions, suspensions, or emulsions in a physiologically acceptable diluent with or without a pharmaceutical carrier.

In another embodiment, the active compound is delivered in a vesicle, e.g. a liposome.

In other embodiments, carriers or diluents used in uses of the present invention include, but are not limited to, a gum, a starch (e.g. corn starch, pregeletanized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, talc, or mixtures thereof.

In other embodiments, pharmaceutically acceptable carriers for liquid formulations are aqueous or non-aqueous solutions, suspensions, emulsions or oils. Examples of non-aqueous solvents are propylene glycol, polyethylene glycol, and injectable organic esters such as ethyl oleate. Aqueous carriers include water, alcoholic/aqueous solutions, emulsions or suspensions, including saline and buffered media. Examples of oils are those of animal, vegetable, or synthetic origin, for example, peanut oil, soybean oil, olive oil, sunflower oil, fish-liver oil, another marine oil, or a lipid from milk or eggs.

In another embodiment, parenteral vehicles (for subcutaneous, intravenous, intraarterial, or intramuscular injection) include sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Examples are sterile liquids such as water and oils, with or without the addition of a surfactant and other pharmaceutically acceptable adjuvants. In general, water, saline, aqueous dextrose and related sugar solutions, and glycols such as propylene glycols or polyethylene glycol are preferred liquid carriers, particularly for injectable solutions. Examples of oils are those of animal, vegetable, or synthetic origin, for example, peanut oil, soybean oil, olive oil, sunflower oil, fish-liver oil, another marine oil, or a lipid from milk or eggs.

In other embodiments, the compositions further comprises binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCl., acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hyroxypropylmethyl cellulose), viscosity increasing agents(e.g. carbomer, colloidal silicon dioxide, ethyl cellulose, guar gum), sweeteners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants. Each of the above excipients represents a separate embodiment of the present invention.

The compositions also include, in another embodiment, incorporation of the active material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts.) Such compositions influence, in another embodiment, the physical state, solubility, stability, rate of *in vivo* release, and rate of *in vivo* clearance.

The preparation of pharmaceutical compositions that contain an active component, for example by mixing, granulating, or tablet-forming processes, is well understood in the art. The active therapeutic ingredient is often mixed with excipients that are pharmaceutically acceptable and compatible with the active ingredient. For oral administration, the active agents are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions. For parenteral administration, the active agents are converted into a solution, suspension, or emulsion, if desired with the substances customary and suitable for this purpose, for example, solubilizers or other substances.

Each of the above additives, excipients, formulations and methods of administration represents a separate embodiment of the present invention.

In one embodiment, the term "administering" refers to bringing a subject in contact with an active compound of the present invention. In another embodiment, administration is accomplished *in vitro,* i.e. in a test tube. In another embodiment, administration is accomplished *in vivo,* i.e. in cells or tissues of a living organism. Each possibility represents a separate embodiment of the present invention.

In one embodiment, the uses of the present invention comprise administering an active composition or compound of the present invention as the sole active ingredient. However, also encompassed within the scope of the present invention are chemotherapy uses that comprise administering the active composition or compound in combination with one or more therapeutic agents (e.g. anti-tumor agents or cancer chemotherapy agents).

Insofar as the following Examples do not concern the ADAM12 TVM, they are comparative Examples.

### EXPERIMENTAL DETAILS SECTION

### EXAMPLE 1: CD31-BASED IMMUNO-LCM SUCCESSFULLY CAPTURES TUMOR VASCULAR CELLS, INCLUDING MYELOID CELLS

### MATERIALS AND EXPERIMENTAL METHODS

### Tissues

Stage-III epithelial ovarian cancer and ductal breast cancer specimens were collected at the University of Turin, Italy, following informed consent, from previously untreated patients. Additional ovarian cancer specimens, and normal ovaries were collected at the University of Pennsylvania Medical Center after obtaining written informed consent under Institutional Review Board (IRB)-approved protocols. Malignant mesothelioma (n=3), non-small cell lung carcinoma (n = 3) (provided by Dr. Steven M. Albelda) and malignant melanoma (n = 3) (provided by Dr. David Elder) were collected after obtaining written informed consent under IRB-approved protocols. A panel of normal
human tissues (Figure 3B) was provided by the Cooperative Human Tissue Network. All specimens were processed in compliance with HIPAA requirements.

### Reagents

Antibody against human CD31 (BD Pharmingen) followed by secondary antibodies (Vector, Burlingame, CA) were diluted (1:10) in PBS containing RNA Protector (1:10, Roche, Basel, Switzerland). Streptavidin conjugate and AEC chromagen (Dako, Carpenteria, CA) were diluted in PBS containing RNA Protector. Laser Capture Microdissection (LCM) was performed using Microcut (MMI, Glattbrugg, Switzerland), employing less than three hours per slide.

### RNA isolation

In order to increase RNA yield, dissected samples were treated with pre-digested proteinase-K. RNA was isolated using TRIzol reagent microprotocol (Gibco, Carlsbad, CA). Glycogen carrier (20 µg) was utilized to increase RNA yield in all protocols. RNA integrity and quantity were assayed using the Bioanalyzer (Agilent, Foster City, CA).

### RNA amplification

RNA was amplified using Messageamp® (Ambion, Austin, TX), with the following modifications: First-strand synthesis was performed at 42 °C (2 hours), then 48 °C (10 min). After second-strand synthesis, RNA was transcribed at 37 °C (12 hours); T7-polymerase and RNAse inhibitor were added and transcription was continued for 12 more hours. After 2 rounds of amplification, cRNA was biotin-labeled (12-24 hours, ENZO RNA labeling kit, Farmingdale, NY) and purified using RNA cleanup (Qiagen, Valencia, CA).

### Array Analysis

Biotin-labeled cRNA was fragmented and hybridized to U 133 (HG-U133) Set arrays (Affymetrix, Santa Clara, CA) representing more than 39,000 transcripts were derived from approximately 33,000 well-substantiated human genes, and scanned.

### qRT-PCR

qRT-PCR was performed using primers to the 3' end of transcripts spanning intron-exon boundaries whenever possible (Table 3) for 35 cycles using Sybergreen® (ABI, Foster City, CA), with primers at 150 nM concentrations. Primers were 18-24 nucleotides and were designed to have a TM of 59-61 °C. All transcripts were confirmed using 3% agarose gel electrophoresis. Gene expression was normalized against β-actin in all studies unless stated otherwise.

### Immunostaining

For validation studies, immunohistochemistry (IHC) was performed using the VECTASTAIN ABC kit (Vector, Burlingame, CA). All primary antibodies were incubated for one hour. Immune-reaction was visualized with 3,3'-diaminobenzidine (Vector). All staining steps were performed at room temperature.

### In Situ Hybridization (ISH)

DIG-labeled antisense RNA probes were generated by PCR amplification of 500-600-bp products incorporating T7 promoters into the antisense primers, followed by *in vitro* transcription with DIG RNA labeling reagents and T7 RNA polymerase (Roche, Indianapolis, IN). For immunodetection, an HRP-rabbit anti-DIG antibody (GenPoint kit; DAKO) was used and biotinyl-tyramide was included to catalyze biotin deposition (Perkin Elmer, Boston, MA). A second round of amplification utilized anti-biotin-HRP linked antibody (DAKO) followed by biotinyl-tyramide. Biotin was detected using a streptavidin-Cy3 conjugate (Arcturus) at 1:200 for 5 minutes. Slides were then washed and mounted with fluorescent mounting media containing DAPI (Vector).

### Cells

Human PBMCs were obtained through leukapheresis from healthy donors and were cultured for 7 days in DC media supplemented with 800 IU/ml human GM-CSF and 400 IU of IL-4. Cells were collected on day 7, labeled with CFSE, and incubated in media enriched with recombinant human (rh)VEGF₁₆₅(60 ng/ml, Peprotech, Rocky Hill, NJ) with or without VEGF-A neutralizing antibody (R&D Systems, Minneapolis MN) for seven days. Human peripheral blood monocytes were collected through elutriation. This population is highly enriched (greater than 95%) for CD14⁺ monocytes. Cells were processed exactly as above, treating them with GM-CSF and IL-4 for 7 days and with rhVEGF-A with or without VEGF-A neutralizing antibody for 7 additional days.

Endothelial progenitor assays were performed using the Endocult® assay (Stem Cell Technologies, Vancouver, Canada).

Human CD45⁺VE-cadherin⁺vascular leukocytes and CD45⁻VE-cadherin⁺tumorendothelial cellswere sorted from mechanically dispersed ovarian cancers by FACS performed with MoFlo Cell Sorter (Cytomation, Fort Collins, CO) using a FITC-labeled rabbit anti-human VE-cadherin antibody (Medsystems Diagnostics GmbH, Vienna, Austria) and APC-anti-CD45 (HI30; BD Pharmingen, San Diego, CA). PBS containing 10% normal murine serum (Sigma, St. Louis, MO) and 25 mg/ml anti-mouse Fc receptor (2.4G2 BD Pharmingen) was added prior to incubation to avoid nonspecific binding.

### Mice and xenograft tumors

NOD/LtSz-SCID/β2-microglobulin knockout (NOD/SCID/β2M^{-/-}) mice were bred and maintained under conditions approved by the Animal Care Committee of the University of Pennsylvania. These mice lack adaptive immunity and exhibit a severe defect in NK cell function, permitting superiorengraftment of human leukocytes. Female mice were transplanted subcutaneously with 0.3 ml cold Matrigel® (BD Biosciences, Bedford, MA) containing 1 x 10⁶ 2008 cells alone or enriched with 1 × 10⁵ DC, VLC or TEC into bilateral axillae. Tumors were resected at 3 weeks, RNA was extracted by the TRIzol method and analyzed for tumor vascular markers by qRT-PCR.

### Flow cytometry

DCs generated *in vitro* from PBMC or monocytes, and CFSE-labeled DC treated *in vitro* with VEGF-A were subjected to flow cytometry, using FACSCanto® flow cytometer and CellQuest 3.2.1fI software (both Becton Dickinson). These studies used biotin anti-VE-cadherin antibody (Medsystems Diagnostics GmbH, Vienna, Austria) coupled with streptavidin-PE-Cy7, APC anti human CD45 antibody and IgG control (BD Pharmingen).

### Bioinformatic and statistical analyses

Statistical significance for mRNA expression differences between tissue types was determined using a two-tailed Student's t-Test. Pearson's correlation was used to determine linearity of arrays performed with one versus two rounds of amplification or before and after immuno-LCM. Analyses of expression profiles were performed using Genespring software (Agilent); all samples were normalized with the median defined as 1.0. A heat map condition tree was developed using a hierarchical clustering algorithm (Genespring®) excluding all genes where the difference between the means of the tumor and normal vascular samples was less than its standard error. Descriptive statistics were performed with the SPSS® statistics software package (SPSS, IL, USA). The algorithm for the nonparametric method based on the ranks of the expression level for tumor and normal samples was developed in SAS 9.1.

### Optimization of immunostaining

To procure highly purified tumor vasculature, a rapid and reliable immuno-LCM protocol was established for microarray applications. Different fixation conditions were tested, including -20 °C acetone; 70% ethanol:10% acetic acid (1:1, vol:vol); methanol; or 4% paraformaldehyde. Fixation with acetone or ethanol-based fixatives resulted in the greatest RNA yield (Figure 2A). Acetic acid:ethanol fixation did not enable optimal IHC visualization of select target proteins. Acetone fixation was chosen for all further experiments.

Next, immunostaining was optimized. RNA isolated from tissue sections after standard IHC using LSAB (Dako) or Vectastain (Vector) showed near-complete degradation (Figure 2B-6). We thus developed an ultra-fast IHC protocol with increased concentrations of reagents. High concentrations of RNAse inhibitor were added to all aqueous solutions. The choice of RNAse inhibitor was critical for RNA integrity. RNA Protector® (Roche) was found to be superior to placental RNAse inhibitor (Stratagene) or SuperRNAsin® (Ambion), leading to two-fold increase in RNA yield and integrity (Figure 2B). Combining RNAse inhibitors reduced the efficacy of RNA Protector. Addition of RNA Protector to IHC allowed for 90% preservation of RNA integrity, based upon comparison of ribosomal RNA ratios determined using the Agilent Bioanalyzer.

Next detection systems were compared. AEC chromagen resulted in 40% greater RNA yield than DAB. Immunofluorescence resulted in 100% increased RNA yield compared to AEC (Figure 1C), but contaminating cells were poorly identifiable without counterstaining, as assessed by qRT-PCR detection of the T-cell marker CD3-ε. Furthermore, fluorescence quenching limited LCM time. Thus, AEC IHC was used for subsequent experiments.

In addition, the effect of LCM time on RNA yield and integrity was examined. Leaving immunostained tissue sections at room temperature for up to three hours before RNA was isolated had no significant impact on the quality or quantity of RNA isolated (Figure 1 D).

### Optimization of RNA purification.

RNA amplification methods (Arcturus Picopure kit, Stratagene microRNA isolation kit, Zymo mini RNA isolation kit and the modified TRIzol method for less than 10⁵ cells) were compared for RNA yield and quality after immuno-LCM. Arcturus Picopure gave the highest RNA yield for tissues stained with hematoxylin alone, but not following IHC (Figure 2E). The protocol from ZYMO also resulted in low RNA yield. Conversely, the Stratagene micro RNA isolation kit and the modified TRIzol method gave significantly better and similar yields by quantification with the Agilent Bioanalyzer.

RNA quality was tested by qRT-PCR of GADPH and β-actin transcripts in total RNA procured from 1 x 10⁸ cells microdissected and processed as in Table 2. GADPH or β-actin transcripts were detected at similar levels in RNA isolated with the modified TRIzol method using phase-lock tubes (Eppendorf, Hamburg, Germany) or with the Stratagene micro RNA isolation kit. Arcturus picopure and ZYMO RNA isolation kits were 10-fold and 256-fold less sensitive, respectively (Figure 2F).

The resulting protocol, requiring approximately 25 minutes for IHC, proved successful for numerous antibodies (Table 1). While some antibodies required longer incubation times (up to 15 minutes), there was no loss of RNA yield or integrity. Staining was quite specific, even with high concentrations of antibody. The protocol was reproducibly able to capture 500,000 µm² of tumor vascular cells in 3 hours of microdissection and recover -20 ng total RNA. RNA was reproducibly amplified to 15 µg of biotin-labeled cRNA.

### Optimization of RNA amplification

The linearity of amplifications using Ambion MessageAmp was tested by comparing the gene expression profile of 10 µg unamplified whole tumor RNA to amplified 6, 24 or 60 ng of the same RNA. Transcriptional profiling was performed using Affymetrix U133 chips. Correlation between unamplified RNA and 24 or 60 ng input RNA was high (r²=0.93 and 0.94, respectively) (Figure 2G). Correlation was lower with 6 ng input RNA (r²=0.87). High correlation was found between gene expression profiles from amplifications of input. RNA procured from the same tumor performed within the same experiment (intra-assay, r²=0.99) or in different experiments (inter-assay, r² =0.97). Immuno-LCM had no impact on expression profile (Figure 1C).

**Table 1. List of antibodies tested, company and clone used in the study as indicated. Success with staining using the rapid IHC protocol for LCM following fixation in acetone or acetic acid/ethanol**

| **Antibody** | **Company** | **Clone** | **Acetone** | **AA / EtOH** |
|---|---|---|---|---|
| Biot hCD45 | BD Pharm | H130 | *** | -- |
| Biot hCD31 | Ancell | 158-2B3 | *** | ** |
| Biot hCD31 | Caltag | MBC 78.2 | ** | ** |
| hCD31 | Dako | JC70A | ** | ** |
| Biot CD146 | Chemicon | MAB16985B | *** | *** |
| CytoKeratin | Dako | AO575 | * | -- |
| Biot hCD3 | BD Pharm | UCHT1 | *** | -- |
| Fitc hCD31 | BD Pham | WM59 | *** | *** |
| SMA-α-Cy3 | Sigma | 1A4 | ND | ND |
| FOLH1 | Zymed | ZMD.80 | ND | ND |
| STC2 | Genway | A22017 | ND | ND |
| Biot CD74 | BD Pharm | Mb741 | ND | ND |
| AML-1 | Active Motif | Polyclonal | ND | ND |
| hCD34-PE | BD Pharm | 581 | ND | ND |
| F-Spon | Abcam | Ab14271-50 | ND | ND |
| Lrp4 | Abcam | Ab13388-25 | ND | ND |
| Endothelial Lipase | Cayman Chemical | Polyclonal | ND | ND |

| | | | | |
|---|---|---|---|---|
| (AA/EtOH) is reported on the side for tested antibodies. (--), poor stain, (*) fair stain, (**) good stain, (***) excellent stain. ND, not determined. | | | | |

The optimized Immuno-LCM protocol is summarized in Table 2.

**Table 2. Summary of Immuno-LCM Protocol**

| | |
|---|---|
| Tumor | Freshly cut 8 µm sections of snap frozen tumor |
| IHC** | Fix in -20°C Acetone - 4 min |
| | Incubate with primary Ab 1 :10 - 5 min |
| | Incubate with 3x biotinylated anti-mouse Ab (Vector) - 5 min. |
| | Brief wash in PBS |
| | 2.5X Streptavidin-biotin amplification (DAKO) - 5min |
| | Brief wash in PBS |
| | AEC (DAKO) stain - 3 to 5 min |
| | Brief wash in PBS |
| | Stain with dilute hematoxylin |
| | Rinse |
| | (** All steps with 1:10 RNAse Protector) |
| LCM | Dry tissue sections with hair dryer - 1 min |
| | Microdissect cells - up to 3 hours |
| RNA isolation | Treat with Proteinase K (10 µg/ml) - 8 min |
| | Extract RNA with TRIzol in phase lock gel - 1 hour |
| RNA Amplification | Use Ambion MessageAmp |

During the optimization of RNA isolation and amplification methodology, it was found that the immuno-LCM procedure had minimal impact on RNA integrity (Figure 2A-F) or gene expression profiling (Figures 1C and 2G).

The PCR primers used for qRT-PCR and in situ hybridization are set forth in Table 3 below. A: qRT-PCR; B: T7 polymerase. (This table also includes primers used to amplify controls [those from CD3eps to GAPDH] and additional markers that do not appear in Table 5; selection of these additional markers is described below in Example 11).

**Table 3A**

| GENE | FORWARD PRIMER; SEQ ID | REVERSE PRIMER; SEQ D |
|---|---|---|
| Adlican 5' | cattgctagacacgtggaaaga; 212 | tctcattgccgtgaatcataag; 213 |
| Adlican 3' | aggaaccctcaagctggact; 214 | cattggcaaacaccttgattc; 215 |
| C11ORF8 | cttcaaagagtgggctgtgtg; 216 | gaggcattgatgtacgttgtg; 217 |
| CD31 | tttggctagtccttgttctttgg; 218 | cggtgtcttcaggttggtattc; 219 |
| Coll11□1 | agggtgaagatggagatcctg; 220 | tttcaccttgtcttccctctg; 221 |
| Cytokeratin 18 | gcgtacagagatggagaacgaat; 222 | cgagactccagctctaccttgtt; 223 |
| EGFL6 | agaagaccacgagtgaggatg; 224 | ccacgttctgcttcaaaaatg; 225 |
| F2RL1 | tgctagcagcctctctctcc; 226 | tttccagtgacgtgggatg; 227 |
| FJX1 | actacctgacggccaacttc; 228 | cgcctcattgtccagaaag; 229 |
| FZ10 | cagactaaaacgctggactgc; 230 | tcttggaggtccaaatccac; 231 |
| GAPDH | cctgcaccaccaactgctta; 232 | catgagtccttccacgatacca; 233 |
| GPM6B | tcctatcacctgttcattgtgg; 234 | gcagcaatcttcccgactc; 235 |
| LZTS1 | agggagaagaccagcttcg; 236 | aggatctcgctagccttgg; 237 |
| OLFML2B | ccaaaggatgagcggatttac; 238 | gctgtacgggagcttgtagg; 239 |
| □□SMA | actgagcgtggctattccttc; 240 | agtggccatctcattttcaaag; 241 |
| SLIT2 | gaaatctcttgtcgaggggaaag; 242 | acacccacctctgcactctaatc; 243 |
| SPON-1 | agtgagcagctgaaggaagagt; 244 | catgtaacgttcctgaattccac; 245 |
| STC2 | tacctcaagcacgacctgtg; 246 | gtcagcagcaagttcacgag; 247 |
| TNF□IP6 | cgcaacttacaagcagctagag; 248 | taatgccagtttttccaaatcc; 249 |
| TNFRSF21 (DR6) | ggcttcttcgtggatgagtc; 250 | ccgcaacactgtgtccttc; 251 |

**Table 3B**

| Gene | FORWARD, ISH; SEQ ID | REVERSE, T7; SEQ ID |
|---|---|---|
| Adlican | caagatcgaggccagtatatgtg; 252 | gcaaacaccttgattctgctatc; 253 |
| CD31 | tgaggttctgagggtgaagg; 254 | ggatcctaatacgactcactatagggagattgggatctgac attttctcg; 255 |
| EGFL6 | agaagaccacgagtgaggatg; 256 | ggatcctaatacgactcactatagggagatgaggaggga gataaactgac; 257 |
| FJX1 | actacctgacggccaacttc; 258 | ggatcctaatacgactcactatagggagattcctcccggtga cactaag; 259 |
| FZ10 | cagactaaaacgctggactgc; 260 | ggatcctaatacgactcactatagggagatcctttcagtgtat cacagcatc; 261 |
| GPM6B | tcctatcacctgttcattgtgg; 262 | ggatcctaatacgactcactatagggagatgaaactggttat caggcaatc; 263 |
| TNF□IP6 | acggtctggcaaatacaagc; 264 | ggatcctaatacgactcactatagggagaactgatgatgtc atctggaagc; 265 |
| TNFRSF21 | ggcttcttcgtggatgagtc; 266 | ggatcctaatacgactcactatagggagacacacacacac accccaaac; 267 |

### RESULTS

A rapid protocol was developed and optimized for immuno-LCM, followed by extraction and amplification of RNA for array analysis of tumor vascular cells (TVC). 4 x 10³ CD31⁺ cells with vascular morphology were isolated (Figure 1A). RT-PCR analysis of RNA from purified TVC confirmed the absence of lineage-specific markers CD3-ε and CD5 (T-cells); CD16 (NK cells); CD19 and CD20 (B-cells); and cytokeratin-18 (tumor cells) (Figure 1 B). In addition, expression of the myeloid markers CD45, CD11c and CD14 was detected, confirming that TVC had been isolated. These findings were further confirmed by quantitative real-time polymerase chain reaction (qRT-PCR), which revealed a 15-fold enrichment of CD31 mRNA, similar expression of monocyte markers, and absence of cytokeratins and CD3-ε in micro-dissected TVC specimens relative to whole tumor. Smooth muscle α-actin was detectable in only 50% of samples and at 10-fold reduced levels relative to whole tumor, showing that few, in any perivascular cells were present.

Thus, the method successfully captured vascular cells, including vascular cells of monocytic lineage.

### EXAMPLE 2: mRNA PROFILE OF IMMUNO-LCM PROCURED TUMOR VASCULAR CELLS

The mRNA profiles of micro-dissected TVC from 2 tumor samples were analyzed in parallel to 3 micro-dissected normal ovary vascular cells and to cultured human umbilical vein endothelial cells (HUVEC) using Affymetrix-U133 arrays. 13/13 known pan-endothelial markers in were detected in the TVC arrays. Similarly, 14/15 known tumor endothelial-specific markers (those listed below, Tem-4, and Tem-9) were exclusively expressed or markedly overexpressed (p<0.001) in TVC arrays (Table 4). These findings indicate that the protocol exhibits a sensitivity of greater than 90%.

**Table 4. Expression of pan-endothelial and tumor endothelial markers in HUVEC; endothelial cells from normal ovary isolated through immuno-LCM (Normal); and tumor endothelial cells from ovarian cancer specimens isolated through immuno-LCM (Tumor1 and Tumor2). (+) indicates detection of the gene with all associated probe sets, when more than one probe set exists; (+/-): detection with one but not all probe sets associated with the gene; (--): lack of detection with any associated probe sets for the indicated gene.**

| **Pan Endothelial Markers** | **HUVEC** | **Normal** | **Tumor1** | **Tumor2** |
|---|---|---|---|---|
| Angiomodulin | + | + | + | + |
| Hevin | + | + | + | + |
| Connective tissue growth factor | + | + | + | + |
| Collagen, type VI, alpha 1 | + | + | + | + |
| Interferon induced transmembrane protein 1 | + | + | + | + |
| Guanine nucleotide binding protein 11 | + | + | + | + |
| Von Willebrand factor | + | + | + | + |
| SmiLIM | + | + | + | + |
| Collagen, type XVIII, alpha 1 | + | + | + | + |
| Insulin-like growth factor-binding protein 4 | + | + | - | + |
| P1H12 (CD146) | +/- | +/- | +/- | +/- |
| Osteonectin | + | + | + | + |
| Matrix Gla protein | + | + | + | + |
| | | | | |

| **Tumor Endothelial Markers** | **HUVEC** | **Normal** | **Tumor1** | **Tumor2** |
|---|---|---|---|---|
| TEM-1 | -- | + | + | + |
| TEM-3/ TEM-7 | -- | + | + | + |
| TEM-5 | +/- | +/- | +/- | + |
| TEM-6 | + | + | + | + |
| TEM-8 | -- | -- | -- | -- |
| MMP-11 | -- | -- | +/- | + |
| Gelatinase A | +/- | +/- | + | + |
| Nidogen | + | -- | +/- | + |
| Collagen, type VI, alpha 3 | -- | + | + | + |
| Thy-1 cell surface antigen | -- | | + | + |
| Collagen, type III, alpha 1 | + | + | + | + |
| Bone morphogenetic protein 1 | -- | -- | +/- | + |
| Cystatin S | + | -- | -- | - |
| Macrophage mannose receptor family | +/- | +1- | +/- | +/- |

Next, vascular cells from 21 stage-III ovarian tumors and 4 normal ovaries were microdissected, profiled and analyzed. In order to determine if there was a distinct signature that could distinguish tumor from normal vasculature, unsupervised hierarchical clustering was performed using 17,920 genes (after elimination of all genes wherein the difference between tumor and normal means was less than the standard error of the difference in the means). Hierarchical clustering of these samples accurately classified tumor from normal vascular cells, demonstrating a clear difference in the molecular profile between tumor and normal vasculature and further confirming the validity and accuracy of the method used (Figure 1D).

To identify robust, tumor vasculature-specific genes, the 17,920 genes were further characterized using (1) the fold difference comparing tumor to normal vascular samples; (2) the number of tumor and normal vascular samples where a gene was classified as "present" or "absent" by MAS5.0 Suite (Affymetrix); and (3) the gene-specific ranks of the normalized gene expression values for all samples. 70 genes were selected as tumor vascular markers (TVM) that fulfilled at least 1 of the following 3 criteria: (1) present in more than 15/21 TVC samples and absent in more than 3/4 normal vascular samples; (2) present in more than 18/21 tumor vascular samples and at least 3-fold up-regulated on average in tumor vascular samples; or (3) at least 3/4 normal samples had 1 of the 5 lowest ranked expression values (therefore at least 20/21 tumor vascular samples had higher expression values relative to normal samples) (Table 5). By this method, a number of transcripts previously not known to be TVM were identified, e.g. adlican, GPM6B, TNFaIP6, LZTS1 and numerous expressed sequence tags (EST). The identified markers also included several known TVM (FoIH1 (PSMA), Thy-1, TEM-7, SLIT2, and chondroitin-sulfate proteoglycan-2 (versican)), further validating the methods used to identify TVM.

**Table 5, part 1. List of 70 TVM identified by three independent analyses. Genes were included in the list if they fulfilled tumor vascular marker criteria in at least one of the analyses. The table describes results from all analyses for each gene. First column lists genes reported by common gene name and ESTs reported by Genebank admission; second column describes expression fold change in tumor relative to normal vascular cells; third column describes the number of tumor (total of 21) and normal (total of four) vascular cell specimens for which the gene was determined to be expressed; fourth column describes the expression rank of the four normal vascular cell samples (with 1 being the lowest expression and 25 the highest).**

| **Common Gene Name or Genebank No.** | **Fold Change Tumor vs. Normal** | **No. Present in** | | **Rank of Normal Specimens** |
|---|---|---|---|---|
| | | **Tumor [21]** | **Normal [4]** | |
| ADAM12 | 5.1 | 13 | 0 | 1,2,4,6 |
| Adlican | 5.4 | 19 | 2 | 1,2,5,8 |
| Al798118 | 7.7 | 15 | 0 | 1,2,3,5 |
| AK000168 | 3.0 | 17 | 1 | 4,6,7,15 |
| AML-1 | 30 | 12 | 0 | 1,2,3,5 |
| ANKRD9 | 3.2 | 16 | 1 | 1,4,7,16 |
| Av758342 | 4.0 | 17 | 2 | 1,2,9,10 |
| AW242409 | 4.9 | 17 | 2 | 1,3,5,9 |
| AW510657 | 3.9 | 18 | 4 | 4,5,6,7 |
| AW517716 | 7.4 | 12 | 0 | 1,2,3,13 |
| BDKRB2 | 3.7 | 15 | 1 | 2,3,5,11 |
| C6orf37 | 5.2 | 15 | 1 | 2,3,5,7 |
| C6orf55 | 2.4 | 18 | 1 | 1,2,3,8 |
| C6orf69 | 2.0 | 19 | 3 | 1,2,4,7 |
| C7orf32 | 3.4 | 16 | 1 | 1,2,4,7 |
| C11 orf8 | 4.08 | 13 | 1 | 1,2,5,17 |
| CD24 | 3.7 | 16 | 1 | 2,6,7,8 |
| CDS1 | 3.7 | 15 | 2 | 1,2,3,8 |
| CENTA2 | 2.1 | 16 | 3 | 1,3,4,5 |
| CLDN1 | 2.4 | 16 | 1 | 1,2,3,13 |
| COL11A1 | 10.1 | 15 | 0 | 1,2,7,8 |
| COL15A1 | 4.8 | 21 | 4 | 1,3,8,12 |
| COL22A1 | 3.6 | 11 | 0 | 1,2,3,5 |
| CSPG2 | 5.8 | 21 | 4 | 1,2,3,6 |
| DEFB1 | 2.4 | 11 | 0 | 1,2,4,13 |
| DSG2 | 4.2 | 11 | 1 | 2,3,15,17 |
| EGFL6 | 4.6 | 19 | 4 | 1,2,6,7 |
| EIF2AK4 | 2.8 | 18 | 1 | 1,2,4,9 |
| EBP41L3 | 4.0 | 16 | 2 | 1,4,5,13 |
| ESM1 | 3.7 | 20 | 1 | 1,4,5,7 |
| FBXO32 | 2.2 | 16 | 1 | 1,2,5,8 |
| FJX1 | 11.3 | 16 | 0 | 1,2,3,5 |
| FOLH1 | 8.8 | 13 | 1 | 1,2,4,6 |
| FLJ22875 | 2.3 | 16 | 0 | 2,3,6,7 |
| FLJ46072 | 3.8 | 20 | 1 | 1,2,3,5 |
| Fz2RL1 | 3.6 | 13 | 0 | 1,7,10,12 |
| FZD10 | 2.0 | 15 | 0 | 2,3,6,10 |
| GPM6B | 14.63 | 16 | 0 | 1,2,5,6 |

**Table 5, part 2.**

| | | | | |
|---|---|---|---|---|
| GPR105 | 3.3 | 11 | 0 | 1,2,6,10 |
| HOXB7 | 2.8 | 16 | 1 | 1,2,7,14 |
| IGSF4 | 6.0 | 18 | 1 | 1,4,5,6 |
| IVNS1ABP | 3.3 | 21 | 4 | 1,2,3,5 |
| KCNK5 | 2.5 | 16 | 1 | 1,3,5,9 |
| LZTS1 | 4.3 | 19 | 1 | 1,2,6,7 |
| MCM4 | 2.4 | 13 | 0 | 1,2,4,6 |
| ND2 | 4.1 | 16 | 1 | 1,4,8,15 |
| OlfML2B | 2.4 | 18 | 1 | 1,2,4,11 |
| OSSPL3 | 4.1 | 17 | 1 | 1,3,4,20 |
| PCDH17 | 2.9 | 21 | 4 | 1,3,4,7 |
| PLCE1 | 3.8 | 13 | 1 | 1,3,4,16 |
| PLXDC1 | 4.0 | 21 | 3 | 1,3,5,8 |
| PRDM1 | 2.4 | 15 | 0 | 1,3,4,5 |
| RGC32 | 9.0 | 16 | 2 | 1,3,6,8 |
| RGS1 | 8.0 | 12 | 1 | 2,3,5,16 |
| RRM2 | 4.7 | 16 | 1 | 2,4,5,21 |
| SCNN1A | 15.8 | 16 | 0 | 1,2,3,6 |
| SCO2 | 4.42 | 20 | 2 | 1,2,6,10 |
| Slam F8 (BLAME) | 3.5 | 13 | 0 | 2,3,6,8 |
| SLIT2 | 2.0 | 16 | 1 | 3,4,6,9 |
| SPON1 | 2.5 | 16 | 1 | 1.,6,7,17 |
| SPRY4 | 2.8 | 20 | 2 | 1,2,3,5 |
| STC2 | 9.0 | 11 | 0 | 1,2,4,5 |
| STCH | 4.0 | 15 | 2 | 1,6,7,9 |
| TACSTD1 | 11.6 | 16 | 2 | 1,2,7,10 |
| TARS | 2.8 | 16 | 1 | 2.5-9.11 |
| TBX2 | 2.2 | 14 | 1 | 1,5,6,24 |
| THY1 | 3.1 | 19 | 3 | 1,2,4,8 |
| TNFAIP6 | 5.6 | 17 | 0 | 1,2,3,6 |
| TNFSFR21 (DR6) | 8.2 | 11 | 0 | 1,3,4,7 |
| UPP1 | 4.97 | 15 | 1 | 1,3,5,20 |

The sequence of meltrin-L precursor (ADAM12) is listed above as SEQ ID NO: 1. Exemplary GenBank Accession Numbers for this gene are AF023476 and NM_003474.

### EXAMPLE 3: FURTHER VALIDATION OF TVM

To test the specificity of identified genes to tumors relative to normal tissues, previously uninvestigated genes were selected from the above candidates for further validation. As positive controls for our assays, known TVM were included. First, 12 selected TVM were tested for enrichment of expression in ovarian tumors versus normal ovarian tissue, by analyzing their whole tissue expression by qRT-PCR in an independent set of stage-III ovarian cancers (n=20) and normal ovaries (n=5). All 12 TVM tested were upregulated in cancer tissue versus normal ovaries (p<0.05 for all). Many TVM were expressed at or below the lowest limits of detection in normal ovaries (Figure 3A). TVM expression in normal tissues was also examined by analyzing pre-existing SAGE data (Boon K et al, An anatomy of normal and malignant gene expression. Proc Natl Acad Sci USA 2002 Aug 20;99(17):11287-92.) (Figure 3E). Genes exhibiting no or minimal *in silico* expression in normal tissues were then screened in a panel of normal tissues with qRT-PCR (Figure 3B). All TVM tested, except for C11orf8 and FJX1, were highly expressed in tumors and were expressed at or below the lowest limits of detection in normal tissues tested. C11 orf8 was significantly enriched in TVC relative to all cell types tested, and was expressed at or below the lowest limit of detection in all cell types except brain and spleen. FJX1 was significantly enriched in TVC relative to all cell types tested except for spleen.

Quantitative Real-time Polymerase Chain Reaction (qRT-PCR) was used to evaluate the expression of select TVM in different malignancies, including lung cancer, mesothelioma, breast cancer and melanoma (Figure 3C). To ensure expression differences were not due to differences in vascular density, the qRT-PCR data were normalized against CD31 expression, a reliable marker of endothelial cells. Collagen 11α1 was expressed at even higher levels in breast and lung relative to ovarian tumors, LZTS1 was relatively specific for melanoma and ovarian cancer, and FZD10 and EMBPL1 was relatively specific for ovarian tumors. Other TVM exhibited quantitative differences in expression among different tumor types, indicating that, under the conditions utilized, TVC signatures differ among tumor types.

In addition, TVM that exhibited minimal expression in normal tissues were evaluated for expression in tissues with physiologic angiogenesis including corpus luteum, proliferative endometrium and placenta. Data were normalized against CD31 expression. Some of the TVM (adlican, collagen 11α1, F2RL1, GPM6B and STC2) were expressed at 40-350 fold higher levels in tumor versus tissues with physiologic angiogenesis (Figure 3D). However, EGFL6, OLFML2B and previously established TVM such as TEM-1 and TEM-7 were expressed in reproductive tissues with physiologic angiogenesis.

These findings further validated the identified proteins and transcripts as TVM. Expression of the TVM in other tumors besides ovarian cancer show that their utility (e.g. in tumor localization and diagnostic and therapeutic methods) is not limited to ovarian tumors, but rather includes many other solid tumors. In addition, these findings validated the methods used in the present invention for identifying TVM, with regard to both those TVM tested *in vivo* and those not yet tested *in vivo.*

### EXAMPLE 4: LOCALIZATION OF TVM EXPRESSION TO TUMOR VASCULATURE

To confirm that genes identified through immuno-LCM and transcriptional profiling were localized to ovarian cancer tumor vasculature, immunohistochemistry (IHC) was performed. Both CD24 (Figure 4) and CD74, normally expressed by hematopoietic cells of the monocytic/macrophage lineage, but also reported as expressed in endothelium, were clearly expressed in ovarian tumor vessels. Endothelial lipase and FoIlH1, genes previously identified as expressed in tumor vasculature, were also expressed in ovarian tumor endothelium. SPON-1 was also found to be expressed in a vascular pattern. PAR2 (F2RL1) was localized primarily in peri-endothelial location, while STC2 co-localized with CD31 expression. AML-1 was expressed in stromal cells, but also in vascular structures by IHC (Figure 4, second row). In addition, AML-1 expression was only detected in small tumor vessels, but was absent in large, smooth muscle actin-positive, mature vessels. Double immunofluorescence indicated that AML-1 co-localized with CD31 expression (Figure 4, last row); localization in adjacent cells could not be excluded, however.

For TVM against which antibodies were unavailable, expression in tumor vasculature was confirmed using *in-situ* hybridization (ISH). ISH revealed clear expression of GPM6B, adlican, TNF□IP6, FZD10, EGFL6, DR6 and FJX1 in 5 independent ovarian cancer samples (Figure 5). TVM were detected in vascular structures and were absent in tumor cells. Expression was generally limited to small neo-vascular structures, and with the exception of FZD10, was absent in large, muscular vessels. With the exception of FJX1, which was detected at very low levels in normal ovarian vasculature, TVM were not detected in 3 independent normal ovary specimens. No specific staining was observed with sense strand RNA controls, showing that the signals observed accurately reflected expression of the RNA molecules measured. Pre-treatment of slides with RNAse prior to probe hybridization eliminated all specific staining for all TVM tested, providing an additional control for veracity of the data.

These findings confirm that the identified TVM were expressed in tumor vasculature.

### EXAMPLE 5: TVM EXPRESSION IN DIFFERENT TVC

To test which cells in the tumor vasculature express the identified TVM, mRNA expression was evaluated by qRT-PCR in VE-cadherin⁺ CD146⁺ CD45⁻ tumor endothelial cells (TEC) isolated freshly by FACS from ovarian cancer specimens. TVM expression in VE-cadherin⁺CD146⁺CD45⁺ vascular leukocytes (VLC); myeloid cells with vasculogenic potential; and control peripheral blood mononuclear cells (PBMC) was also tested. All TVM were expressed by VLC, and all except AML-1 were expressed by purified TEC (Figure 6). Adlican, EGFL6, FJX1, FZD10, GPM6B and SPON-1 were expressed at approximately 10-fold higher levels in TEC than VLC. TEC expressed these genes at greater than 100-fold higher levels than PBMC. BCM-like membrane protein precursor (BLAME)was higher in VLC (and PBMC) than TEC, and TNF□IP6 was higher in TEC and VLC than PBMC. AML-1 demonstrated highest expression in PBMC and intermediate in VLC, while it was nearly absent in TEC. Thus, genes identified through immuno-LCM and transcriptional profiling are expressed by TVC, at different levels in TEC and VLC.

Thus, expression of most TVM tested in tumor endothelial cells provided still further evidence that the TVM identified were genuine, and that cell contamination did not occur during immuno-LCM.

### EXAMPLE 6: DENDRITIC CELL PRECURSORS UPREGULATE TVM UNDER THE INFLUENCE OF VEGF-A

To address the origin of human VLC, DC were generated from human PBMC with GM-CSF and IL-4 (Benencia F et al, HSV oncolytic therapy upregulates interferon-inducible chemokines and recruits immune effector cells in ovarian cancer. Mol Ther. 2005 Nov;12(5):789-802). FACS analysis demonstrated 100% CD45 expression in these cells (Figure 7A, top left). DC were then labeled with CFSE and treated with recombinant human (rh)VEGF₁₆₅ for 7 days. VEGF₁₆₅ induced significant up-regulation of VE-cadherin in a population of PBMC-derived DC (Figure 7A, top right). The monocyte fraction of PBMC was primarily responsible forgiving rise to the VE⁻cadherin⁺CD45⁺ population upon treatment with VEGF₁₆₅ (Figure 7A, middle). Lack of CFSE dye dilution with VEGF-A treatment indicated that the phenotype switch was not due to expansion of endothelial progenitor contaminants (Figure 7A, bottom). In addition, endothelial progenitor assays performed with these cells demonstrated no proliferation. Thus, under the conditions utilized, VEGF-A induces monocyte-derived DC to acquire VLC features.

To test whether VEGF₁₆₅ treatment induced TVM in human PBMC-derived DC, expression of collagen 11α1, DR6, FZD10, GPM6B, SCP1, SPON-1, and TEM-1 was measured, following VEGF₁₆₅ treatment. All these transcripts were markedly up-regulated, and VEGF-neutralizing antibody abrogated their up-regulation (Figure 7B). Expression of AML-1 and LZTS1 were down-regulated in DC by VEGF₁₆₅. This down-regulation was abrogated by anti-VEGF-A antibody in 3-day cultures but not after 2 weeks of VEGF₁₆₅ treatment. Other TVM, such as adlican and SLIT2, were not up-regulated in DC by VEGF₁₆₅ treatment, showing that, under the conditions utilized, other mediators besides VEGF-A were necessary to complete the full differentiation of these cells into VLC.

### EXAMPLE 7: DC PRECURSORS CONTRIBUTE TO TUMOR VASCULATURE AND EXPRESS A LARGER REPERTOIRE OF TVM IN VIVO

To determine whether additional TVC could be contributed by DC *in vivo,* human PBMC-derived DC were treated with rhVEGF₁₆₅ for one week *in vitro,* admixed with 2008 human ovarian cancer cells (1:10; DC, tumor cell) and transplanted within Matrigel® into NOD/SCID/β2M-/- recipient mice. As controls, 2008 cells were injected alone, or co-injected together with FACS-isolated TEC or VLC in the opposite side. Tumors were assessed for expression of TVM 3 weeks later using qRT-PCR. Three TVM, adlican, FJX1 and STC2, as well as known TVM, SLIT2 and TEM-1, were clearly detected in tumor xenografts enriched with human VEGF-treated DC, VLC or TEC, while they were not detected in tumors generated with tumor cells alone (Figure 7C). Immunofluorescence analysis to identify human endothelium using antibodies specific to human (h)CD34 and hCD31 identified small clusters of human vascular structures in tumors enriched with rhVEGF-treated DC, VLC or TEC (Figure 7D). No staining for human endothelium was identified when 2008 cells were injected alone. These cells exhibited markers and *in vivo* behavior that were similar to tumor-derived VLC, showing that certain tumor VLC derive from myeloid DC.

Induction of TVM by VEGF-A *in vitro* was not due to expansion of contaminating endothelial precursor cells, as shown by negative CFSE dilution and endothelial progenitor cell assays. When VEGF-treated DC were exposed to the tumor microenvironment in vivo, additional TVM could be induced, showing that, under the conditions utilized, *in vivo* factors further contribute to acquisition of a VLC phenotype by DC. Importantly, tumors enriched with VEGF-treated DC exhibited vascular structures expressing human CD31 and CD34, while control tumors generated with only tumor cells did not. Thus, tumor cells were not responsible for the expression of TVM in vivo.

Thus, human VEGF-primed monocyte-derived DC can give rise to vascular cells in tumors. These findings demonstrate that many of the novel TVM identified are expressed by vascular cells of myeloid lineage.

### EXAMPLE 8: IDENTIFICATION OF ADDITIONAL TRANSMEMBRANE AND SECRETED TVM

The tumor vasculature-specific genes identified in Example 2 were further analyzed by t-statistics, ranked and nonparametric analyses, and Genespring®. Over 200 genes were identified that were either exclusively expressed in tumor ECs or significantly overexpressed in tumor ECs and absent in 75% of normal ECs. From among these genes, transcripts were then identified that encoded proteins that were transmembrane or secreted, based on known structure or sequence analysis. 108 such transcripts were identified, and are listed and described in Figure 8.

### EXAMPLE 9: FURTHER TESTING OF TRANSMEMBRANE AND SECRETED TVM

### MATERIALS AND EXPERIMENTAL METHODS

Cell-free, *in vitro* rapid translation system® (RTS) technology (Roche Applied Science) is used to produce whole proteins or polypeptides derived from extracellular regions.

### RESULTS

The new transmembrane and secreted TVM identified in Example 8 are tested for enrichment of expression in ovarian tumors versus normal ovarian tissue and in different malignancies and tissues with physiologic angiogenesis, as described in Example 3. In other experiments, frozen specimens are used. In other experiments, paraffin-embedded specimens are used. Alternatively, the new TVM are used to localize ovarian cancer tumor vasculature using IHC or ISH, as described in Example 4. In another embodiment, double immuno-staining for CD31 in parallel is used to show co-localization in the endothelium. In another embodiment, antibodies raised against the proteins, or against extracellular fragments thereof, are utilized. In another embodiment, the antibodies are generated using methods described herein. In another embodiment, nucleic acids that hybridize with the identified transcripts are utilized. These experiments confirm that the identified transcripts, and the proteins they encode, are robust TVM.

### EXAMPLE 10: USE OF ADLICAN AS A TUMOR MARKER IN VIVO

### MATERIALS AND EXPERIMENTAL METHODS

Anti-Adlican antibody was produced by Prosci Inc., Poway, CA.

### RESULTS

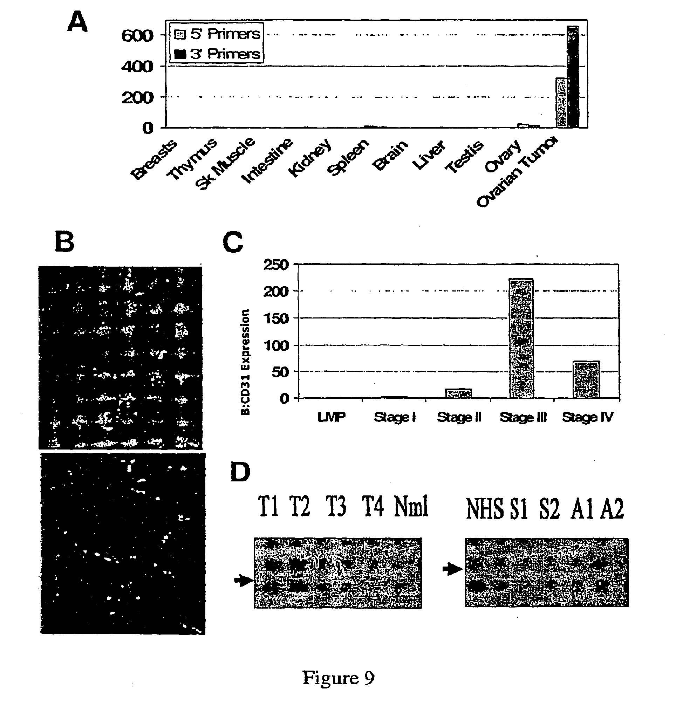

One extracellular matrix marker identified, adlican, was cloned, a fragment of it (CPGAKALSRVREDIVEDE; SEQ ID No: 88) was expressed, and rabbit anti-adlican antiserum was produced and used to screen normal and tumor tissue samples. Adlican was detected by immuno-staining in tumor but not in normal ovary stroma or vasculature. In addition, the antiserum was used to screen serum and ascites of patients with stage III ovarian cancer, and control healthywomen. Adlican was detected by Western blot in the tumor samples, but not the control samples (Figure 9D).

Thus, TMV of the present invention are efficacious in detecting, localizing, and specifically delivering drugs or anti-tumor agents to tumor cells. In addition, these results showthat certain secretoryTVM of the present invention are shed into the circulation, where they can function as tumor biomarkers. Thus, tests of biological fluids (e.g. blood, lymph, etc) can be successfully used to screen for tumors using TVM of the present invention.

### EXAMPLE 11: IDENTIFICATION OF ADDITIONAL TVM

### MATERIALS AND EXPERIMENTAL METHODS

Additional TVM were identified from the list of genes described in Example 1, according to the following criteria: (1) Genes which were determined to be present in 75% of tumor vasculature specimens and absent in normal vasculature; and (2) genes which were present in 75% of tumor vasculature specimens and greater than 5-fold upregulated versus normal ovarian endothelium.

### RESULTS

Additional TVM were identified.

*In vivo* expression analyses validated the identified markers, and DEFB1, IL10RA, ADAM12, PCDH17, F-spondin, TNFAIP6, BLAME, AML-1, HEVIN, EPB41 L3, SLIT2, and LZTS1, which also were identified in this analysis. Measurement of expression in normal ovarian tissue vs. tumor, and an array of normal tissues vs. tumors, performed as described in the above Examples, both validated each of these markers (Figure 3).

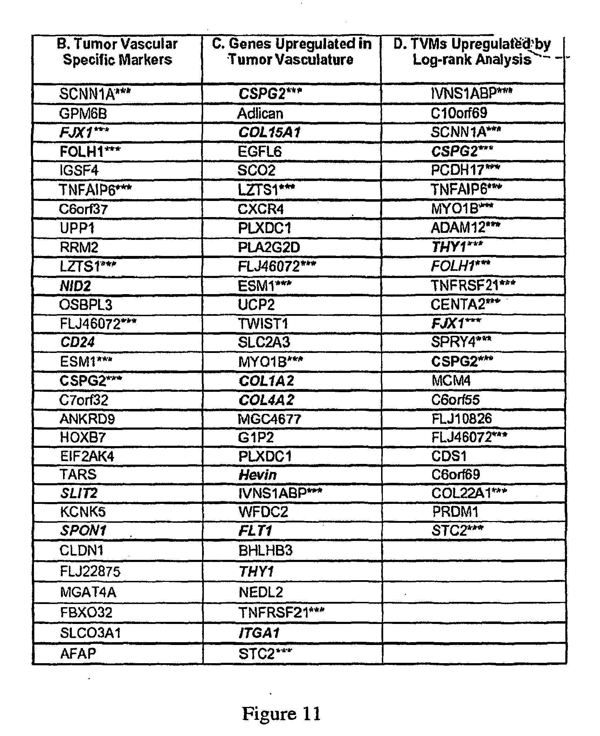

Additional TVM identified in the above analysis include MGAT4A, AFAP, CXCR4, UCP2, TWIST, SLC2A3, MYO1B COL4A2, MGC4677, G1P2, BHLHB3, NEDL2, and ITGA1 (Figure 11).

Thus, as in previous Examples, each of the markers tested in this Example are bona fide TVM.

### EXAMPLE 12: IDENTIFICATION OF ADDITIONAL TRANSMEMBRANE (TM) AND SECRETED TVM

Additional TVM were identified from the list of genes described in Example 1, according to the following criteria: A. For each gene, the individual rank values of the 5 normal endothelium samples were summed; the sum of ranks had to be less or equal to 25; (2) AND/OR the fold change (mean tumor to mean normal) had to be greater or equal to 3. From these genes, all genes with either TM or extracellular function were selected.

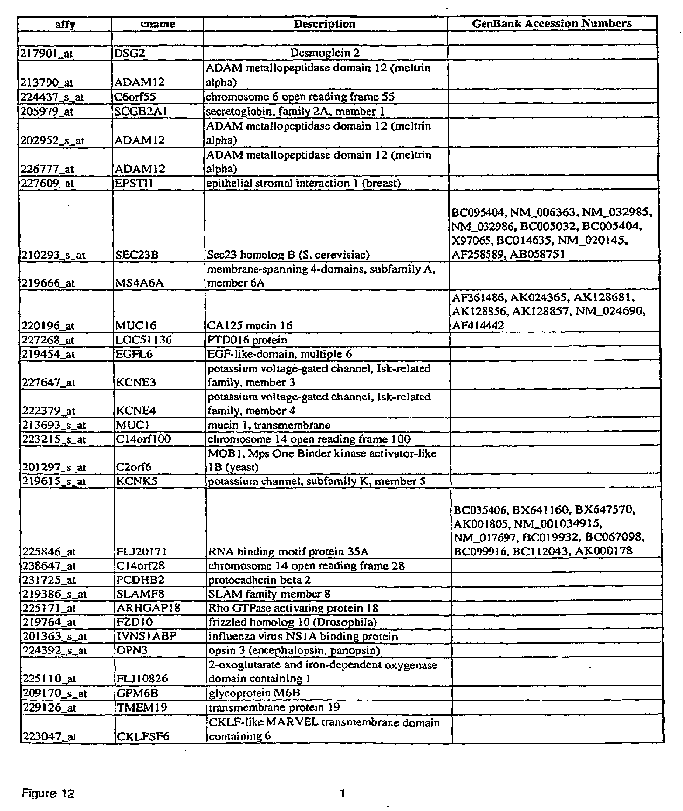

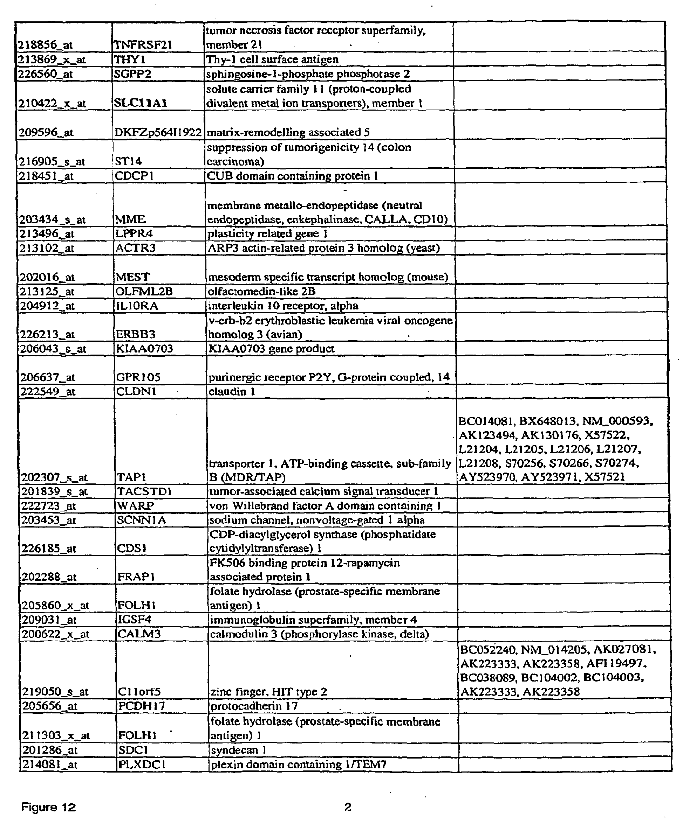

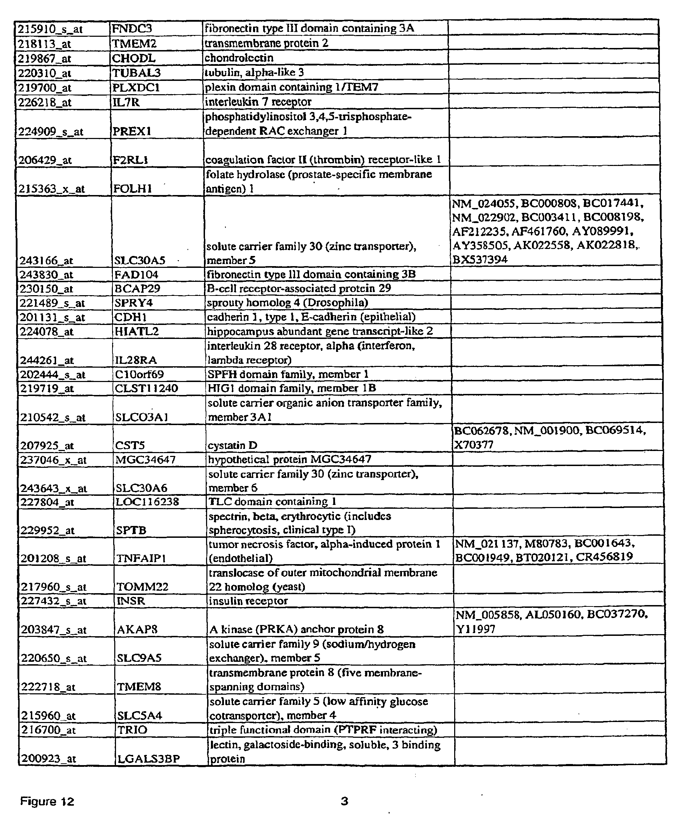

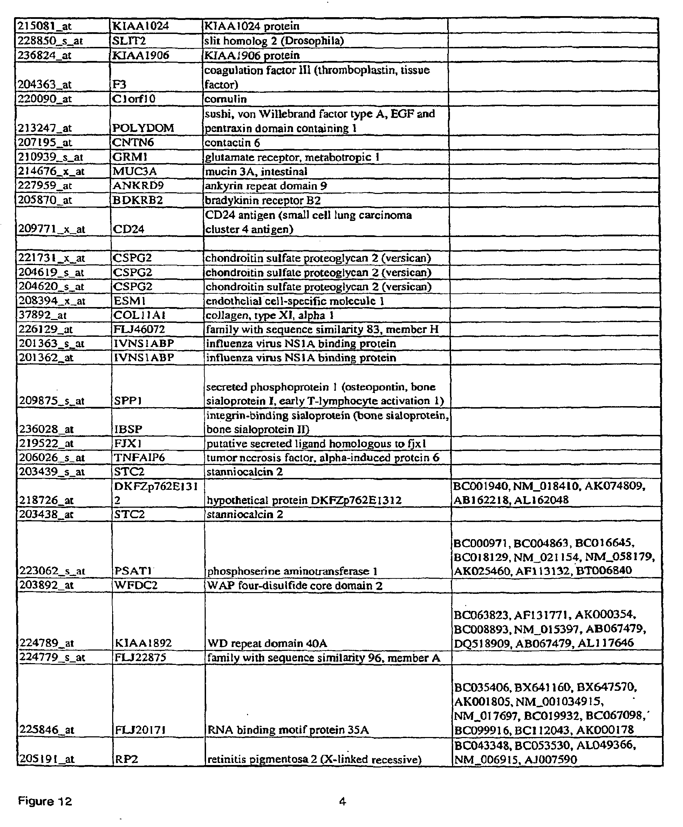

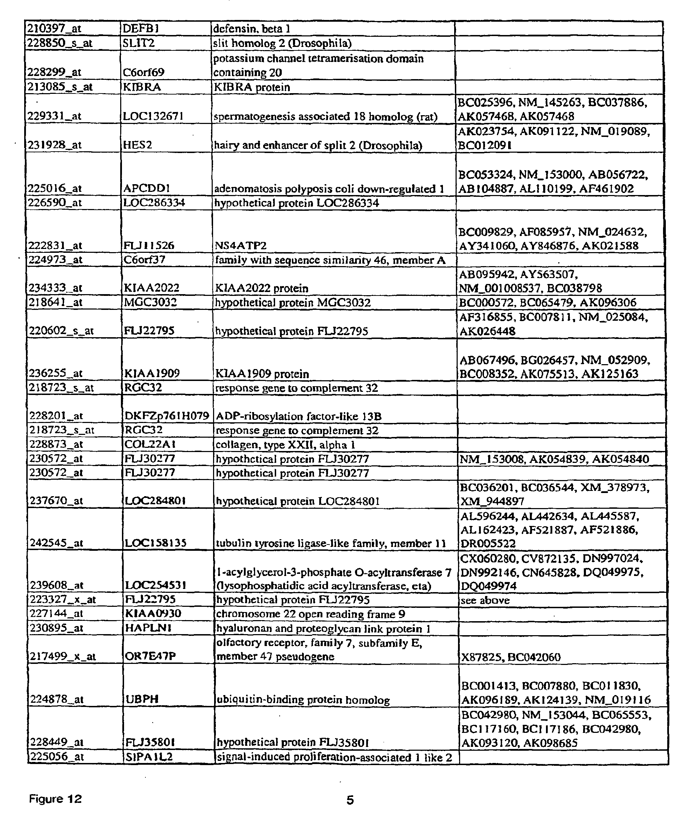

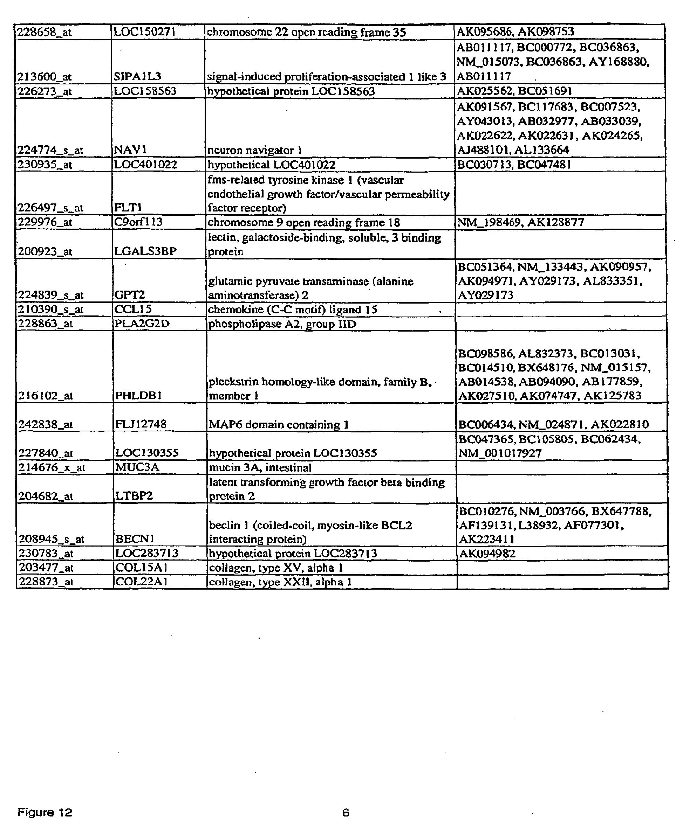

The additional TVM are listed in Figure 12.

### EXAMPLE 13: DEVELOPMENT OF NEW ANTIBODIES AGAINST TVM

### MATERIALS AND EXPERIMENTAL METHODS

### Peptides

Peptides are synthesized using an Fmoc-based chemical strategy.

### Antibodies

Antibody production is outsourced to specialized biotechnology companies; e.g. Prosci Inc. (Poway, CA). To exclude any Fc fragment-mediated effects that may interfere with Ab specificity, Abs are prepared as F(ab')₂ fragments.

### RESULTS

The TVM identified in the above Examples are used as targets for the development of epitope-specific monoclonal (m)Abs and affinity-purified anti-peptide polyclonal (p)Abs. For target screening, affinity-purified pAbs are initially generated. In other experiments, synthetic peptides spanning epitopic regions of 20-30 extracellular hydrophilic residues from secretory or transmembrane proteins from the above Examples are synthesized. In another embodiment, the epitopic regions of extracellular hydrophilic residues are deduced based on their primary sequence.

Monoclonal antibodies to whole proteins are generated for promising candidates.

The work described herein generates and identifies mAb and pAb for screening and validation studies. Certain of these Abs are also suitable to clinical applications for serum cancer diagnostics, e.g. as described below

### EXAMPLE 14: IDENTIFICATION OF ADDITIONAL TVM USING PHAGE DISPLAY LIBRARIES

### MATERIALS AND EXPERIMENTAL METHODS

### Phage display libraries

To derive human Abs, a non-immune library of phage-displayed human single-chain Fv Abs is generated.

### Construction of the VH library

Total RNA is prepared from ovarian cancer cells and normal ovary samples. cDNA is synthesized from total RNA primed with the HulgMFOR primer (TGGAAGAGGCACGTTCTTTTCTTT; SEQ ID No: 71). VH gene repertoires are amplified from the cDNA by using Vent DNA polymerase (New England Biolabs) in combination with the HulgMFOR primer and an equimolar mixture of HuVHBACK primers (CAGGTGCAGCTGGTGCAGTCTGG- SEQ ID No: 72- CAGGTCAACTTAAGGGAGTCTGG- SEQ ID No: 73; GAGGTGCAGCTGGTGGAGTCTGG- SEQ ID No:74; CAGGTGCAGCTGCAGGAGTCGGG-SEQ ID No: 75; CAGGTACAGCTGCAGCAGTCAGG- SEQ ID No: 76). PCR products areagarose gel-purified and re-amplified to append Ncol and Notl restriction sites by using Tth DNA polymerase (Epicentre Technologies, Madison, WI) and an equimolar mixture of the HuVHBACK Sfi primers (GTCCTCGCAACTGCGGCCCAGCCGGCCATGGCCCAGGTCAACTTAAGGGAG TCTGG- SEQ ID No: 77) GTCCTCGCAACTGCGGCCCAGCCGGCCATGGCCGAGGTGCAGCTGGTGGAG TCTGG-- SEQ ID No: 78; GTCCTCGCAACTGCGGCCCAGCCGGCCATGGCCCAGGTGCAGCTGCAGGAG TCGGG - SEQ ID No: 79; GTCCTCGCAACTGCGGCCCAGCCGGCCATGGCCCAGGTGCAGCTGTTGCAG TCTGC - SEQ ID No: 80; GTCCTCGCAACTGCGGCCCAGCCGGCCATGGCCCAGGTACAGCTGCAGCAG TCAGG - SEQ ID No: 81) and the HuCMFor Not primer (5'-GAGTCATTCTCGACTTGCGGCCGCTGGAAGAGGCACGTTCTTTTCTTT-3'; SEQ ID No: 82). The PCR products are digested with Ncol and Notl and agarose gel-purified. The resulting DNA fragments are ligated into the plasmid pCITE3A (Novagen; any plasmid that contains the proper restriction sites for cloning and unique sequences for PCR amplification works as well) and cut with restriction enzymes Ncol and Notl, and the ligated DNA is electroporated into the E. coli strain TG1. If desired, cloning efficiency and library diversity is determined by PCR screening.

### Construction of the scFv Library

The VH gene repertoire is PCR-amplified from the pCITE-VH library by using 300 ng of library plasmid DNA as a template, Vent DNA polymerase, the CITE3 primer (GATCTGATCTGGGGCCTCGGTGC-SEQ ID No: 83), and an equimolar mixture of HuJH primers. The VL genes for scFv assembly are obtained from an available scFv phage antibody library. The VL gene repertoire, including DNA encoding the scFv peptide linker (G₄S)₃, is amplified from 300 ng of library plasmid DNA by using Vent DNA polymerase, the Gene3 primer (5'-GCAAGCCCAATAGGAACCCATGTACCG- SEQ ID No: 84), and an equimolar mixture of RHuJH primers. The amplified VH and VL genes are agarosegel-purified and spliced together with overlap extension PCR to create a scFv gene repertoire. To facilitate joining VH and VL gene repertoires with overlap extension PCR, the input DNA fragments have blunt ends. The proofreading DNA polymerase Vent is used to generate the VH and VL DNA fragments for scFv assembly. For all subsequent PCR steps of library construction, Tth DNA polymerase was found to be the optimal enzyme. The VH and VL gene repertoires are spliced together in 100- µl PCR reactions containing 100 ng of the VH and VL DNA fragments and Tth DNA polymerase. The reactions are cycled eight times (95°C 2 min, 55°C, 1 min, and 72°C 3 min) to join the fragments. Then the CITE3 and Gene3 primers are added, and the reaction is cycled 30 times (94°C 1 min, 55°C 1 min, and 72°C 3 min) to amplify the assembled scFv genes. The scFv genes are digested with restriction enzymes Ncol and Notl, agarose gel-purified, and ligated into the plasmid pHEN-1 digested with Ncol and Notl. The ligated DNA is electroporated into E. coli TG1 cells

This method enables rapid production of panels (∼7 × 10⁹) of high-affinity (<1 nM) mAbs, which are desirable for therapeutics and diagnostics.

### RESULTS

Single-chain Fv (scFv) Ab phage display libraries are generated from patients with ovarian cancer and screened to identify antibodies (Abs) recognizing TVM identified in the above Examples. To identify auto-Abs that recognize TVM in ovarian cancer patients, phage display libraries are generated from ovarian cancer tumor samples. Separate IgGκ and IgGλ phage libraries are constructed, using the phagemid vector pComb3X (Scripps Research Institute) from 4 x 10⁷ peripheral blood or ascites mononuclear cells. For library selection, IgGκ and IgGλ Ab phage display libraries are combined in equal amounts and panned by solid-phase selection on ELISA plates coated with generated peptides or proteins. Binding of individual phage clones isolated from each round of panning can be confirmed by ELISA, if desired. The VH and VL nucleic acid sequences are determined for positive clones to establish a cohort of unique mAbs specific to TVM. scFv monoclonal preparations unlinked to phages are produced using TOP10F' non-suppressor *E*. *coli.* In other experiments, phage display libraries are panned on frozen tissue sections of normal ovaries and then on sections of ovarian cancer. Slides are then subjected to immuno-LCM, as described in previous Examples. With this approach, phages recognizing TVM are removed selectively from the tissue. RNA is isolated and used to infect additional phages. Serial panning between normal and tumor tissue leads to the selection to tumor vascular-specific phages, which are then used to develop Abs as above. With this alternative approach, additional TVM are identified

In other experiments, human scFv Ab libraries from normal donors are screened.

### EXAMPLE 15: USE OF TRANSMEMBRANE TVM FOR TUMOR LOCALIZATION IN VIVO

The transmembrane TVM identified in the above Examples are used as targets for localizing ovarian or other solid tumors, as described for Adlican in Example 10. In other embodiments, radio-labeled antibodies are used for imaging. In another embodiment, positron-emission tomography is used.

### EXAMPLE 16: USE OF TRANSMEMBRANE TVM FOR DELIVERY OF ANTI-TUMOR AGENTS IN VIVO

In another embodiment, an antibody identified in one of the above Examples is used to target an anti-tumor agent to ovarian or other solid tumor *in vivo*. In some experiments, the anti-tumor agent is a radioactive antibody. In other experiments, the anti-tumor agent is an antibody conjugated to a nanosphere that contains an anti-tumor drug.

### EXAMPLE 17: USE OF SECRETED TVM FOR SERUM DIAGNOSTIC TESTING OF CANCER IN VIVO

Existing Abs and novel Abs or scFv identified in the above Examples, recognizing secretory TVM of the present invention, are tested for serum diagnostic applications. ELISA is utilized to test how well each TVM performs as a biomarker for ovarian cancer serum detection. In addition, TVM are tested as biomarkers for other solid tumors.

### EXAMPLE 18: USE OF TRANSMEMBRANE TVM FOR VASCULATURE-REDIRECTED LYMPHOCYTE THERAPY

Chimeric immuno-receptors composed of high-affinity scFv recognizing TVM of the present invention (Example 14) fused to the cytoplasmic signaling modules of T cell receptor, CD28 and 4-1 BB are created as described in Ledbetter JA et al (Enhanced transmembrane signaling activity of monoclonal antibody hetero-conjugates suggests molecular interactions between receptors on the T cell surface. Mol Immunol. 1989 Feb;26(2):137-45). The chimeric receptors are then utilized for vasculature-redirected lymphocyte therapy.

### EXAMPLE 19: ADDITIONAL STUDIES WITH TNFRSF21 (DR6)

Expression of DR6 mRNA was determined in a variety of normal and tumor tissues by Affymetrix® array. Expression was relatively low in normal tissues (Table 6), while DR6 was heavily expressed in many tumor tissues, particularly bladder (Table 7).

**Table 6: Affymetrix array scores of DR6 in normal tissues.**

| Tissue | Affy score | Tissue | Affy score |
|---|---|---|---|
| Adipose tissue | 6 | Myometrium | 5 |
| Adrenal gland cortex | 4 | Nipple cross section | 5 |
| Bone marrow | 4 | Oral mucosa | 6 |
| Bronchus | 6 | Ovary | 4 |
| Cerebellum | 5 | Pharyngeal mucosa | 6 |
| Cerebral cortex | 6 | Pituitary gland | 5 |
| Cerebrum | 6 | Prostate gland | 5 |
| Cervix | 5 | Salivary gland | 5 |
| Coronary artery | 5 | Saphenous vein | 5 |
| Colon cecum | 5 | Skeletal muscle | 4 |
| Endometrium | 5 | Spinal cord | 7 |
| Esophagus | 6 | Spleen | 4 |
| Heart atrium | 5 | Stomach | 6 |
| Heart ventricle | 5 | Testes | 4 |
| Kidney cortex | 6 | Thyroid gland | 4 |
| Kidney medulla | 6 | Tongue | 6 |
| Liver | 4 | Tonsil | 6 |
| Lung | 5 | Trachea | 7 |
| Lymph nodes | 6 | Trigeminal ganglia | 6 |
| Mammary gland | 5 | Urethra | 6 |
| Medulla | 6 | Vagina | 5 |
| Midbrain | 6 | Vulva | 5 |

**Table 7: Affymetrix array scores of DR6 in tumor tissues.**

| Tissue | Affy score | Tissue | Affy score |
|---|---|---|---|
| Adrenal gland | 7 | Lung | 7 |
| Bladder | 8 | Omentum | 6 |
| Brain | 6 | Ovary | 7 |
| Breast | 6 | Pancreas | 7 |
| Cervix | 7 | Prostate | 6 |
| Colon | 7 | Rectosigmoid | 7 |
| Corpus uteri | 6 | Small intestine | 6 |
| Endometrium | 7 | Stomach | 7 |
| Esophagus | 6 | Testis | 7 |
| Kidney | 7 | Thyroid | 5 |
| Liver | 7 | Vulva | 6 |

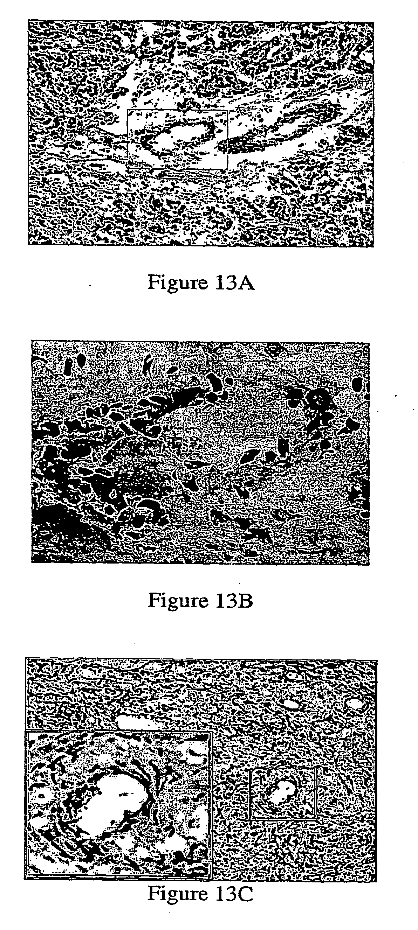

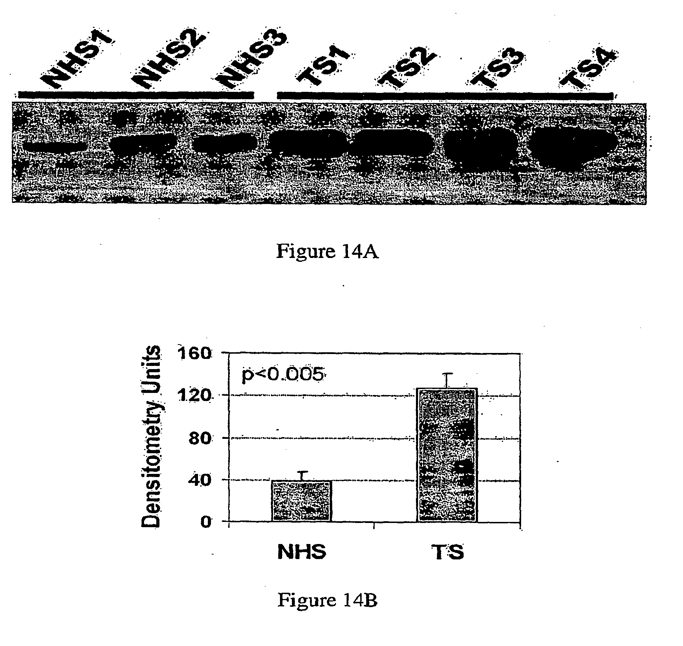

Further, enhanced expression of DR6 in ovarian tumor relative to normal ovary tissue was confirmed by qPCR and IHC (Figure 13A-B). In addition, increased serum levels of DR6 were found in serum of patients with ovarian tumors compared to healthy individuals (Figure 14). Thus, DR6 is efficacious as a tumor marker (e.g. for ovarian tumors), both *in situ* and in tumors.

### EXAMPLE 20: ADDITIONAL STUDIES WITH ADAM12

Expression of ADAM12 mRNA was determined in a variety of normal and tumor tissues by Affymetrix® array. Expression was relatively low in normal tissues (Table 8); while ADAM12 was significantly enriched in several tumor tissues (Table 9).

**Table 8: Affymetrix array scores of ADAM12 in normal tissues.**

| Tissue | Affy score | Tissue | Affy score |
|---|---|---|---|
| Adipose tissue | 3 | Myometrium | 2 |
| Adrenal gland cortex | 2 | Nipple cross section | 2 |
| Bone marrow | 2 | Oral mucosa | 2 |
| Bronchus | 2 | Ovary | 4 |
| Cerebellum | 2 | Pharyngeal mucosa | 2 |
| Cerebral cortex | 2 | Pituitary gland | 2 |
| Cerebrum | 2 | Prostate gland | 2 |
| Cervix | 2 | Salivary gland | 2 |
| Colon cecum | 2 | Saphenous vein | 2 |
| Coronary artery | 2 | Skeletal muscle | 2 |
| Endometrium | 5 | Spinal cord | 2 |
| Esophagus | 2 | Spleen | 2 |
| Heart atrium | 2 | Stomach | 2 |
| Heart ventricle | 2 | Testes | 2 |
| Kidney cortex | 2 | Thyroid gland | 2 |
| Kidney medulla | 2 | Tongue | 2 |
| Liver | 2 | Tonsil | 3 |
| Lung | 2 | Trachea | 2 |
| Lymph nodes | 3 | Trigeminal ganglia | 2 |
| Mammary gland | 3 | Urethra | 2 |
| Medulla | 2 | Vagina | 2 |
| Midbrain | 2 | Vulva | 3 |

**Table 9: Affymetrix array scores of ADAM12 in tumor tissues.**

| Tissue | Affy score | Tissue | Affy score |
|---|---|---|---|
| Adrenal gland | 5 | Lung | 5 |
| Bladder | 5 | Omentum | 5 |
| Brain | 3 | Ovary | 4 |
| Breast | 6 | Pancreas | 5 |
| Cervix | 5 | Prostate | 3 |
| Colon | 5 | Rectosigmoid | 5 |
| Corpus uteri | 6 | Small intestine | 3 |
| Endometrium | 5 | Stomach | 3 |
| Esophagus | 3 | Testis | 3 |
| Kidney | 3 | Thyroid | 3 |
| Liver | 5 | Vulva | 6 |

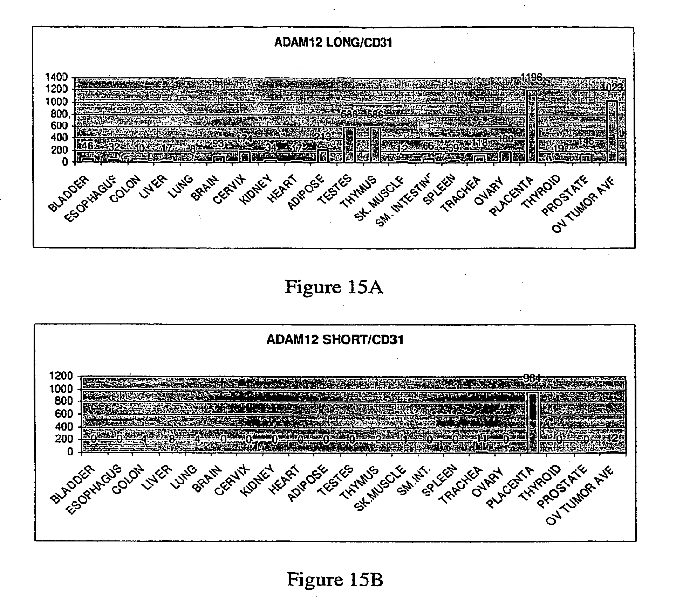

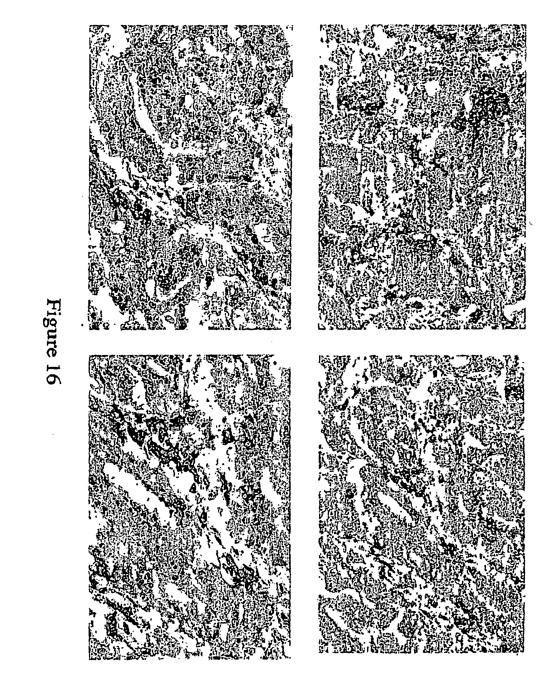

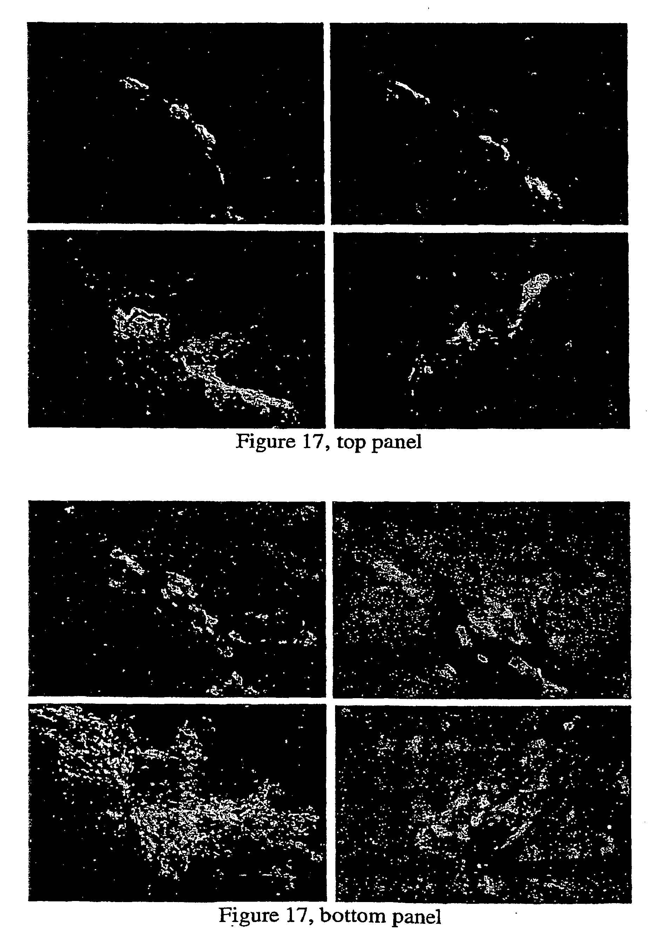

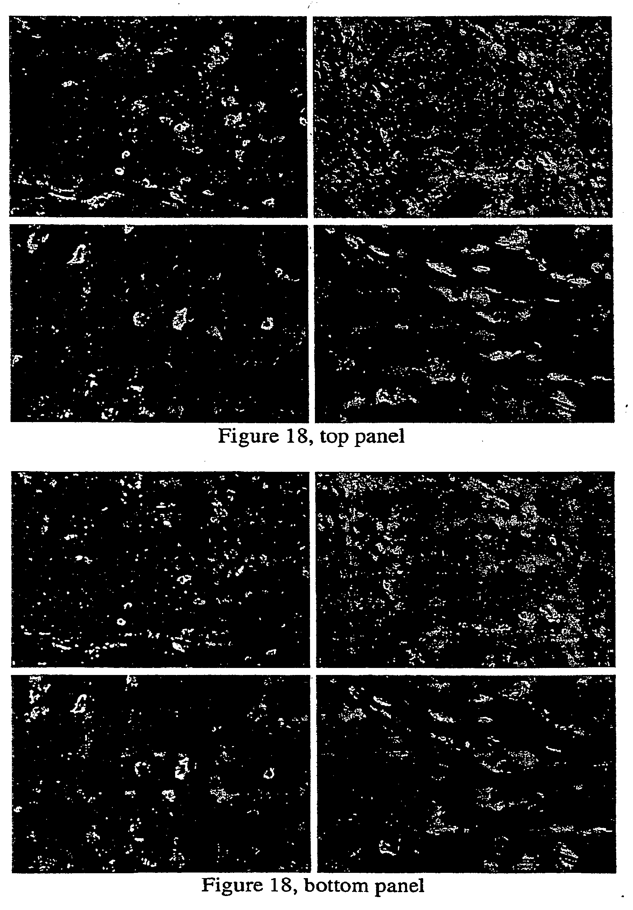

Further, enhanced expression of ADAM12 in ovarian tumor relative to normal tissues was confirmed by qPCR (Figure 15). In addition, staining of ADAM12 was demonstrated in ovarian tumors (Figure 16). The relevance of ADAM12 as a TVM was further confirmed by co-staining with CD31 and CD45 (Figures 17-18). Thus, ADAM12 is efficacious as a tumor marker (e.g. for ovarian tumors), further validating the results of the present invention.

### EXAMPLE 21: ADDITIONAL STUDIES WITH CDCP1

Expression of CDCP1 mRNA was determined in a variety of normal and tumor tissues by Affymetrix® array. Expression was significantly enriched in several tumor tissues compared to most normal tissues (Tables 11 and 10, respectively).

**Table 10: Affymetrix array scores of CDCP1 in normal tissues.**

| Tissue | Affy score | Tissue | Affy score |
|---|---|---|---|
| Adipose tissue | 3 | Myometrium | 3 |
| Adrenal gland cortex | 3 | Nipple cross section | 4 |
| Bone marrow | 3 | Oral mucosa | 5 |
| Bronchus | 3 | Ovary | 3 |
| Cerebellum | 3 | Pharyngeal mucosa | 5 |
| Cerebral cortex | 3 | Pituitary gland | 3 |
| Cerebrum | 3 | Prostate gland | 4 |
| Cervix | 3 | Salivary gland | 3 |
| Colon cecum | 3 | Saphenous vein | 3 |
| Coronary artery | 3 | Skeletal muscle | 3 |
| Endometrium | 3 | Spinal cord | 3 |
| Esophagus | 5 | Spleen | 3 |
| Heart atrium | 3 | Stomach | 4 |
| Heart ventricle | 3 | Testes | 3 |
| Kidney cortex | 3 | Thyroid gland | 4 |
| Kidney medulla | 3 | Tongue | 4 |
| Liver | 3 | Tonsil | 4 |
| Lung | 3 | Trachea | 4 |
| Lymph nodes | 3 | Trigeminal ganglia | 3 |
| Mammary gland | 3 | Urethra | 4 |
| Medulla | 3 | Vagina | 4 |
| Midbrain | 3 | Vulva | 5 |

**Table 11: Affymetrix array scores of CDCP1 in tumor tissues.**

| Tissue | Affy score | Tissue | Affy score |
|---|---|---|---|
| Adrenal gland | 5 | Lung | 5 |
| Bladder | 5 | Omentum | 5 |
| Brain | 4 | Ovary | 5 |
| Breast | 5 | Pancreas | 5 |
| Cervix | 5 | Prostate | 4 |
| Colon | 5 | Rectosigmoid | 5 |
| Corpus uteri | 5 | Small intestine | 5 |
| Endometrium | 5 | Stomach | 5 |
| Esophagus | 5 | Testis | 3 |
| Kidney | 4 | Thyroid | 4 |
| Liver | 5 | Vulva | 5 |

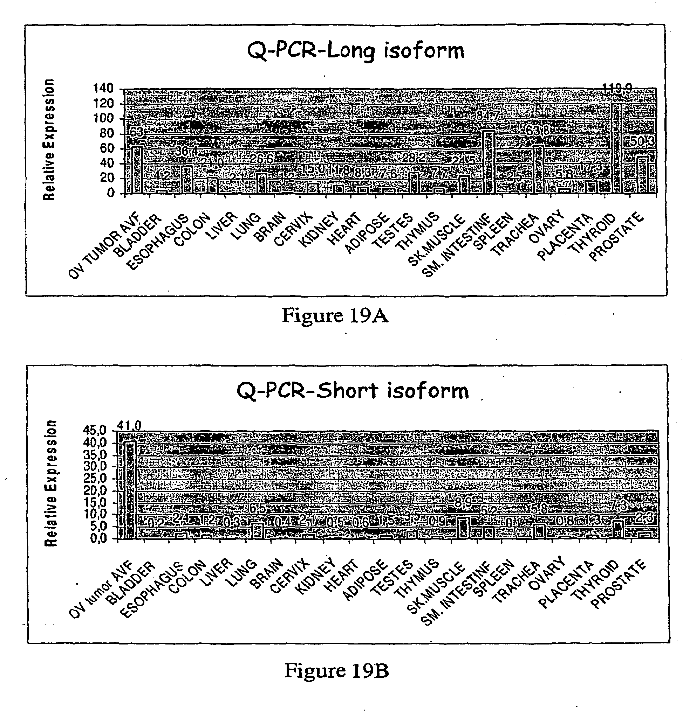

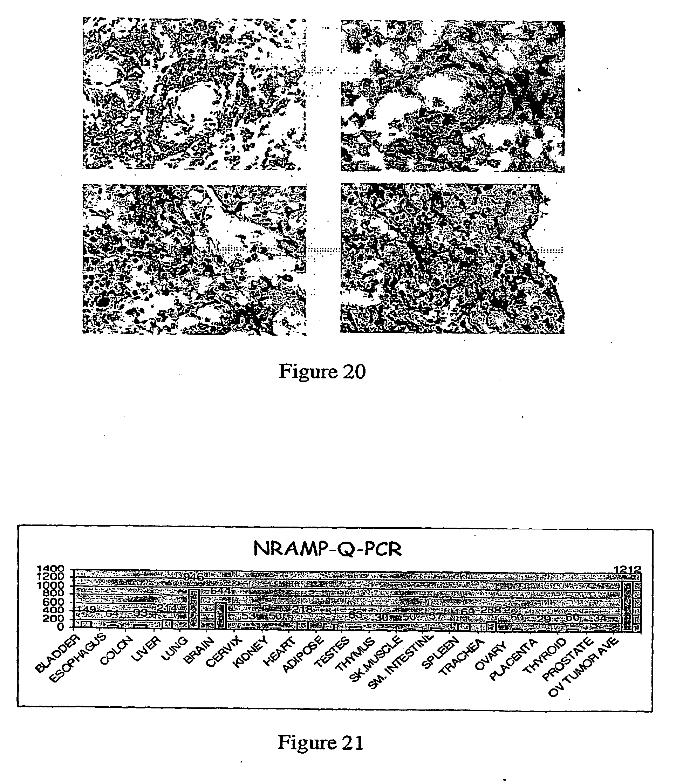

Further, enhanced expression of the short isoform of CDCP1 in ovarian tumor relative to normal tissues was confirmed by qPCR (Figure 19). In addition, staining of CDCP1 was demonstrated in ovarian tumors (Figure 20). Thus, CDCP1 is efficacious as a tumor marker (e.g. for ovarian tumors), further validating the results of the present invention.

### EXAMPLE 22: ADDITIONAL STUDIES WITH SLC11A1

Expression of SLC11A1 mRNA was determined in a variety of normal and tumor tissues by Affymetrix® array. Expression was significantly enriched in several tumor tissues compared to most normal tissues (Tables 13 and 12, respectively).

**Table 12: Affymetrix array scores of SLC11A1 in normal tissues.**

| Tissue | Affy score | Tissue | Affy score |
|---|---|---|---|
| Adipose tissue | 4 | Myometrium | 4 |
| Adrenal gland cortex | 4 | Nipple cross section | 4 |
| Bone marrow | 5 | Oral mucosa | 4 |
| Bronchus | 4 | Ovary | 4 |
| Cerebellum | 4 | Pharyngeal mucosa | 4 |
| Cerebral cortex | 4 | Pituitary gland | 4 |
| Cerebrum | 4 | Prostate gland | 4 |
| Cervix | 4 | Salivary gland | 4 |
| Colon cecum | 4 | Saphenous vein | 4 |
| Coronary artery | 4 | Skeletal muscle | 5 |
| Endometrium | 4 | Spinal cord | 4 |
| Esophagus | 4 | Spleen | 4 |
| Heart atrium | 4 | Stomach | 4 |
| Heart ventricle | 4 | Testes | 4 |
| Kidney cortex | 4 | Thyroid gland | 4 |
| Kidney medulla | 4 | Tongue | 4 |
| Liver | 4 | Tonsil | 4 |
| Lung | 5 | Trachea | 4 |
| Lymph nodes | 4 | Trigeminal ganglia | 4 |
| Mammary gland | 4 | Urethra | 4 |
| Medulla | 4 | Vagina | 4 |
| Midbrain | 4 | Vulva | 5 |

**Table 13: Affymetrix array scores of SLC11A1 in tumor tissues.**

| Tissue | Affy score | Tissue | Affy score |
|---|---|---|---|
| Adrenal gland | 4 | Lung | 5 |
| Bladder | 5 | Omentum | 5 |
| Brain | 5 | Ovary | 5 |
| Breast | 5 | Pancreas | 5 |
| Cervix | 5 | Prostate | 5 |
| Colon | 5 | Rectosigmoid | 5 |
| Corpus uteri | 5 | Small intestine | 5 |
| Endometrium | 5 | Stomach | 5 |
| Esophagus | 4 | Testis | 5 |
| Kidney | 5 | Thyroid | 5 |
| Liver | 5 | Vulva | 5 |

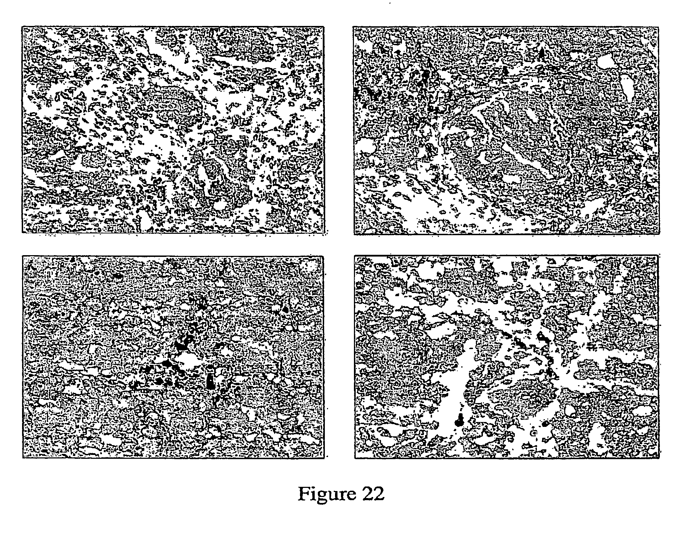

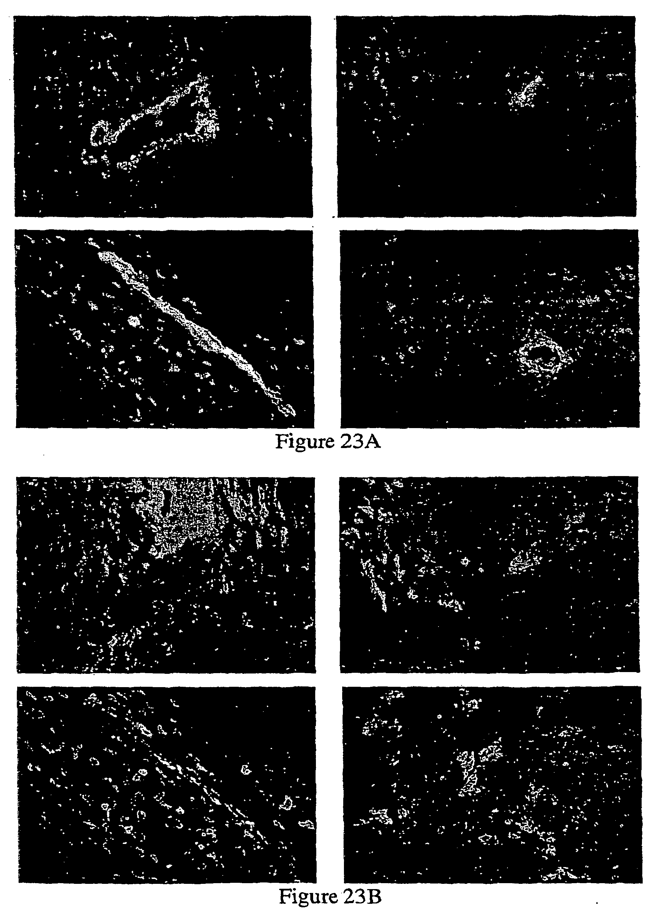

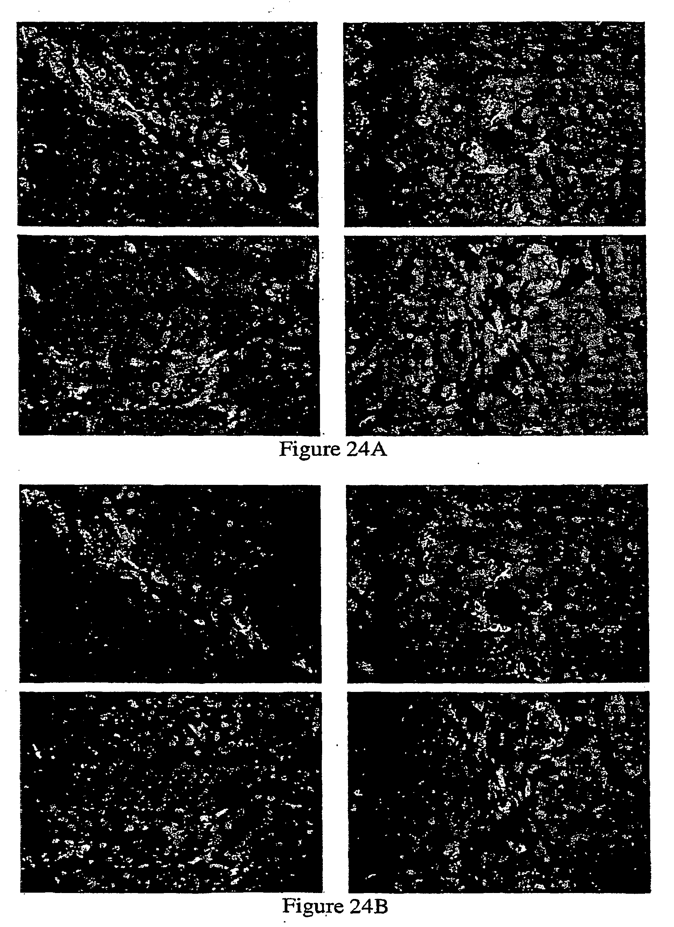

Further, enhanced expression of SLC11A1 in ovarian tumor relative to normal tissues was confirmed by qPCR (Figure 21). In addition, staining of SLC11A1 was demonstrated in ovarian tumors (Figure 22). The relevance of SLC11A1 as a TVM was further confirmed by co-staining with CD31 and CD45 (Figures 23-24). Thus, SLC11A1 is efficacious as a tumor marker (e.g. for ovarian tumors).

### EXAMPLE 23: ADDITIONAL STUDIES WITH BLAME

Expression of BLAME mRNA was determined in a variety of normal and tumor tissues by Affymetrix® array. Expression was significantly enriched in most tumor tissues compared to most normal tissues (Tables 15 and 14, respectively).

**Table 14: Affymetrix array scores of BLAME in normal tissues.**

| Tissue | Affy score | Tissue | Affy score |
|---|---|---|---|
| Adipose tissue | 3 | Myometrium | 2 |
| Adrenal gland cortex | 2 | Nipple cross section | 3 |
| Bone marrow | 3 | Oral mucosa | 3 |
| Bronchus | 3 | Ovary | 2 |
| Cerebellum | 2 | Pharyngeal mucosa | 3 |
| Cerebral cortex | 2 | Pituitary gland | 2 |
| Cerebrum | 2 | Prostate gland | 2 |
| Cervix | 2 | Salivary gland | 2 |
| Colon cecum | 3 | Saphenous vein | 3 |
| Coronary artery | 3 | Skeletal muscle | 2 |
| Endometrium | 2 | Spinal cord | 2 |
| Esophagus | 2 | Spleen | 3 |
| Heart atrium | 2 | Stomach | 3 |
| Heart ventricle | 2 | Testes | 2 |
| Kidney cortex | 2 | Thyroid gland | 3 |
| Kidney medulla | 2 | Tongue | 3 |
| Liver | 3 | Tonsil | 4 |
| Lung | 4 | Trachea | 3 |
| Lymph nodes | 5 | Trigeminal ganglia | 3 |
| Mammary gland | 3 | Urethra | 3 |
| Medulla | 2 | Vagina | 2 |
| Midbrain | 2 | Vulva | 2 |

**Table 15: Affymetrix array scores of BLAME in tumor tissues.**

| Tissue | Affy score | Tissue | Affy score |
|---|---|---|---|
| Adrenal gland | 6 | Lung | 5 |
| Bladder | 5 | Omentum | 5 |
| Brain | 3 | Ovary | 4 |
| Breast | 5 | Pancreas | 5 |
| Cervix | 4 | Prostate | 4 |
| Colon | 5 | Rectosigmoid | 5 |
| Corpus uteri | 5 | Small intestine | 5 |
| Endometrium | 4 | Stomach | 4 |
| Esophagus | 4 | Testis | 7 |
| Kidney | 5 | Thyroid | 4 |
| Liver | 5 | Vulva | 5 |

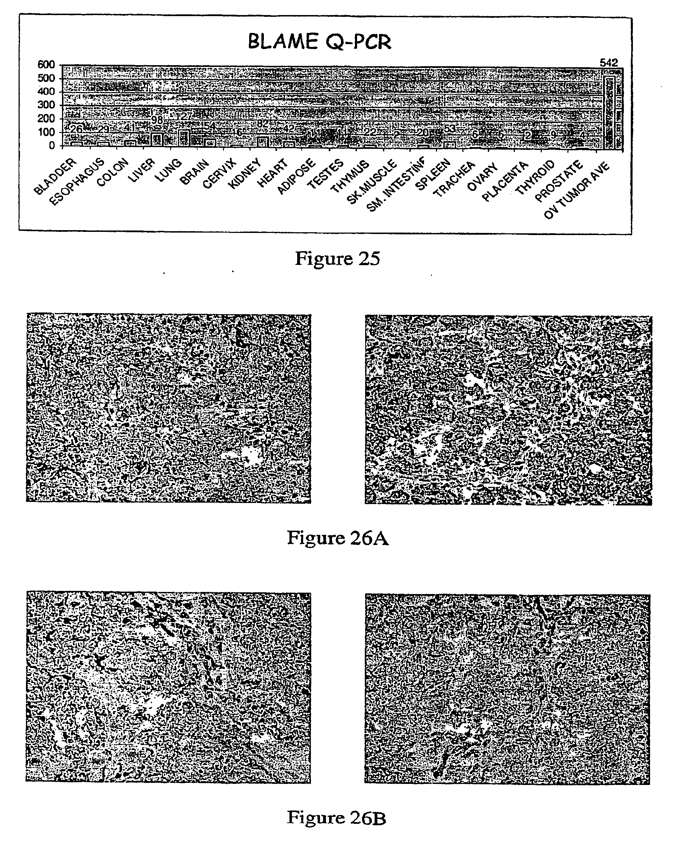

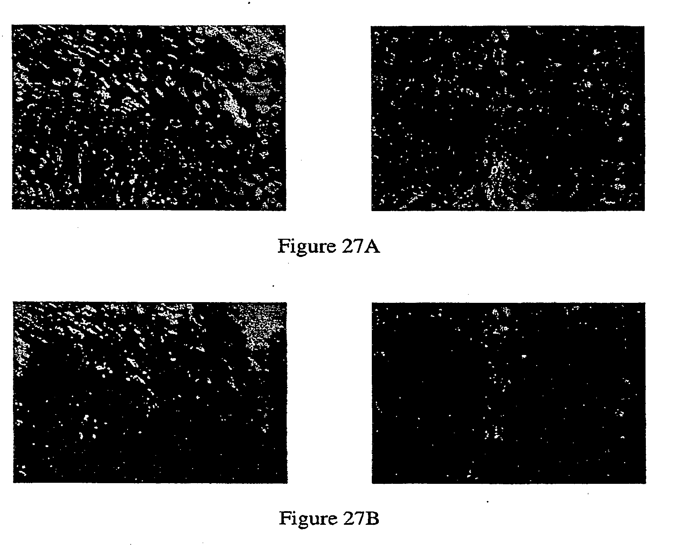

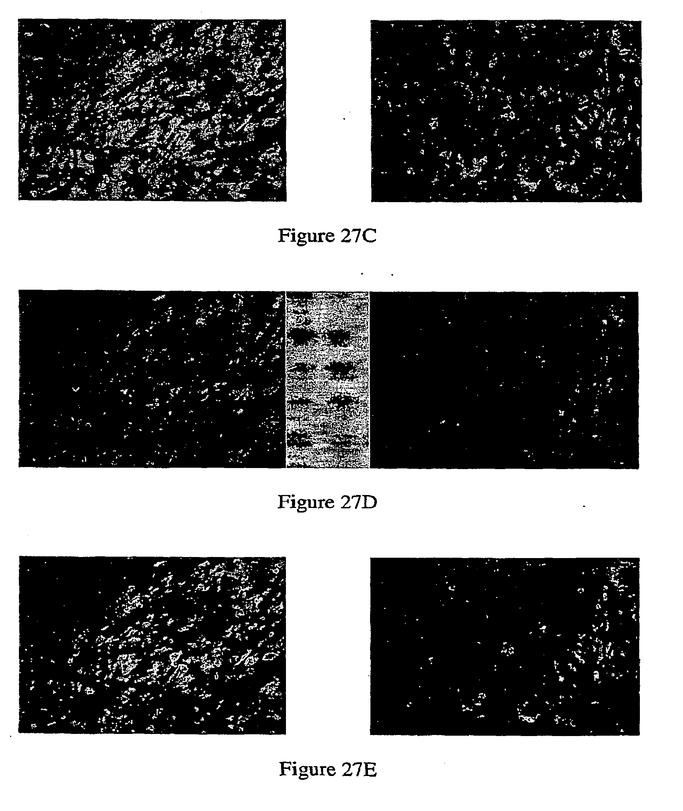

Further, enhanced expression of BLAME in ovarian tumor relative to normal tissues was confirmed by qPCR (Figure 25). In addition, staining of BLAME was demonstrated in ovarian tumors (Figure 26). The relevance of BLAME as a TVM was further confirmed by co-staining with CD31 and CD45 (Figure 27). Thus, BLAME is efficacious as a tumor marker (e.g. for ovarian, adrenal, and testis tumors).

### EXAMPLE 24: ADDITIONAL STUDIES WITH ESM1

Expression of ESM1 mRNA was determined in a variety of normal and tumor tissues by Affymetrix® array. Expression was significantly enriched in tumor tissues compared to normal tissues (Tables 17 and 16, respectively).

**Table 16: Affymetrix array scores of ESM1 in normal tissues.**

| Tissue | Affy score | Tissue | Affy score |
|---|---|---|---|
| Adipose tissue | 2 | Myometrium | 2 |
| Adrenal gland cortex | 2 | Nipple cross section | 2 |
| Bone marrow | 2 | Oral mucosa | 2 |
| Bronchus | 2 | Ovary | 2 |
| Cerebellum | 2 | Pharyngeal mucosa | 2 |
| Cerebral cortex | 2 | Pituitary gland | 2 |
| Cerebrum | 2 | Prostate gland | 2 |
| Cervix | 2 | Salivary gland | 2 |
| Colon cecum | 2 | Saphenous vein | 2 |
| Coronary artery | 2 | Skeletal muscle | 2 |
| Endometrium | 3 | Spinal cord | 2 |
| Esophagus | 2 | Spleen | 2 |
| Heart atrium | 2 | Stomach | 2 |
| Heart ventricle | 2 | Testes | 2 |
| Kidney cortex | 3 | Thyroid gland | 2 |
| Kidney medulla | 2 | Tongue | 2 |
| Liver | 2 | Tonsil | 2 |
| Lung | 3 | Trachea | 2 |
| Lymph nodes | 2 | Trigeminal ganglia | 2 |
| Mammary gland | 2 | Urethra | 2 |
| Medulla | 2 | Vagina | 2 |
| Midbrain | 2 | Vulva | 2 |

**Table 17: Affymetrix array scores of ESM1 in tumor tissues.**

| Tissue | Affy score | Tissue | Affy score |
|---|---|---|---|
| Adrenal gland | 6 | Lung | 4 |
| Bladder | 3 | Omentum | 4 |
| Brain | 3 | Ovary | 4 |
| Breast | 3 | Pancreas | 3 |
| Cervix | 3 | Prostate | 3 |
| Colon | 3 | Rectosigmoid | 3 |
| Corpus uteri | 4 | Small intestine | 3 |
| Endometrium | 3 | Stomach | 3 |
| Esophagus | 3 | Testis | 3 |
| Kidney | 6 | Thyroid | 3 |
| Liver | 3 | Vulva | 3 |

Thus, ESM1 is efficacious as a tumor marker (e.g. for ovarian, adrenal, and kidney tumors).

### EXAMPLE 25: ADDITIONAL EXPRESSION STUDIES

mRNA expression levels of additional markers were determined in a variety of normal and tumor tissues by Affymetrix® array. Results are depicted in Tables 18-25.

**Table 18: Affymetrix array scores of additional markers in normal tissues.**

| Tissue | c6orf55 | SCGB-2A1 | DSG2 | EPSTI1 | SEC-23B | MS4A6A | LOC51136 |
|---|---|---|---|---|---|---|---|
| Adipose tissue | 4 | 2 | 4 | 4 | 3 | 5 | 3 |
| Adrenal gland cortex | 4 | 2 | 2 | 4 | 2 | 5 | 3 |
| Bone marrow | 4 | 2 | 2 | 4 | 2 | 5 | 3 |
| Bronchus | 4 | 3 | 4 | 4 | 5 | 4 | 2 |
| Cerebellum | 4 | 2 | 2 | 3 | 2 | 4 | 2 |
| Cerebral cortex | 4 | 2 | 2 | 3 | 2 | 4 | 2 |
| Cerebrum | 4 | 2 | 2 | 3 | 2 | 4 | 2 |
| Cervix | 4 | 4 | 3 | 4 | 2 | 4 | 3 |
| Colon cecum | 4 | 2 | 4 | 4 | 2 | 4 | 2 |
| Coronary artery | 4 | 2 | 3 | 4 | 4 | 5 | 2 |
| Endometrium | 4 | 7 | 4 | 4 | 4 | 4 | 3 |
| Esophagus | 5 | 2 | 3 | 3 | 2 | 4 | 2 |
| Heart atrium | 4 | 2 | 5 | 4 | 2 | 5 | 3 |
| Heart ventricle | 4 | 2 | 4 | 4 | 2 | 4 | 2 |
| Kidney cortex | 4 | 2 | 5 | 4 | 2 | 4 | 2 |
| Kidney medulla | 4 | 2 | 5 | 4 | 2 | 4 | 2 |
| Liver | 4 | 2 | 3 | 4 | 2 | 6 | 3 |
| Lung | 4 | 2 | 3 | 5 | 2 | 5 | 3 |
| Lymph nodes | 4 | 2 | 2 | 5 | 2 | 6 | 3 |
| Mammary gland | 4 | 4 | 5 | 4 | 3 | 5 | 3 |
| Medulla | 4 | 2 | 2 | 4 | 2 | 4 | 2 |
| Midbrain | 4 | 2 | 2 | 4 | 2 | 4 | 2 |
| Myometrium | 4 | 2 | 2 | 3 | 2 | 4 | 2 |
| Nipple cross section | 4 | 3 | 3 | 3 | 2 | 4 | 2 |
| Oral mucosa | 5 | 2 | 3 | 4 | 2 | 5 | 3 |
| Ovary | 4 | 2 | 3 | 4 | 2 | 4 | 3 |
| Pharyngeal mucosa | 5 | 3 | 3 | 3 | 3 | 5 | 3 |
| Pituitary gland | 4 | 3 | 2 | 4 | 2 | 4 | 2 |
| Prostate gland | 4 | 4 | 5 | 4 | 2 | 4 | 3 |
| Salivary gland | 4 | 4 | 5 | 4 | 3 | 4 | 3 |
| Saphenous vein | 4 | 2 | 2 | 4 | 3 | 5 | 2 |
| Skeletal muscle | 5 | 2 | 2 | 4 | 3 | 4 | 2 |
| Spinal cord | 4 | 2 | 2 | 4 | 2 | 5 | 2 |
| Spleen | 5 | 2 | 2 | 5 | 2 | 5 | 3 |
| Stomach | 4 | 4 | 4 | 4 | 2 | 5 | 3 |
| Testes | 4 | 2 | 2 | 4 | 3 | 4 | 6 |
| Thyroid gland | 4 | 3 | 5 | 4 | 2 | 5 | 3 |
| Tongue | 5 | 4 | 3 | 4 | 3 | 5 | 3 |
| Tonsil | 4 | 2 | 2 | 5 | 2 | 5 | 3 |
| Trachea | 4 | 4 | 4 | 4 | 6 | 5 | 3 |
| Trigeminal ganglia | 4 | 2 | 2 | 4 | 2 | 5 | 2 |
| Urethra | 5 | 3 | 4 | 4 | 2 | 5 | 3 |
| Vagina | 5 | 2 | 3 | 4 | 2 | 5 | 3 |
| Vulva | 5 | 2 | 3 | 4 | 2 | 4 | 3 |

**Table 19: Affymetrix array scores of additional markers in normal tissues.**

| Tissue | EGFL6 | KCNE3 | KCNE4 | c14orf-100 | C2orf6 | KCNK5 | C14orf28 |
|---|---|---|---|---|---|---|---|
| Adipose tissue | 4 | 4 | 3 | 5 | 2 | 3 | 2 |
| Adrenal gland cortex | 2 | 4 | 2 | 5 | 2 | 3 | 2 |
| Bone marrow | 3 | 5 | 2 | 5 | 3 | 3 | 2 |
| Bronchus | 3 | 5 | 2 | 5 | 2 | 3 | 2 |
| Cerebellum | 2 | 3 | 2 | 5 | 2 | 3 | 2 |
| Cerebral cortex | 3 | 3 | 2 | 5 | 2 | 3 | 2 |
| Cerebrum | 3 | 4 | 2 | 5 | 2 | 3 | 2 |
| Cervix | 3 | 5 | 3 | 5 | 3 | 3 | 2 |
| Colon cecum | 2 | 5 | 2 | 4 | 2 | 3 | 2 |
| Coronary artery | 2 | 4 | 3 | 4 | 2 | 3 | 2 |
| Endometrium | 4 | 5 | 3 | 5 | 3 | 3 | 2 |
| Esophagus | 3 | 4 | 2 | 5 | 3 | 3 | 2 |
| Heart atrium | 2 | 4 | 2 | 5 | 2 | 3 | 2 |
| Heart ventricle | 3 | 4 | 2 | 4 | 2 | 3 | 2 |
| Kidney cortex | 2 | 5 | 2 | 5 | 3 | 5 | 2 |
| Kidney medulla | 3 | 5 | 2 | 5 | 2 | 4 | 2 |
| Liver | 3 | 4 | 2 | 5 | 2 | 4 | 3 |
| Lung | 5 | 5 | 2 | 5 | 2 | 3 | 2 |
| Lymph nodes | 3 | 4 | 3 | 5 | 3 | 3 | 2 |
| Mammary gland | 6 | 4 | 2 | 5 | 2 | 3 | 2 |
| Medulla | 3 | 4 | 2 | 5 | 2 | 3 | 2 |
| Midbrain | 3 | 4 | 2 | 5 | 2 | 3 | 2 |
| Myometrium | 3 | 4 | 4 | 5 | 2 | 3 | 3 |
| Nipple cross section | 3 | 4 | 2 | 5 | 2 | 3 | 2 |
| Oral mucosa | 3 | 3 | 2 | 5 | 3 | 3 | 2 |
| Ovary | 3 | 5 | 3 | 5 | 3 | 3 | 3 |
| Pharyngeal mucosa | 3 | 4 | 2 | 5 | 3 | 3 | 2 |
| Pituitary gland | 3 | 4 | 2 | 5 | 2 | 3 | 2 |
| Prostate gland | 3 | 4 | 2 | 6 | 3 | 3 | 3 |
| Salivary gland | 3 | 4 | 2 | 5 | 2 | 3 | 3 |
| Saphenous vein | 3 | 4 | 5 | 5 | 2 | 3 | 4 |
| Skeletal muscle | 3 | 3 | 2 | 5 | 2 | 3 | 2 |
| Spinal cord | 3 | 4 | 2 | 6 | 2 | 3 | 2 |
| Spleen | 2 | 5 | 3 | 5 | 3 | 3 | 2 |
| Stomach | 3 | 6 | 3 | 5 | 3 | 3 | 3 |
| Testes | 2 | 3 | 2 | 5 | 2 | 3 | 4 |
| Thyroid gland | 2 | 5 | 2 | 5 | 2 | 3 | 3 |
| Tongue | 3 | 4 | 2 | 5 | 3 | 3 | 2 |
| Tonsil | 3 | 4 | 2 | 5 | 3 | 3 | 2 |
| Trachea | 3 | 5 | 2 | 5 | 2 | 3 | 2 |
| Trigeminal ganglia | 4 | 4 | 3 | 6 | 2 | 3 | 2 |
| Urethra | 3 | 5 | 3 | 6 | 3 | 3 | 3 |
| Vagina | 3 | 5 | 2 | 5 | 3 | 3 | 2 |
| Vulva | 3 | 4 | 2 | 5 | 3 | 3 | 2 |

**Table 20: Affymetrix array scores of additional markers in normal tissues.**

| Tissue | PCDHB2 | FZD10 | ST14 | OLFML2 B | GPR105 | SDC1 | FLJ46072 |
|---|---|---|---|---|---|---|---|
| Adipose tissue | 4 | 3 | 3 | 5 | 4 | 4 | 4 |
| Adrenal gland cortex | 5 | 2 | 2 | 4 | 2 | 3 | 4 |
| Bone marrow | 4 | 2 | 2 | 3 | 2 | 3 | 3 |
| Bronchus | 4 | 3 | 4 | 4 | 2 | 4 | 5 |
| Cerebellum | 5 | 3 | 2 | 3 | 2 | 3 | 3 |
| Cerebral cortex | 6 | 2 | 2 | 3 | 2 | 3 | 4 |
| Cerebrum | 6 | 2 | 2 | 3 | 2 | 3 | 4 |
| Cervix | 5 | 4 | 3 | 5 | 2 | 5 | 4 |
| Colon cecum | 5 | 3 | 4 | 4 | 5 | 4 | 4 |
| Coronary artery | 5 | 2 | 2 | 5 | 3 | 2 | 3 |
| Endometrium | 4 | 4 | 3 | 6 | 5 | 3 | 4 |
| Esophagus | 4 | 5 | 2 | 3 | 3 | 7 | 6 |
| Heart atrium | 4 | 2 | 2 | 4 | 3 | 2 | 4 |
| Heart ventricle | 5 | 2 | 2 | 3 | 3 | 3 | 3 |
| Kidney cortex | 5 | 2 | 3 | 3 | 2 | 6 | 5 |
| Kidney medulla | 4 | 2 | 4 | 3 | 2 | 6 | 5 |
| Liver | 4 | 2 | 3 | 3 | 2 | 7 | 5 |
| Lung | 6 | | 3 | 5 | 4 | 4 | 4 |
| Lymph nodes | 5 | 3 | 2 | 4 | 3 | 3 | 3 |
| Mammary gland | 4 | 3 | 3 | 5 | 3 | 4 | 5 |
| Medulla | 6 | 2 | 2 | 3 | 2 | 2 | 3 |
| Midbrain | 6 | 2 | 2 | 4 | 3 | 3 | 3 |
| Myometrium | 5 | 2 | 2 | 4 | 2 | 3 | 3 |
| Nipple cross section | 4 | 4 | 4 | 4 | 4 | 6 | 6 |
| Oral mucosa | 4 | 5 | 6 | 4 | 4 | 7 | 7 |
| Ovary | 4 | 2 | 2 | 4 | 2 | 3 | 3 |
| Pharyngeal mucosa | 4 | 6 | 4 | 4 | 4 | 6 | 6 |
| Pituitary gland | 5 | 3 | 3 | 3 | 4 | 3 | 4 |
| Prostate gland | 4 | 2 | 4 | 3 | 2 | 5 | 5 |
| Salivary gland | 4 | 3 | 4 | 4 | 2 | 4 | 5 |
| Saphenous vein | 5 | 3 | 3 | 6 | 4 | 3 | 3 |
| Skeletal muscle | 4 | 3 | 3 | 4 | 4 | 3 | 3 |
| Spinal cord | 6 | 3 | 2 | 3 | 2 | 2 | 3 |
| Spleen | 7 | 2 | 3 | 3 | 3 | 3 | 3 |
| Stomach | 5 | 3 | 4 | 4 | 5 | 4 | 5 |
| Testes | 6 | 2 | 2 | 5 | 2 | 4 | 4 |
| Thyroid gland | 4 | 3 | 4 | 5 | 3 | 4 | 6 |
| Tongue | 4 | 2 | 4 | 4 | 3 | 5 | 5 |
| Tonsil | 4 | 3 | 4 | 4 | 4 | 5 | 5 |
| Trachea | 4 | 3 | 5 | 4 | 3 | 5 | 5 |
| Trigeminal ganglia | 5 | 3 | 2 | 5 | 4 | 3 | 4 |
| Urethra | 5 | 2 | 4 | 6 | 5 | 6 | 5 |
| Vagina | 4 | 2 | 4 | 4 | 2 | 6 | 5 |
| Vulva | 4 | 5 | 4 | 4 | 3 | 6 | 6 |

**Table 21: Affymetrix array scores of additional markers in normal tissues.**

| Tissue | IVNS1ABP | SPP1 | DKFZ p762 E1312 | WFDC2 | KIAA1 892 | C6orf69 | KIBRA |
|---|---|---|---|---|---|---|---|
| Adipose tissue | 4 | 5 | 3 | 4 | 5 | 6 | 4 |
| Adrenal gland cortex | 4 | 3 | 3 | 3 | 7 | 6 | 4 |
| Bone marrow | 6 | 5 | 5 | 4 | 8 | 5 | 4 |
| Bronchus | 5 | 5 | 3 | 7 | 5 | 5 | 5 |
| Cerebellum | 4 | 6 | 3 | 4 | 5 | 5 | 5 |
| Cerebral cortex | 5 | 8 | 3 | 4 | 5 | 5 | 5 |
| Cerebrum | 5 | 8 | 3 | 4 | 5 | 5 | 6 |
| Cervix | 5 | 6 | 3 | 5 | 6 | 6 | 4 |
| Colon cecum | 4 | 5 | 4 | 4 | 5 | 5 | 4 |
| Coronary artery | 5 | 5 | 3 | 4 | 5 | 5 | 4 |
| Endometrium | 5 | 5 | 4 | 6 | 6 | 5 | 5 |
| Esophagus | 4 | 3 | 4 | 4 | 6 | 6 | 5 |
| Heart atrium | 4 | 4 | 3 | 3 | 5 | 5 | 4 |
| Heart ventricle | 5 | 3 | 3 | 4 | 5 | 5 | 4 |
| Kidney cortex | 6 | 9 | 3 | 5 | 5 | 5 | 6 |
| Kidney medulla | 6 | 9 | 3 | 5 | 6 | 5 | 6 |
| Liver | 4 | 5 | 3 | 3 | 5 | 6 | 5 |
| Lung | 5 | 5 | 3 | 5 | 5 | 6 | 5 |
| Lymph nodes | 5 | 4 | 3 | 4 | 6 | 6 | 4 |
| Mammary gland | 5 | 6 | 4 | 5 | 5 | 6 | 5 |
| Medulla | 5 | 9 | 3 | 3 | 5 | 5 | 6 |
| Midbrain | 5 | 9 | 3 | 4 | 5 | 5 | 5 |
| Myometrium | 4 | 4 | 3 | 4 | 5 | 5 | 3 |
| Nipple cross section | 4 | 3 | 3 | 5 | 5 | 6 | 5 |
| Oral mucosa | 4 | 3 | 4 | 4 | 5 | 6 | 6 |
| Ovary | 5 | 4 | 4 | 4 | 5 | 6 | 4 |
| Pharyngeal mucosa | 5 | 3 | 4 | 5 | 6 | 6 | 5 |
| Pituitary gland | 5 | 4 | 4 | 6 | 5 | 5 | 5 |
| Prostate gland | 5 | 5 | 3 | 5 | 5 | 5 | 6 |
| Salivary gland | 5 | 4 | 3 | 7 | 5 | 5 | 4 |
| Saphenous vein | 4 | 3 | 3 | 5 | 5 | 6 | 4 |
| Skeletal muscle | 4 | 3 | 4 | 4 | 5 | 5 | 4 |
| Spinal cord | 5 | 9 | 3 | 4 | 5 | 5 | 6 |
| Spleen | 5 | 3 | 3 | 3 | 5 | 6 | 4 |
| Stomach | 5 | 4 | 4 | 4 | 5 | 5 | 5 |
| Testes | 5 | 3 | 5 | 4 | 6 | 4 | 4 |
| Thyroid gland | 5 | 4 | 3 | 6 | 5 | 5 | 6 |
| Tongue | 5 | 4 | 4 | 6 | 5 | 5 | 5 |
| Tonsil | 5 | 3 | 4 | 4 | 5 | 5 | 4 |
| Trachea | 5 | 5 | 3 | 8 | 5 | 5 | 5 |
| Trigeminal ganglia | 5 | 7 | 3 | 6 | 5 | 6 | 4 |
| Urethra | 5 | 4 | 3 | 4 | 5 | 6 | 4 |
| Vagina | 5 | 4 | 4 | 4 | 5 | 5 | 4 |
| Vulva | 5 | 3 | 4 | 4 | 5 | 5 | 5 |

**Table 22: Affymetrix array scores of additional markers in tumor tissues.**

| Tissue | c6orf55 | SCGB-2A1 | DSG2 | EPSTI1 | SEC-23B | MS4A6A | LOC51136 |
|---|---|---|---|---|---|---|---|
| Adrenal gland | 6 | 3 | 7 | 7 | 6 | 8 | 5 |
| Bladder | 5 | 3 | 7 | 5 | 5 | 5 | 4 |
| Brain | 5 | 6 | 5 | 5 | 5 | 5 | 3 |
| Breast | 6 | 6 | 7 | 5 | 6 | 6 | 5 |
| Cervix | 5 | 5 | 7 | 5 | 6 | 5 | 4 |
| Colon | 6 | 5 | 8 | 6 | 6 | 6 | 4 |
| Corpus uteri | 6 | 8 | 7 | 5 | 6 | 6 | 4 |
| Endometrium | 5 | 9 | 7 | 5 | 6 | 5 | 4 |
| Esophagus | 5 | 2 | 5 | 5 | 5 | 5 | 3 |
| Kidney | 5 | 3 | 7 | 5 | 6 | 6 | 4 |
| Liver | 5 | 3 | 7 | 6 | 6 | 6 | 5 |
| Lung | 5 | 5 | 7 | 6 | 6 | 6 | 4 |
| Omentum | 6 | 8 | 7 | 6 | 6 | 6 | 4 |
| Ovary | 6 | 8 | 7 | 6 | 6 | 6 | 4 |
| Pancreas | 5 | 6 | 6 | 6 | 6 | 6 | 3 |
| Prostate | 5 | 4 | 6 | 4 | 6 | 5 | 4 |
| Rectosigmoid | 5 | 4 | 8 | 6 | 6 | 6 | 4 |
| Small intestine | 5 | 6 | 6 | 5 | 6 | 5 | 4 |
| Stomach | 5 | 4 | 7 | 5 | 6 | 5 | 4 |
| Testis | 5 | 2 | 4 | 7 | 6 | 8 | 4 |
| Thyroid | 5 | 4 | 7 | 5 | 5 | 5 | 4 |
| Vulva | 6 | 2 | 6 | 5 | 6 | 5 | 4 |

**Table 23: Affymetrix array scores of additional markers in tumor tissues.**

| Tissue | EGFL6 | KCNE3 | KCNE4 | C14orf-100 | C2orf6 | KCNK5 | C14orf28 |
|---|---|---|---|---|---|---|---|
| Adrenal gland | 3 | 7 | 5 | 7 | 5 | 5 | 3 |
| Bladder | 5 | 4 | 3 | 5 | 4 | 3 | 3 |
| Brain | 5 | 5 | 3 | 5 | 3 | 3 | 2 |
| Breast | 5 | 5 | 5 | 6 | 4 | 4 | 3 |
| Cervix | 5 | 5 | 4 | 5 | 4 | 3 | 3 |
| Colon | 4 | 6 | 3 | 6 | 4 | 4 | 3 |
| Corpus uteri | 7 | 5 | 4 | 6 | 4 | 3 | 3 |
| Endometrium | 5 | 6 | 4 | 6 | 4 | 4 | 3 |
| Esophagus | 3 | 6 | 3 | 5 | 3 | 3 | 2 |
| Kidney | 3 | 7 | 4 | 6 | 4 | 4 | 3 |
| Liver | 4 | 6 | 4 | 6 | 4 | 4 | 3 |
| Lung | 6 | 6 | 4 | 6 | 4 | 4 | 3 |
| Omentum | 6 | 5 | 4 | 6 | 4 | 4 | 3 |
| Ovary | 5 | 6 | 4 | 6 | 5 | 4 | 3 |
| Pancreas | 4 | 5 | 5 | 6 | 4 | 4 | 3 |
| Prostate | 3 | 4 | 3 | 6 | 4 | 3 | 3 |
| Rectosigmoid | 5 | 7 | 4 | 6 | 4 | 5 | 3 |
| Small intestine | 5 | 5 | 3 | 6 | 4 | 4 | 3 |
| Stomach | 3 | 7 | 4 | 6 | 4 | 4 | 3 |
| Testis | 3 | 5 | 3 | 6 | 5 | 4 | 3 |
| Thyroid | 5 | 5 | 4 | 6 | 4 | 4 | 3 |
| Vulva | 5 | 5 | 3 | 5 | 4 | 3 | 3 |

**Table 24: Affymetrix array scores of additional markers in tumor tissues.**

| Tissue | PCDHB2 | FZD10 | ST14 | OLFML 2B | GPR105 | SDC1 | FLJ46072 |
|---|---|---|---|---|---|---|---|
| Adrenal gland | 6 | 2 | 6 | 7 | 4 | 7 | 6 |
| Bladder | 5 | 3 | 5 | 5 | 3 | 7 | 6 |
| Brain | 6 | 3 | 4 | 4 | 2 | 5 | 5 |
| Breast | 5 | 3 | 5 | 6 | 4 | 7 | 6 |
| Cervix | 5 | 5 | 5 | 5 | 3 | 7 | 6 |
| Colon | 5 | 4 | 6 | 5 | 4 | 6 | 6 |
| Corpus uteri | 5 | 6 | 5 | 7 | 3 | 5 | 5 |
| Endometrium | 5 | 5 | 5 | 5 | 3 | 6 | 6 |
| Esophagus | 5 | 2 | 5 | 5 | 3 | 6 | 5 |
| Kidney | 6 | 2 | 4 | 5 | 4 | 6 | 5 |
| Liver | 5 | 3 | 6 | 6 | 3 | 7 | 6 |
| Lung | 6 | 4 | 5 | 6 | 4 | 7 | 6 |
| Omentum | 5 | 5 | 5 | 6 | 3 | 6 | 6 |
| Ovary | 5 | 5 | 5 | 5 | 3 | 6 | 6 |
| Pancreas | 6 | 2 | 5 | 6 | 4 | 6 | 5 |
| Prostate | 4 | 3 | 5 | 4 | 3 | 5 | 5 |
| Rectosigmoid | 5 | 4 | 6 | 6 | 5 | 6 | 6 |
| Small intestine | 5 | 4 | 5 | 4 | 3 | 5 | 5 |
| Stomach | 6 | 3 | 5 | 5 | 6 | 6 | 6 |
| Testis | 4 | 2 | 3 | 6 | 6 | 4 | 4 |
| Thyroid | 5 | 3 | 4 | 4 | 4 | 6 | 5 |
| Vulva | 5 | 4 | 5 | 6 | 3 | 8 | 6 |

**Table 25: Affymetrix array scores of additional markers in tumor tissues.**

| Tissue | IVNS1 ABP | SPP1 | DKFZp762 E1312 | WFDC2 | KIAA1 892 | C6orf69 | KIBRA |
|---|---|---|---|---|---|---|---|
| Adrenal gland | 7 | 9 | 6 | 6 | 6 | 7 | 5 |
| Bladder | 5 | 7 | 4 | 6 | 5 | 6 | 4 |
| Brain | 5 | 6 | 3 | 6 | 5 | 6 | 5 |
| Breast | 5 | 8 | 4 | 6 | 6 | 6 | 5 |
| Cervix | 6 | 7 | 4 | 6 | 5 | 6 | 4 |
| Colon | 6 | 7 | 4 | 6 | 6 | 6 | 5 |
| Corpus uteri | 6 | 7 | 5 | 7 | 6 | 6 | 5 |
| Endometrium | 6 | 8 | 4 | 8 | 6 | 6 | 5 |
| Esophagus | 5 | 6 | 4 | 5 | 4 | 5 | 4 |
| Kidney | 7 | 9 | 3 | 6 | 5 | 6 | 6 |
| Liver | 6 | 9 | 4 | 6 | 6 | 6 | 5 |
| Lung | 6 | 8 | 4 | 7 | 6 | 6 | 5 |
| Omentum | 6 | 8 | 5 | 7 | 6 | 6 | 5 |
| Ovary | 6 | 8 | 5 | 8 | 6 | 6 | 5 |
| Pancreas | 5 | 7 | 3 | 5 | 5 | 6 | 5 |
| Prostate | 5 | 5 | 3 | 5 | 5 | 6 | 6 |
| Rectosigmoid | 6 | 8 | 4 | 6 | 6 | 6 | 5 |
| Small intestine | 5 | 7 | 4 | 5 | 5 | 6 | 4 |
| Stomach | 5 | 7 | 4 | 4 | 5 | 6 | 5 |
| Testis | 7 | 6 | 4 | 5 | 7 | 6 | 4 |
| Thyroid | 6 | 7 | 3 | 6 | 5 | 5 | 5 |
| Vulva | 6 | 7 | 4 | 4 | 6 | 6 | 4 |

Thus, TVM disclosed herein are enriched in a wide variety of tumor cells. These findings further validate the TVM identified herein, and demonstrate that the TVM are relevant in general to tumor cells. Further, the results show that diagnostic, localization, and therapeutic methods based on the disclosed TVM are not limited to ovarian tumors, but rather are applicable to all solid tumors.

## Claims

1. A ligand selected from a nucleic acid molecule which can hybridize to a nucleic acid molecule set forth in SEQ ID No: 1 and an antibody to a protein encoded by a nucleic acid molecule of SEQ ID No:1, for use in localizing an ovarian tumor vasculature.

2. A ligand selected from a nucleic acid molecule which can hybridize to a nucleic acid molecule set forth in SEQ ID No: 1 and an antibody to a protein encoded by a nucleic acid molecule of SEQ ID No:1, for use in treating a solid ovarian tumor in a subject, wherein said treating an ovarian tumor comprises (a) contacting said subject with a the photoactivatable cytotoxic drug; (b) localizing an ovarian tumor vasculature with said ligand; and (c) contacting said solid ovarian tumor with a concentrated light source.

3. A ligand selected from a nucleic acid molecule which can hybridize to a nucleic acid molecule set forth in SEQ ID No: 1 and an antibody to a protein encoded by a nucleic acid molecule of SEQ ID No: 1, for use in treating an ovarian tumor in a subject, wherein said antibody or nucleic acid molecule is conjugated to a cytotoxic agent.

4. A ligand according to any one of claims 1-3 for use as defined in any one of claims 1-3, wherein said ligand is a nucleic acid molecule which can hybridize to a nucleic acid molecule set forth in SEQ ID No: 1 and is selected from an antisense molecule, an inhibitory RNA (RNAi), a short hairpin RNA (shRNA), a small inhibitory RNA (siRNA), or a microRNA (miRNA).

5. A ligand according to claim 3 for use in treating an ovarian tumor in a subject according to claim 3, wherein said cytotoxic drug is selected from a radioactive isotope, a cytotoxic virus, a cytotoxic pathogen or an engineered cytotoxic cell.

6. An antisense or RNAi molecule that is taken up by a vasculature cell for use in impeding a vascularization of an ovarian tumor in a subject, wherein the antisense or RNAi molecule is capable of inhibiting an activity of a nucleic acid molecule set forth in SEQ ID No: 1 or a protein encoded by said nucleic acid molecule.

7. Use of a ligand selected from a nucleic acid molecule which can hybridize to a nucleic acid molecule set forth in SEQ ID No: 1 and an antibody to a protein encoded by a nucleic acid molecule of SEQ ID No:1, in the manufacture of an agent for localizing an ovarian tumor vasculature.

8. Use of a ligand selected from a nucleic acid molecule which can hybridize to a nucleic acid molecule set forth in SEQ ID No: 1 and an antibody to a protein encoded by a nucleic acid molecule of SEQ ID No:1, in the manufacture of a medicament for treating a solid ovarian tumor in a subject, wherein said treating an ovarian tumor comprises (a) contacting said subject with a photoactivatable cytotoxic drug; (b) localizing an ovarian tumor vasculature with said ligand; and (c) contacting said solid ovarian tumor with a concentrated light source.

9. Use of ligand selected from a nucleic acid molecule which can hybridize to a nucleic acid molecule set forth in SEQ ID No: 1 and an antibody to a protein encoded by a nucleic acid molecule of SEQ ID No:1, in the manufacture of a medicament for treating an ovarian tumor in a subject, wherein said antibody or nucleic acid molecule is conjugated to a cytotoxic agent.

10. A use according to any one of claims 7-9, wherein said ligand is a nucleic acid molecule which can hybridize to a nucleic acid molecule set forth in SEQ ID No: 1 and is selected from an antisense molecule, an inhibitory RNA (RNAi), a short hairpin RNA (shRNA), a small inhibitory RNA (siRNA), or a microRNA (miRNA).

11. A use of a ligand according to claim 9, wherein said cytotoxic drug is selected from a radioactive isotope, a cytotoxic virus, a cytotoxic pathogen or an engineered cytotoxic cell.

12. Use of an antisense or RNAi molecule that is taken up by a vasculature cell in the manufacture of a medicament for impeding a vascularization of an ovarian tumor in a subject, wherein the antisense or RNAi molecule is capable of inhibiting an activity of a nucleic acid molecule set forth in SEQ ID No: 1 or a protein encoded by said nucleic acid molecule.

## Patentansprüche

1. Ligand, ausgewählt aus einem Nukleinsäuremolekül, das an ein in SEQ ID NO: 1 angegebenes Nukleinsäuremolekül hybridisieren kann, und einem Antikörper gegen ein Protein, für das von einem Nukleinsäuremolekül von SEQ ID NO: 1 codiert wird, zur Verwendung bei der Lokalisierung eines Ovarialtumorgefäßsystems.

2. Ligand, ausgewählt aus einem Nukleinsäuremolekül, das an ein in SEQ ID NO: 1 angegebenes Nukleinsäuremolekül hybridisieren kann, und einem Antikörper gegen ein Protein, für das von einem Nukleinsäuremolekül von SEQ ID NO: 1 codiert wird, zur Verwendung bei der Behandlung eines soliden Ovarialtumors bei einem Probanden, wobei das Behandeln eines Ovarialtumors Folgendes umfasst: (a) Inkontaktbringen des Probanden mit einem fotoaktivierbaren zytotoxischen Arzneistoff; (b) Lokalisieren eines Ovarialtumorgefäßsystems mit dem Liganden; und (c) Inkontaktbringen des soliden Ovarialtumors mit einer konzentrierten Lichtquelle.

3. Ligand, ausgewählt aus einem Nukleinsäuremolekül, das an ein in SEQ ID NO: 1 angegebenes Nukleinsäuremolekül hybridisieren kann, und einem Antikörper gegen ein Protein, für das von einem Nukleinsäuremolekül von SEQ ID NO: 1 codiert wird, zur Verwendung bei der Behandlung eines Ovarialtumors bei einem Probanden, wobei der Antikörper oder das Nukleinsäuremolekül an ein zytotoxisches Mittel konjugiert ist.

4. Ligand nach einem der Ansprüche 1-3 zur Verwendung wie in einem der Ansprüche 1-3 definiert, wobei der Ligand ein Nukleinsäuremolekül ist, das an ein in SEQ ID NO: 1 angegebenes Nukleinsäuremolekül hybridisieren kann und aus einem Antisense-Molekül, einer inhibitorischen RNA (RNAi), einer kurzen Haarnadel-RNA (shRNA), einer kleinen inhibitorischen RNA (siRNA) oder einer microRNA (miRNA) ausgewählt ist.

5. Ligand nach Anspruch 3 zur Verwendung bei der Behandlung eines Ovarialtumors in einem Probanden nach Anspruch 3, wobei der zytotoxische Arzneistoff aus einem radioaktiven Isotop, einem zytotoxischen Virus, einem zytotoxischen Pathogen oder einer gentechnisch veränderten zytotoxischen Zelle ausgewählt ist.

6. Antisense- oder RNAi-Molekül, das durch eine Gefäßzelle zur Verwendung bei der Eindämmung einer Vaskularisierung eines Ovarialtumors in einem Probanden aufgenommen wird, wobei das Antisense- oder RNAi-Molekül eine Aktivität eines in SEQ ID NO: 1 angegebenen Nukleinsäuremoleküls oder eines Proteins, für das von dem Nukleinsäuremolekül codiert wird, hemmen kann.

7. Verwendung eines Liganden, ausgewählt aus einem Nukleinsäuremolekül, das an ein in SEQ ID NO: 1 angegebenes Nukleinsäuremolekül hybridisieren kann, und einem Antikörper gegen ein Protein, für das von einem Nukleinsäuremolekül von SEQ ID NO: 1 codiert wird, bei der Herstellung eines Mittels zur Lokalisierung eines Ovarialtumorgefäßsystems.

8. Verwendung eines Liganden, ausgewählt aus einem Nukleinsäuremolekül, das an ein in SEQ ID NO: 1 angegebenes Nukleinsäuremolekül hybridisieren kann, und einem Antikörper gegen ein Protein, für das von einem Nukleinsäuremolekül von SEQ ID NO: 1 codiert wird, bei der Herstellung eines Medikaments zur Behandlung eines soliden Ovarialtumors bei einem Probanden, wobei das Behandeln eines Ovarialtumors Folgendes umfasst: (a) Inkontaktbringen des Probanden mit einem fotoaktivierbaren zytotoxischen Arzneistoff; (b) Lokalisieren eines Ovarialtumorgefäßsystems mit dem Liganden; und (c) Inkontaktbringen des soliden Ovarialtumors mit einer konzentrierten Lichtquelle.

9. Verwendung eines Liganden, ausgewählt aus einem Nukleinsäuremolekül, das an ein in SEQ ID NO: 1 angegebenes Nukleinsäuremolekül hybridisieren kann, und einem Antikörper gegen ein Protein, für das von einem Nukleinsäuremolekül von SEQ ID NO: 1 codiert wird, bei der Herstellung eines Medikaments zur Behandlung eines Ovarialtumors bei einem Probanden, wobei der Antikörper oder das Nukleinsäuremolekül an ein zytotoxisches Mittel konjugiert ist.

10. Verwendung nach einem der Ansprüche 7-9, wobei der Ligand ein Nukleinsäuremolekül ist, das an ein in SEQ ID NO: 1 angegebenes Nukleinsäuremolekül hybridisieren kann und aus einem Antisense-Molekül, einer inhibitorischen RNA (RNAi), einer kurzen Haarnadel-RNA (shRNA), einer kleinen inhibitorischen RNA (siRNA) oder einer microRNA (miRNA) ausgewählt ist.

11. Verwendung eines Liganden nach Anspruch 9, wobei der zytotoxische Arzneistoff aus einem radioaktiven Isotop, einem zytotoxischen Virus, einem zytotoxischen Pathogen oder einer gentechnisch veränderten zytotoxischen Zelle ausgewählt ist.

12. Verwendung eines Antisense- oder RNAi-Moleküls, das durch eine Gefäßzelle aufgenommen wird, bei der Herstellung eines Medikaments zur Eindämmung einer Vaskularisierung eines Ovarialtumors in einem Probanden, wobei das Antisense- oder RNAi-Molekül eine Aktivität eines in SEQ ID NO: 1 angegebenen Nukleinsäuremoleküls oder eines Proteins, für das von dem Nukleinsäuremolekül codiert wird, hemmen kann.

## Revendications

1. Ligand sélectionné parmi une molécule d'acide nucléique pouvant s'hybrider à une molécule d'acide nucléique présentée dans SEQ ID No. : 1 et un anticorps à une protéine codée par une molécule d'acide nucléique de SEQ ID No. : 1, destiné à être utilisé pour la localisation d'un réseau vasculaire tumoral ovarien.

2. Ligand sélectionné parmi une molécule d'acide nucléique pouvant s'hybrider à une molécule d'acide nucléique présentée dans SEQ ID No. : 1 et un anticorps à une protéine codée par une molécule d'acide nucléique de SEQ ID No. : 1, destiné à être utilisé pour le traitement d'une tumeur ovarienne solide chez un sujet, dans lequel ledit traitement de tumeur ovarienne comprend (a) la mise en contact dudit sujet avec le médicament cytotoxique photoactivable ; (b) la localisation d'un réseau vasculaire tumoral ovarien avec ledit ligand ; et (c) la mise en contact de ladite tumeur ovarienne solide avec une source de lumière concentrée.

3. Ligand sélectionné parmi à partir d'une molécule d'acide nucléique pouvant s'hybrider à une molécule d'acide nucléique présentée dans SEQ ID No. : 1 et un anticorps à une protéine codée par une molécule d'acide nucléique de SEQ ID No. : 1, destiné à être utilisé pour le traitement d'une tumeur ovarienne chez un sujet, dans lequel ledit anticorps ou ladite molécule d'acide nucléique est conjuguée à un agent cytotoxique.

4. Ligand selon l'une quelconque des revendications 1 à 3, pour une utilisation définie dans l'une quelconque des revendications 1 à 3, dans lequel ledit ligand est une molécule d'acide nucléique pouvant s'hybrider à une molécule d'acide nucléique présentée dans SEQ ID No. : 1 et est sélectionné parmi une molécule antisens, un ARN inhibiteur (ARNi), un ARN court en épingle à cheveux (ARNsh), un ARN inhibiteur court (ARNic), ou un microARN (miARN).

5. Ligand selon la revendication 3 destiné à être utilisé pour le traitement d'une tumeur ovarienne chez un sujet selon la revendication 3, dans lequel ledit médicament cytotoxique est sélectionné parmi un isotope radioactif, un virus cytotoxique, un agent pathogène cytotoxique ou une cellule cytotoxique manipulée.

6. Molécule antisens ou d'ARNi absorbée par une cellule de réseau vasculaire destinée à être utilisée pour entraver une vascularisation d'une tumeur ovarienne chez un sujet, dans laquelle la molécule antisens ou d'ARNi est susceptible d'inhiber une activité d'une molécule d'acide nucléique présentée dans SEQ ID No. : 1 ou une protéine codée par ladite molécule d'acide nucléique.

7. Utilisation d'un ligand sélectionné parmi une molécule d'acide nucléique pouvant s'hybrider à une molécule d'acide nucléique présentée dans SEQ ID No. : 1 et un anticorps à une protéine codée par une molécule d'acide nucléique de SEQ ID No. : 1, dans la fabrication d'un agent pour localiser un réseau vasculaire tumoral ovarien.

8. Utilisation d'un ligand sélectionné parmi une molécule d'acide nucléique pouvant s'hybrider à une molécule d'acide nucléique présentée dans SEQ ID No. : 1 et un anticorps à une protéine codée par une molécule d'acide nucléique de SEQ ID No. : 1, dans la fabrication d'un médicament destiné au traitement d'une tumeur ovarienne solide chez un sujet, dans laquelle ledit traitement de tumeur ovarienne comprend (a) la mise en contact dudit sujet avec le médicament cytotoxique photoactivable ; (b) la localisation d'un réseau vasculaire tumoral ovarien avec ledit ligand ; et (c) la mise en contact de ladite tumeur ovarienne solide avec une source de lumière concentrée.

9. Utilisation d'un ligand sélectionné parmi une molécule d'acide nucléique pouvant s'hybrider à une molécule d'acide nucléique présentée dans SEQ ID No. : 1 et un anticorps à une protéine codée par une molécule d'acide nucléique de SEQ ID No. : 1, dans la fabrication d'un médicament destiné au traitement d'une tumeur ovarienne chez un sujet, dans laquelle ledit anticorps ou ladite molécule d'acide nucléique est conjugué à un agent cytotoxique.

10. Utilisation selon l'une quelconque des revendications 7 à 9, dans laquelle ledit ligand est une molécule d'acide nucléique pouvant s'hybrider à une molécule d'acide nucléique présentée dans SEQ ID No. : 1 et est sélectionné parmi une molécule antisens, un ARN inhibiteur (ARNi), un ARN court en épingle à cheveux (ARNsh), un ARN inhibiteur court (ARNic), ou un microARN (miARN) .

11. Utilisation d'un ligand selon la revendication 9, dans laquelle ledit médicament cytotoxique est sélectionné parmi un isotope radioactif, un virus cytotoxique, un agent pathogène cytotoxique ou une cellule cytotoxique manipulée.

12. Utilisation d'une molécule antisens ou d'ARNi qui est absorbée par une cellule de réseau vasculaire dans la fabrication d'un médicament pour entraver une vascularisation d'une tumeur ovarienne chez un sujet, dans laquelle la molécule antisens ou d'ARNi est susceptible d'inhiber une activité d'une molécule d'acide nucléique présentée dans SEQ ID No. : 1 ou d'une protéine codée par ladite molécule d'acide nucléique.
